# EUROPEAN PATENT SPECIFICATION

(11) **EP 3 149 017 B1**
(45) Date of publication and mention of the grant of the patent: **11.12.2019**
(21) Application number: 15770630.0
(22) Date of filing: 27.05.2015
(51) Int. Cl.: C07H 19/10, C07H 19/11, C07H 19/20, C07H 19/213, A61K 31/7068, A61K 31/7076, A61P 35/02

(54) **NUCLEOSIDE DERIVATIVES FOR THE TREATMENT OF CANCER**
NUKLEOSIDDERIVATE ZUR BEHANDLUNG VON KREBS
DÉRIVÉS DE NUCLÉOSIDES POUR LE TRAITEMENT DU CANCER

(30) Priority: 28.05.2014 US 201462004066 P
(43) Date of publication of application: 05.04.2017
(73) Proprietor: Idenix Pharmaceuticals LLC, Cambridge, MA 02141 (US)
(72) Inventor: DOUSSON, Cyril, F-34800 Canet (FR); DUKHAN, David, F-34980 Saint Gely Du Fesc (FR); PARSY, Christophe Claude, F-34830 Jacou (FR); ALEXANDRE, François-René, F-34090 Montpellier (FR); RAHALI, Rachid, F-30126 Saint Laurent Des Arbres (FR); PAPARIN, Jean-Laurent, F-34230 Vendemian (FR)
(74) Representative: Jaap, David Robert
(86) International application number: PCT/IB2015/000957
(87) International publication number: WO 2015/181624

(56) References cited:
- WO-A1-99/45935
- WO-A1-2006/100439
- WO-A1-2012/117246
- US-A- 3 804 827
- US-A1- 2008 261 913
- US-A1- 2008 292 553
- US-A1- 2013 210 757
- US-A1- 2013 315 866
- BAZZANINI ET AL.: "PRODRUGS OF ARA-CMP AND ARA-AMP WITH A S-ACYL-2-THIOETHYL (SATE) BIOLABILE PHOSPHATE PROTECTING GROUP: SYNTHESIS AND BIOLOGICAL EVALUATION", NUCLEOSIDES & NUCLEOTIDES, vol. 18, no. 4/05, 1 January 1999 (1999-01-01), page 971/972, XP009029311, ISSN: 0732-8311
- BAZZANINI ET AL.: "Synthetic approaches to a mononucleotide prodrug of cytarabine", NUCLEOSIDES, NUCLEOTIDES AND NUCLEIC ACIDS, vol. 24, no. 10-12, 2005, pages 1635-1643, XP008141704, ISSN: 1525-7770, DOI: 10.1080/15257770500267006 [retrieved on 2006-08-16]
- GOUY ET AL.: "Special feature of mixed phosphotriester derivatives of cytarabine", BIOORG. MED. CHEM., vol. 17, no. 17, 1 September 2009 (2009-09-01), pages 6340-6347, XP026471113, ISSN: 0968-0896, DOI: 10.1016/J.BMC.2009.07.038 [retrieved on 2009-07-23]
- TOBIAS; BORCH: "Synthesis and Biological Evaluation of a Cytarabine Phosphoramidate Prodrug", MOLECULAR PHARMACEUTICS, vol. 1, no. 2, 2004, pages 112-116, XP9186775, DOI: 10.1021/mp034019v
- ROBAK ET AL.: "Current status of older and new purine nucleoside analogues in the treatment of lymphoproliferative diseases", MOLECULES, vol. 14, no. 3, 23 March 2009 (2009-03-23) , pages 1183-1226, XP002583109, ISSN: 1420-3049, DOI: 10.3390/MOLECULES14031183 [retrieved on 2009-01-01]
- JORDHEIM ET AL.: "Advances in the development of nucleoside and nucleotide analogues for cancer and viral diseases", NATURE REVIEWS. DRUG DISCOVERY, vol. 12, no. 6, 31 May 2013 (2013-05-31), pages 447-464, XP055221647, GB ISSN: 1474-1776, DOI: 10.1038/nrd4010
- LONG ET AL.: "Synthesis and antitumor antiviral activities of 1-.beta.-D-arabinofuranosylpyrimidine 3',5'-cyclic phosphates", J. MED. CHEM., vol. 15, no. 12, 1 December 1972 (1972-12-01), pages 1215-1218, XP055221902, US ISSN: 0022-2623, DOI: 10.1021/jm00282a003
- DRUMMOND ET AL.: "Deoxyribonucleoside-3',5' Cyclic Phosphates. Synthesis and Acid-Catalyzed and Enzymic Hydrolysis", J. AM. CHEM. SOC., vol. 86, no. 8, 1 April 1964 (1964-04-01), pages 1626-1630, XP055221899, US ISSN: 0002-7863, DOI: 10.1021/ja01062a036
- SYMONS ET AL.: "Improved synthesis of <32>P-labelled 3',5'-cyclic AMP, 3',5'-cyclic GMP and other 3',5'cyclic ribo- and deoxyribonucletodies of high specific activity", BIOCHIMICA ET BIOPHYSICA ACTA (BBA), vol. 320, no. 2, 14 September 1973 (1973-09-14), pages 535-539, XP025789832, ISSN: 0304-4165, DOI: 10.1016/0304-4165(73)90334-6 [retrieved on 1973-09-14]

## Description

### FIELD

Provided herein are nucleoside derivatives, pharmaceutical compositions comprising the compounds, processes of preparation thereof, and method thereof for treating cancer.

### BACKGROUND

Cancer is a disease characterized primarily by an uncontrolled divisions of abnormal cells derived from a given normal tissue and the invasion of adjacent tissues by these malignant cells. Blood or lymphatic transportation can spread cancer cells to other parts of the body leading to regional lymph nodes and to distant sites (metastasis). Cancer is a complex, multistep process that begins with minor preneoplastic changes, which may under certain conditions progress to neoplasia. There are more than 100 different types of cancer, which can be grouped into broader categories. The main categories include: carcinoma, sarcoma, leukemia, lymphoma and myeloma, and central nervous system cancers. The incidence of cancer continues to climb as the general population ages, as new cancers develop, and as susceptible populations (e.g., people infected with AIDS or excessively exposed to sunlight) grow. A tremendous demand therefore exists for new methods and compositions that can be used to treat patients with cancer.

Hematologic or hematopoietic malignancies are cancers of the blood or bone marrow, including leukemia and lymphoma. Leukemia is a type of cancer of the blood characterized by abnormal accumulation of immature white blood cells. There are four types of leukemia: acute lymphocytic leukemia (ALL), acute myelogenous leukemia (AML), chronic lymphocytic leukemia (CLL), and chronic myelogenous leukemia (CML). Acute leukemia is a rapidly progressing disease that results in the accumulation of immature, functionless cells in the marrow and blood. The marrow often stops producing enough normal red cells, white cells and platelets. On the other hand, chronic leukemia progresses more slowly and allows greater numbers of more mature, functional cells to be made.

Leukemia can affect people at any age. The cause of most cases of leukemia is not known. Extraordinary doses of radiation and certain cancer therapies are possible causes. About 90% of leukemia are diagnosed in adults. In 2000 approximately 256,000 children and adults around the world developed some form of leukemia, and 209,000 died from it. Cases of Chronic leukemia account for 4.5 percent more cases than acute leukemia. The most common types of leukemia in adults are acute myelogenous leukemia (AML), with estimated 14,590 new cases in 2013, and chronic lymphocytic leukemia (CLL), with about 15,680 new cases in 2013. Chronic myelogenous leukemia (CML) was estimated to affect about 5,920 persons in 2013 (data from the Leukemia and Lymphoma Society, Facts 2013, August 2013).

The dramatic improvement in blood cancer treatment in the latter part of the 20^{th} century is largely the result of chemotherapy. In addition, there are more than 50 drugs individually used to treat these disorders and a number of potential new therapies are under investigation in clinical trials. While current chemotherapy can result in complete remissions, the long term disease-free survival rate for leukemia, in particular AML, is low. For example, the overall relative survival rate for AML was estimated to be about 59% from 2003 to 2009. Therefore, there is a clear and unmet need for effective therapeutics for treatment of blood cancers, including leukemia.

International Patent Application publication number WO 2006/100439 (University College Cardiff Consultants Limited) discloses certain phosphoramidate derivatives of nucleoside compounds for use in the treatment of cancer. Long R.A. et al, J. Med. Chem, 1972, 15(12), 1215-1218 discloses the synthesis and anti-tumour and antiviral activities of 1-β-D-arabinofuranosylpyrimidine 3',5' cyclic phosphates.

### SUMMARY OF THE DISCLOSURE

Derivatives, in one embodiment phosphoramidate derivatives and cyclic phosphate derivatives, of a variety of therapeutic agents are provided, as well as pharmaceutical compositions and uses in the treatment of a variety of cancer including hematopoietic malignancies. The therapeutic agent is, for example, an anti-cancer agent that includes, or has been derivatized to include, a reactive group, such as a hydroxyl, for attachment of the phosphoroamidate and cyclic phosphate moiety. Such therapeutic agents include, but are not limited to nucleosides and nucleoside analogs including acyclic nucleosides. A nucleoside analog refers to a structurally modified nucleoside. In one embodiment, the nucleosides or nucleoside analogs are derivitized at the 5'- and/or the 3'-position by removal of a hydrogen from an hydroxyl group to incorporate a phosphoramidate and cyclic phosphate group. In one embodiment, the nucleosides or nucleoside analogs are derivitized at the 5'- and/or the 2'-position by removal of a hydrogen from an hydroxyl group to incorporate a phosphoramidate and cyclic phosphate group. In one embodiment, phosphorodiamidates of nucleosides and nucleoside analogs are provided. In other embodiments, the compound is a *S*-pivaloyl-2-thioethyl phosphoroamidate or *S-*hydroxypivaloyl-2-thioethyl phosphoroamidate. In another embodiment, *S*-pivaloyl-2-thioethyl disulfide phosphoroamidate or *S*-2,2-dimethyl-3-oxopropanoate 2-thioethyl benzyl phosphoroamidate or benzylphosphoramidate of nucleosides and nucleoside analogs are provided. In other embodiments, the derivative is 1-methyl-2-nitro-1H-imidazol-5-methoxymethyl *N*- benzylphosphoramidate or 5-nitrofuran-2-methoxymethyl *N*-benzyl phosphoramidate. In one embodiment the derivative is a 3', 5'-cyclic phosphate. In another embodiment the derivative is a 2', 5'-cyclic phosphate.

Phosphoroamidate and cyclic phosphate compounds of a variety of anti-cancer agents are provided. The anti-cancer agents include, but are not limited to, modified nucleosides. In another embodiment, the anti-cancer agent is clofarabine, cytarabine, isocladribine, cladribine, fludarabine or nelarabine. In another embodiment, the anti-cancer agent is clofarabine, cytarabine, isocladribine, cladribine or fludarabine. In another embodiment, the anti-cancer agent is clofarabine or cytarabine.

### DETAILED DESCRIPTION OF THE INVENTION

The invention is defined by the appendant claims. The description that follows is subject to this limitation. All aspects and embodiments which are not covered by the claims are merely aspects of the present disclosure and do not form part of the invention.

### Definitions

To facilitate understanding of the disclosure set forth herein, a number of terms are defined below.

Generally, the nomenclature used herein and the laboratory procedures in organic chemistry, medicinal chemistry, and pharmacology described herein are those well known and commonly employed in the art. Unless defined otherwise, all technical and scientific terms used herein generally have the same meaning as commonly understood by one of ordinary skill in the art to which this disclosure belongs.

The term "alkyl", as used herein, unless otherwise specified, refers to a saturated straight or branched hydrocarbon. In one embodiment, the alkyl group is a primary, secondary, or tertiary hydrocarbon. In one embodiment, the alkyl group includes one to ten carbon atoms, *i.e.,* C₁ to C₁₀ alkyl. In one embodiment, the alkyl group is methyl, CF₃, CCl₃, CFCl₂, CF₂Cl, ethyl, CH₂CF₃, CF₂CF₃, propyl, isopropyl, butyl, isobutyl, *sec*-butyl, *t-*butyl, pentyl, isopentyl, neopentyl, hexyl, isohexyl, 3-methylpentyl, 2,2-dimethylbutyl, or 2,3-dimethylbutyl. The term includes both substituted and unsubstituted alkyl groups, including halogenated alkyl groups, preferably unsubstituted or halogenated alkyl groups. In one embodiment, the alkyl group is a fluorinated alkyl group. Non-limiting examples of moieties with which the alkyl group can be substituted include halogen (fluoro, chloro, bromo, or iodo), oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

The term "lower alkyl", as used herein, and unless otherwise specified, refers to a saturated straight or branched hydrocarbon having one to six carbon atoms, *i.e.,* C₁ to C₆ alkyl. In one embodiment, the lower alkyl group is a primary, secondary, or tertiary hydrocarbon. The term includes both substituted and unsubstituted moieties, preferably unsubstituted

The term "cycloalkyl", as used herein, unless otherwise specified, refers to a saturated cyclic hydrocarbon. In one embodiment, the cycloalkyl group may be a saturated, and/or bridged, and/or non-bridged, and/or a fused bicyclic group. In one embodiment, the cycloalkyl group includes three to ten carbon atoms, *i.e.,* C₃ to C₁₀ cycloalkyl. In some embodiments, the cycloalkyl has from 3 to 15 (C₃₋₁₅), from 3 to 10 (C₃₋₁₀), or from 3 to 7 (C₃₋₇) carbon atoms. In one embodiment, the cycloalkyl group is cyclopropyl, cyclobutyl, cyclopentyl, cyclopentylmethyl, cyclohexyl, cyclohexylmethyl, cycloheptyl, bicyclo[2.1.1]hexyl, bicyclo[2.2.1]heptyl, decalinyl, or adamantyl. The term includes both substituted and unsubstituted cycloalkyl groups, including halogenated cycloalkyl groups. Non-limiting examples of moieties with which the cycloalkyl group can be substituted include halogen (fluoro, chloro, bromo, or iodo), oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference. In preferred embodiments, the cycloalkyl is unsubstituted or substituted with one or more halogens.

"Alkenyl" refers to monovalent olefinically unsaturated hydrocarbon groups, in certain embodiment, having up to about 11 carbon atoms, from 2 to 8 carbon atoms, or from 2 to 6 carbon atoms, which can be straight-chained or branched and having at least 1 or from 1 to 2 sites of olefinic unsaturation. The term "alkenyl" embraces radicals having a "*cis*" or "*trans*" configuration or a mixture thereof, or alternatively, a "Z" or "E" configuration or a mixture thereof, as appreciated by those of ordinary skill in the art. For example, C₂₋₆ alkenyl refers to a linear unsaturated monovalent hydrocarbon radical of 2 to 6 carbon atoms or a branched unsaturated monovalent hydrocarbon radical of 3 to 6 carbon atoms. In certain embodiments, the alkenyl is a linear monovalent hydrocarbon radical of 2 to 20 (C₂₋₂₀), 2 to 15 (C₂₋₁₅), 2 to 10 (C₂₋₁₀), or 2 to 6 (C₂₋₆) carbon atoms, or a branched monovalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. Examples of alkenyl groups include, but are not limited to, ethenyl, propen-1-yl, propen-2-yl, allyl, butenyl, and 4-methylbutenyl. The term includes both substituted and unsubstituted alkenylene groups, including halogenated cycloalkyl groups, preferably unsubstituted or halogenated cycloalkyl. Non-limiting examples of moieties with which the alkenylene group can be substituted include halogen (fluoro, chloro, bromo, or iodo), oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

The term "cycloalkenyl", as used herein, unless otherwise specified, refers to an unsaturated cyclic hydrocarbon and includes both substituted and unsubstituted cycloalkenyl groups, preferably unsubstituted. Non-limiting examples of moieties with which the cycloalkenyl group can be substituted include halogen (fluoro, chloro, bromo, or iodo), oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

The term "alkenylene" refers to a linear or branched divalent hydrocarbon radical, which contains one or more, in one embodiment, one, two, three, four, or five, in another embodiment, one or two, carbon-carbon double bond(s). The term "alkenylene" embraces radicals having a "*cis*" or "*trans*" configuration or a mixture thereof, or alternatively, a "Z" or "E" configuration or a mixture thereof, as appreciated by those of ordinary skill in the art. For example, C₂₋₆ alkenylene refers to a linear unsaturated divalent hydrocarbon radical of 2 to 6 carbon atoms or a branched unsaturated divalent hydrocarbon radical of 3 to 6 carbon atoms. In certain embodiments, the alkenylene is a linear divalent hydrocarbon radical of 2 to 20 (C₂₋₂₀), 2 to 15 (C₂₋₁₅), 2 to 10 (C₂₋₁₀), or 2 to 6 (C₂₋₆) carbon atoms, or a branched divalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. Examples of alkenylene groups include, but are not limited to, ethenylene, allylene, propenylene, butenylene, and 4-methylbutenylene. The term includes both substituted and unsubstituted groups, including halogenated groups, preferably unsubstituted. Non-limiting examples of moieties with which the alkenylene group can be substituted include halogen (fluoro, chloro, bromo, or iodo), oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

"Alkynyl" refers to acetylenically unsaturated hydrocarbon groups, in certain embodiments, having up to about 11 carbon atoms or from 2 to 6 carbon atoms which can be straight-chained or branched and having at least 1 or from 1 to 2 sites of alkynyl unsaturation. Non-limiting examples of alkynyl groups include acetylenic, ethynyl (-C≡CH), propargyl (-CH₂C≡CH), and the like. The term also includes both substituted and unsubstituted alkynyl groups, preferably unsubstituted. Non-limiting examples of moieties with which the alkynyl group can be substituted include halogen (fluoro, chloro, bromo, or iodo), oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

The term "aryl", as used herein, and unless otherwise specified, refers to phenyl, biphenyl, or naphthyl. The term includes both substituted and unsubstituted moieties. An aryl group can be substituted with any described moiety, including, but not limited to, one or more moieties selected from halogen (fluoro, chloro, bromo, or iodo), alkyl, haloalkyl, alkenyl, alkynyl, cycloalkyl, cycloakenyl, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991.

"Alkoxy" refers to the group -OR' where R' is alkyl or cycloalkyl where alkyl and cycloalkyl are as defined herein. Alkoxy groups include, by way of example, methoxy, ethoxy, n-propoxy, isopropoxy, n-butoxy, tert-butoxy, sec-butoxy, n-pentoxy, n-hexoxy, 1,2-dimethylbutoxy, and the like.

"Aryloxy" refers to the group -OR' where R' is aryl or heteroaryl where aryl and heteroaryl are as defined herein.

"Alkoxycarbonyl" refers to a radical -C(O)-alkoxy where alkoxy is as defined herein.

"Amino" refers to the radical -NH₂.

"Carboxyl" or "carboxy" refers to the radical -C(O)OH. "Carbonyl" refers to the radical - C(O)-.

The term "alkylamino," "arylamino," or "alkylarylamino" refers to an amino group that has one or two alkyl substituents (-NHR' or -NR'R', where R' is alkyl as defined herein), one or two aryl substituents (-NHR' or -NR'R', where R' is aryl as defined herein), or one alkyl substituent and one aryl substituent (-NR'R", where one of R' and R" is alkyl as defined herein and the other is aryl as defined herein), respectively. In one embodiment, the alkyl substituent is lower alkyl. In another embodiment, the alkyl or lower alkyl is unsubstituted.

"Halogen" or "halo" refers to fluoro, chloro, bromo, or iodo.

"Oxo" refers to =O or →O.

"Thioalkoxy" refers to the group -SR' where R' is alkyl or cycloalkyl each of which is as defined herein. "Thioaryloxy" refers to the group -SR' where R' is aryl or heteroaryl each of which is as defined herein.

"Alkyldisulfanyl" refers to the group R'-S-S-, where R' is alkyl or cycloalkyl each of which is as defined herein.

The term "heterocyclyl" or "heterocyclic" refers to a monovalent monocyclic non-aromatic ring system and/or multicyclic ring system that contains at least one non-aromatic ring, wherein one or more of the non-aromatic ring atoms are heteroatoms independently selected from O, S, or N; and the remaining ring atoms are carbon atoms. In certain embodiments, the heterocyclyl or heterocyclic group has from 3 to 20, from 3 to 15, from 3 to 10, from 3 to 8, from 4 to 7, or from 5 to 6 ring atoms. Heterocyclyl groups are bonded to the rest of the molecule through the non-aromatic ring. In certain embodiments, the heterocyclyl is a monocyclic, bicyclic, tricyclic, or tetracyclic ring system, which may include a fused or bridged ring system, and in which the nitrogen or sulfur atoms may be optionally oxidized, the nitrogen atoms may be optionally quaternized, and some rings may be partially or fully saturated, or aromatic. The heterocyclyl may be attached to the main structure at any heteroatom or carbon atom which results in the creation of a stable compound. Examples of such heterocyclic radicals include, but are not limited to, azepinyl, benzodioxanyl, benzodioxolyl, benzofuranonyl, benzopyranonyl, benzopyranyl, benzotetrahydrofuranyl, benzotetrahydrothienyl, benzothiopyranyl, benzoxazinyl, β-carbolinyl, chromanyl, chromonyl, cinnolinyl, coumarinyl, decahydroisoquinolinyl, dihydrobenzisothiazinyl, dihydrobenzisoxazinyl, dihydrofuryl, dihydroisoindolyl, dihydropyranyl, dihydropyrazolyl, dihydropyrazinyl, dihydropyridinyl, dihydropyrimidinyl, dihydropyrrolyl, dioxolanyl, 1,4-dithianyl, furanonyl, imidazolidinyl, imidazolinyl, indolinyl, isobenzotetrahydrofuranyl, isobenzotetrahydrothienyl, isochromanyl, isocoumarinyl, isoindolinyl, isothiazolidinyl, isoxazolidinyl, morpholinyl, octahydroindolyl, octahydroisoindolyl, oxazolidinonyl, oxazolidinyl, oxiranyl, piperazinyl, piperidinyl, 4-piperidonyl, pyrazolidinyl, pyrazolinyl, pyrrolidinyl, pyrrolinyl, quinuclidinyl, tetrahydrofuryl, tetrahydroisoquinolinyl, tetrahydropyranyl, tetrahydrothienyl, thiamorpholinyl, thiazolidinyl, tetrahydroquinolinyl, and 1,3,5-trithianyl. The term also includes both substituted and unsubstituted heterocyclyl groups. Non-limiting examples of moieties with which the heterocyclyl group can be substituted include halogen (fluoro, chloro, bromo, or iodo), oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

The term "heteroaryl" refers to a monovalent monocyclic aromatic group and/or multicyclic aromatic group that contain at least one aromatic ring, wherein at least one aromatic ring contains one or more heteroatoms independently selected from O, S, and N in the ring. Heteroaryl groups are bonded to the rest of the molecule through the aromatic ring. Each ring of a heteroaryl group can contain one or two O atoms, one or two S atoms, and/or one to four N atoms, provided that the total number of heteroatoms in each ring is four or less and each ring contains at least one carbon atom. In certain embodiments, the heteroaryl has from 5 to 20, from 5 to 15, or from 5 to 10 ring atoms. Examples of monocyclic heteroaryl groups include, but are not limited to, furanyl, imidazolyl, isothiazolyl, isoxazolyl, oxadiazolyl, oxadiazolyl, oxazolyl, pyrazinyl, pyrazolyl, pyridazinyl, pyridyl, pyrimidinyl, pyrrolyl, thiadiazolyl, thiazolyl, thienyl, tetrazolyl, triazinyl, and triazolyl. Examples of bicyclic heteroaryl groups include, but are not limited to, benzofuranyl, benzimidazolyl, benzoisoxazolyl, benzopyranyl, benzothiadiazolyl, benzothiazolyl, benzothienyl, benzotriazolyl, benzoxazolyl, furopyridyl, imidazopyridinyl, imidazothiazolyl, indolizinyl, indolyl, indazolyl, isobenzofuranyl, isobenzothienyl, isoindolyl, isoquinolinyl, isothiazolyl, naphthyridinyl, oxazolopyridinyl, phthalazinyl, pteridinyl, purinyl, pyridopyridyl, pyrrolopyridyl, quinolinyl, quinoxalinyl, quinazolinyl, thiadiazolopyrimidyl, and thienopyridyl. Examples of tricyclic heteroaryl groups include, but are not limited to, acridinyl, benzindolyl, carbazolyl, dibenzofuranyl, perimidinyl, phenanthrolinyl, phenanthridinyl, phenarsazinyl, phenazinyl, phenothiazinyl, phenoxazinyl, and xanthenyl. A heteroaryl group can be substituted with any described moiety, including, but not limited to, one or more moieties selected from halogen (fluoro, chloro, bromo, or iodo), oxo, alkyl, haloalkyl, alkenyl, alkynyl, cycloalkyl, cycloakenyl, hydroxyl, amino, alkylamino, arylamino, alkylarylamino, alkoxy, aryloxy, thioalkoxy, thioaryloxy, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991.

The term "alkylaryl" refers to an aryl group with an alkyl substituent. The term "aralkyl" or "arylalkyl" includes an alkyl group with an aryl substituent.

The term "alkylheterocyclyl" refers to a heterocyclyl group with an alkyl substituent. The term "alkylheterocyclyl" includes an alkyl group with a heterocyclyl substituent.

The term "alkylheteroaryl" refers to a heteroaryl group with an alkyl substituent. The term "alkylheteroaryl" includes an alkyl group with a heteroaryl substituent.

The term "protecting group" as used herein and unless otherwise defined refers to a group that is added to an oxygen, nitrogen, or phosphorus atom to prevent its further reaction or for other purposes. A wide variety of oxygen and nitrogen protecting groups are known to those skilled in the art of organic synthesis.

"Pharmaceutically acceptable salt" refers to any salt of a compound provided herein which retains its biological properties and which is not toxic or otherwise undesirable for pharmaceutical use. Such salts may be derived from a variety of organic and inorganic counter-ions well known in the art. Such salts include, but are not limited to: (1) acid addition salts formed with organic or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, *tert*-butylacetic, lauryl sulfuric, gluconic, benzoic, glutamic, hydroxynaphthoic, salicylic, stearic, cyclohexylsulfamic, quinic, muconic acid and the like acids; or (2) salts formed when an acidic proton present in the parent compound either (a) is replaced by a metal ion, *e.g.,* an alkali metal ion, an alkaline earth ion or an aluminum ion, or alkali metal or alkaline earth metal hydroxides, such as sodium, potassium, calcium, magnesium, aluminum, lithium, zinc, and barium hydroxide, ammonia, or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, *N*-methylglucamine piperazine, tris(hydroxymethyl)-aminomethane, tetramethylammonium hydroxide, and the like.

Pharmaceutically acceptable salts further include, by way of example only and without limitation, sodium, potassium, calcium, magnesium, ammonium, tetraalkylammonium, and the like, and when the compound contains a basic functionality, salts of non-toxic organic or inorganic acids, such as hydrohalides, *e*.*g*. hydrochloride and hydrobromide, sulfate, phosphate, sulfamate, nitrate, acetate, trifluoroacetate, trichloroacetate, propionate, hexanoate, cyclopentylpropionate, glycolate, glutarate, pyruvate, lactate, malonate, succinate, sorbate, ascorbate, malate, maleate, fumarate, tartarate, citrate, benzoate, 3-(4-hydroxybenzoyl)benzoate, picrate, cinnamate, mandelate, phthalate, laurate, methanesulfonate (mesylate), ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1 -carboxylate, glucoheptonate, 3-phenylpropionate, trimethylacetate, *tert*-butylacetate, lauryl sulfate, gluconate, benzoate, glutamate, hydroxynaphthoate, salicylate, stearate, cyclohexylsulfamate, quinate, muconate, and the like.

The term "purine" or "pyrimidine" base refers to, but is not limited to, adenine, guanine, adenine, hypoxanthine, 7-deazaguanine, 7-deazaadenine, 2,6-diaminopurine, 6-chloropurine, N⁶-alkylpurines, N⁶-acylpurines (wherein acyl is C(O)(alkyl, aryl, alkylaryl, or arylalkyl), N⁶-benzylpurine, 6-halopurine, N⁶-vinylpurine, N⁶-acetylenic purine, N⁶-hydroxyalkyl purine, N⁶-alkylaminopurine, N⁶-thioalkyl purine, N²-alkylpurines, N²-alkyl-6-thiopurines, thymine, cytosine, 5-fluorocytosine, 5-methylcytosine, 6-azapyrimidine, including 6-azacytosine, 2- and/or 4-mercaptopyrmidine, uracil, 5-halouracil, including 5-fluorouracil, C⁵-alkylpyrimidines, C⁵-benzylpyrimidines, C⁵-halopyrimidines, C⁵-vinylpyrimidine, C⁵-acetylenic pyrimidine, C⁵-acyl pyrimidine, C⁵-hydroxyalkyl purine, C⁵-amidopyrimidine, C⁵-cyanopyrimidine, C⁵-iodopyrimidine, C⁶-iodo-pyrimidine, C⁵-Br-vinyl pyrimidine, C⁶-Br-vinyl pyrimidine, C⁵-nitropyrimidine, C⁵-amino-pyrimidine, 5-azacytidinyl, 5-azauracilyl, triazolopyridinyl, imidazolopyridinyl, pyrrolopyrimidinyl, and pyrazolopyrimidinyl. Functional oxygen and nitrogen groups on the base can be protected as necessary or desired. Suitable protecting groups are well known to those skilled in the art, and include trimethylsilyl, dimethylhexylsilyl, *t*-butyldimethylsilyl, and *t-*butyldiphenylsilyl, trityl, alkyl groups, acyl groups such as acetyl and propionyl, methanesulfonyl, and p-toluenesulfonyl.

The term "acyl" or "O-linked ester" refers to a group of the formula -OC(O)R', wherein R' is alkyl or cycloalkyl (including lower alkyl), carboxylate reside of amino acid, aryl including phenyl, alkaryl, arylalkyl including benzyl, alkoxyalkyl including methoxymethyl, aryloxyalkyl such as phenoxymethyl. For example, R' can be substituted alkyl (including lower alkyl), aryl including phenyl optionally substituted with chloro, bromo, fluoro, iodo, C₁ to C₄ alkyl or to C₄ alkoxy, sulfonate esters such as alkyl or arylalkyl sulphonyl including methanesulfonyl, the mono, di or triphosphate ester, trityl or monomethoxy-trityl, substituted benzyl, alkaryl, arylalkyl including benzyl, alkoxyalkyl including methoxymethyl, aryloxyalkyl such as phenoxymethyl. Aryl groups in the esters optimally comprise a phenyl group. In particular, acyl groups include acetyl, trifluoroacetyl, methylacetyl, cyclpropylacetyl, propionyl, butyryl, hexanoyl, heptanoyl, octanoyl, neo-heptanoyl, phenylacetyl, 2-acetoxy-2-phenylacetyl, diphenylacetyl, α-methoxy-α-trifluoromethyl-phenylacetyl, bromoacetyl, 2-nitro-benzeneacetyl, 4-chloro-benzeneacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, methoxyacetyl, 2-thiopheneacetyl, chlorosulfonylacetyl, 3-methoxyphenylacetyl, phenoxyacetyl, tert-butylacetyl, trichloroacetyl, monochloro-acetyl, dichloroacetyl, 7H-dodecafluoro-heptanoyl, perfluoro-heptanoyl, 7H-dodeca-fluoroheptanoyl, 7-chlorododecafluoro-heptanoyl, 7-chloro-dodecafluoro-heptanoyl, 7H-dodecafluoroheptanoyl, 7H-dodecafluoroheptanoyl, nona-fluoro-3,6-dioxa-heptanoyl, nonafluoro-3,6-dioxaheptanoyl, perfluoroheptanoyl, methoxybenzoyl, methyl 3-amino-5-phenylthiophene-2-carboxyl, 3,6-dichloro-2-methoxy-benzoyl, 4-(1,1,2,2-tetrafluoro-ethoxy)-benzoyl, 2-bromo-propionyl, omega-aminocapryl, decanoyl, n-pentadecanoyl, stearyl, 3-cyclopentyl-propionyl, 1-benzene-carboxyl, O-acetylmandelyl, pivaloyl acetyl, 1-adamantane-carboxyl, cyclohexane-carboxyl, 2,6-pyridinedicarboxyl, cyclopropane-carboxyl, cyclobutane-carboxyl, perfluorocyclohexyl carboxyl, 4-methylbenzoyl, chloromethyl isoxazolyl carbonyl, perfluorocyclohexyl carboxyl, crotonyl, 1-methyl-1H-indazole-3-carbonyl, 2-propenyl, isovaleryl, 1-pyrrolidinecarbonyl, 4-phenylbenzoyl.

The term "amino acid" refers to naturally occurring and synthetic α, β, γ, or δ amino acids, and includes but is not limited to, amino acids found in proteins, *i.e.* glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine. In one embodiment, the amino acid is in the L-configuration. Alternatively, the amino acid can be a derivative of alanyl, valinyl, leucinyl, isoleuccinyl, prolinyl, phenylalaninyl, tryptophanyl, methioninyl, glycinyl, serinyl, threoninyl, cysteinyl, tyrosinyl, asparaginyl, glutaminyl, aspartoyl, glutaroyl, lysinyl, argininyl, histidinyl, β-alanyl, β-valinyl, β-leucinyl, β-isoleuccinyl, β-prolinyl, β-phenylalaninyl, β-tryptophanyl, β-methioninyl, β-glycinyl, β-serinyl, β-threoninyl, β-cysteinyl, β-tyrosinyl, β-asparaginyl, β-glutaminyl, β-aspartoyl, β-glutaroyl, β-lysinyl, β-argininyl, or β-histidinyl.

The term "substantially free of" or "substantially in the absence of" with respect to a nucleoside composition refers to a nucleoside composition that includes at least 85 or 90% by weight, in certain embodiments 95%, 98 % , 99%, or 100% by weight, of the designated nucleoside, the designated diastereomer of such nucleoside, or the designated enantiomer of such nucleoside. In one embodiment, in the uses and compounds provided herein, the compounds are substantially free of other compounds, other nucleosides, other diastereomers, or other enantiomers that are not designated.

Similarly, the term "isolated" with respect to a nucleoside composition refers to a nucleoside composition that includes at least 85, 90%, 95%, 98%, 99%, to 100% by weight, of the nucleoside, the remainder comprising other chemical species or enantiomers.

"Solvate" refers to a compound provided herein or a salt thereof, that further includes a stoichiometric or non-stoichiometric amount of solvent bound by non-covalent intermolecular forces. Where the solvent is water, the solvate is a hydrate.

"Isotopic composition" refers to the amount of each isotope present for a given atom, and "natural isotopic composition" refers to the naturally occuring isotopic composition or abundance for a given atom. Atoms containing their natural isotopic composition may also be referred to herein as "non-enriched" atoms. Unless otherwise designated, the atoms of the compounds recited herein are meant to represent any stable isotope of that atom. For example, unless otherwise stated, when a position is designated specifically as "H" or "hydrogen", the position is understood to have hydrogen at its natural isotopic composition.

"Isotopic enrichment" refers to the percentage of incorporation of an amount of a specific isotope at a given atom in a molecule in the place of that atom's natural isotopic abundance. For example, deuterium enrichment of 1% at a given position means that 1% of the molecules in a given sample contain deuterium at the specified position. Because the naturally occurring distribution of deuterium is about 0.0156%, deuterium enrichment at any position in a compound synthesized using non-enriched starting materials is about 0.0156%. The isotopic enrichment of the compounds provided herein can be determined using conventional analytical methods known to one of ordinary skill in the art, including mass spectrometry and nuclear magnetic resonance spectroscopy.

"Isotopically enriched" refers to an atom having an isotopic composition other than the natural isotopic composition of that atom. "Isotopically enriched" may also refer to a compound containing at least one atom having an isotopic composition other than the natural isotopic composition of that atom.

As used herein, "alkyl," "cycloalkyl," "alkenyl," "cycloalkenyl," "alkynyl," "aryl," "alkoxy," "alkoxycarbonyl," "amino," "carboxyl," "alkylamino," "arylamino," "thioalkyoxy," "heterocyclyl," "heteroaryl," "alkylheterocyclyl," "alkylheteroaryl," "acyl," "aralkyl," "alkaryl," "purine," "pyrimidine," "carboxyl," and "amino acid" groups optionally comprise deuterium at one or more positions where hydrogen atoms are present, and wherein the deuterium composition of the atom or atoms is other than the natural isotopic composition.

Also as used herein, "alkyl," "cycloalkyl," "alkenyl," "cycloalkenyl," "alkynyl," "aryl," "alkoxy," "alkoxycarbonyl," "carboxyl," "alkylamino," "arylamino," "thioalkyoxy," "heterocyclyl," "heteroaryl," "alkylheterocyclyl," "alkylheteroaryl," "acyl," "aralkyl," "alkaryl," "purine," "pyrimidine," "carboxyl," and "amino acid" groups optionally comprise carbon-13 at an amount other than the natural isotopic composition.

Further, "alkyl," "cycloalkyl," "alkenyl," "cycloalkenyl," "alkynyl," "aryl," "heterocyclyl," and "heteroaryl" groups that are included as examples of moieties with which a certain functional group can be substituted can further be substituted.

The term "proliferative disorder or disease" refers to unwanted cell proliferation of one or more subset of cells in a multicellular organism resulting in harm (i.e., discomfort or decreased life expectancy) to the multicellular organisms. A proliferative disorder or disease can occur in different types of animals and humans. For example, as used herein, "proliferative disorder or disease" includes neoplastic disorders and other proliferative disorders.

The term "neoplastic disorder or disease" or "cancer" refers to a tumor resulting from abnormal or uncontrolled cellular growth. Examples of neoplastic disorders include, but are not limited to, hematopoietic disorders, such as the myeloproliferative disorders, thrombocythemia, essential thrombocytosis (ET), angiogenic myeloid metaplasia, myelofibrosis (MF), myelofibrosis with myeloid metaplasia (MMM), chronic idiopathic myelofibrosis (IMF), polycythemia vera (PV), the cytopenias, and pre-malignant myelodysplastic syndromes; cancers, such as glioma cancers, lung cancers, breast cancers, colorectal cancers, prostate cancers, gastric cancers, esophageal cancers, colon cancers, pancreatic cancers, ovarian cancers, and hematologic malignancies.

The term "hematologic malignancy" refers to cancer of the body's blood-forming and immune system-the bone marrow and lymphatic tissue. Examples of hematological malignancies include, for instance, myelodysplasia, lymphomas, leukemias, lymphomas (non-Hodgkin's lymphoma), Hodgkin's disease (also called Hodgkin's lymphoma), and myeloma, such as acute lymphocytic leukemia (ALL), acute myeloid leukemia (AML), acute promyelocytic leukemia (APL), chronic lymphocytic leukemia (CLL), chronic myeloid leukemia (CML), chronic neutrophilic leukemia (CNL), acute undifferentiated leukemia (AUL), anaplastic large-cell lymphoma (ALCL), prolymphocytic leukemia (PML), juvenile myelomonocyctic leukemia (JMML), adult T-cell ALL, AML with trilineage myelodysplasia (AML/TMDS), mixed lineage leukemia (MLL), myelodysplastic syndromes (MDSs), myeloproliferative disorders (MPD), and multiple myeloma (MM).

The term "leukemia" refers to malignant neoplasms of the blood-forming tissues, including, but not limited to, chronic lymphocytic leukemia, chronic myelocytic leukemia, acute lymphoblastic leukemia, acute myeloid leukemia and acute myeloblastic leukemia. The leukemia can be relapsed, refractory, or resistant to conventional therapy.

The term "relapsed" refers to a situation where a subject or a mammal, who has had a remission of cancer after therapy, has a return of cancer cells.

The term "refractory or resistant" refers to a circumstance where a subject or a mammal, even after intensive treatment, has residual cancer cells in his body.

The term "drug resistance" refers to the condition when a disease does not respond to the treatment of a drug or drugs. Drug resistance can be either intrinsic, which means the disease has never been responsive to the drug or drugs, or it can be acquired, which means the disease ceases responding to a drug or drugs that the disease had previously responded to. In certain embodiments, drug resistance is intrinsic. In certain embodiments, the drug resistance is acquired.

As used herein, the term "EC50" refers to a dosage, concentration or amount of a particular test compound that elicits a dose-dependent response at 50% of maximal expression of a particular response that is induced, provoked or potentiated by the particular test compound.

As used herein, the term "Emax" refers to an amount, concentration or dosage of a particular test compound that achieves a 100% inhibition of a maximal response in an assay that measures such response.

As used herein, the term "Eo" refers to an amount, concentration or dosage of a particular test compound that achieves a 0% inhibition of a maximal response in an assay that measures such response.

As used herein, the terms "subject" and "patient" are used interchangeably herein. The terms "subject" and "subjects" refer to an animal, such as a mammal including a non-primate (e.g., a cow, pig, horse, cat, dog, rat, and mouse) and a primate (e.g., a monkey such as a cynomolgous monkey, a chimpanzee and a human), and for example, a human. In another embodiment, the subject is a farm animal (e.g., a horse, a cow, a pig, etc.) or a pet (e.g., a dog or a cat). In one embodiment, the subject is a human.

As used herein, the terms "drug," "therapeutic agent," and "chemotherapeutic agent" refer to any agent(s), compound, or pharmaceutical composition thereof, which can be used in the treatment or prevention of a disorder or one or more symptoms thereof. In certain embodiments, the term "therapeutic agent" includes a compound provided herein. In one embodiment, a therapeutic agent is an agent which is known to be useful for, or has been or is currently being used for the treatment or prevention of a disorder or one or more symptoms thereof.

"Therapeutically effective amount" refers to an amount of a compound or composition that, when administered to a subject for treating a disease, is sufficient to effect such treatment for the disease. A "therapeutically effective amount" can vary depending on, inter alia, the compound, the disease and its severity, and the age, weight, etc., of the subject to be treated.

"Treating" or "treatment" of any disease or disorder refers, in one embodiment, to ameliorating a disease or disorder that exists in a subject. In another embodiment, "treating" or "treatment" includes ameliorating at least one physical parameter, which may be indiscernible by the subject. In yet another embodiment, "treating" or "treatment" includes modulating the disease or disorder, either physically (*e*.*g*., stabilization of a discernible symptom) or physiologically (*e*.*g*., stabilization of a physical parameter) or both. In yet another embodiment, "treating" or "treatment" includes delaying the onset of the disease or disorder.

As used herein, the terms "prophylactic agent" and "prophylactic agents" as used refer to any agent(s) which can be used in the prevention of a disorder or one or more symptoms thereof. In certain embodiments, the term "prophylactic agent" includes a compound provided herein. In certain other embodiments, the term "prophylactic agent" does not refer to a compound provided herein. For example, a prophylactic agent is an agent which is known to be useful for, or has been or is currently being used to prevent or impede the onset, development, progression and/or severity of a disorder.

As used herein, the phrase "prophylactically effective amount" refers to the amount of a therapy (*e*.*g*., prophylactic agent) which is sufficient to result in the prevention or reduction of the development, recurrence or onset of one or more symptoms associated with a disorder or to enhance or improve the prophylactic effect(s) of another therapy (*e*.*g*., another prophylactic agent).

### Compounds

Prodrug compounds of a variety of therapeutic agents can be formed using methods available in the art and those disclosed herein. The therapeutic agent can be derivatized to include a reactive group for attachment of the phosphate moiety. Such therapeutic agent includes but is not limited to nucleosides and nucleoside analogues including acyclic nucleosides.

As used herein, a "phosphoramidate or cyclic phosphate compound of a therapeutic agent" includes a therapeutic agent derivatized to include a phosphoramidate or cyclic phosphate group. The therapeutic agent is, for example, an anti-cancer agent that includes, or has been derivatized to include, a reactive group, such as a hydroxyl, for attachment of the phosphoroamidate and cyclic phosphate moiety. Such therapeutic agents include, but are not limited to nucleosides and nucleoside analogs including acyclic nucleosides. A nucleoside analog refers to a structurally modified nucleoside. In one embodiment, the nucleosides or nucleoside analogs are derivitized at the 5'- and/or the 3'-position by removal of a hydrogen from an hydroxyl group to include a phosphate moiety comprising a phosphoramidate and a cyclic phosphate group. In another embodiment, the nucleosides or nucleoside analogs are derivitized at the 5'- and/or the 2'-position by removal of a hydrogen from an hydroxyl group to include a phosphate moiety comprising a phosphoramidate and a cyclic phosphate group.

Modified phosphate derivatives comprising phosphoramidate and cyclic phosphate derivatives of nucleoside analogues comprising natural and non-natural nucleosides described herein can be formed as described herein and used for the treatment of cancer. In one embodiment, the derivative moiety can be at the 3' position. In another embodiment, the derivative moiety can be at the 5' position. In another embodiment, the derivative moiety can be at the 3', 5' positions linked together to form a cyclic derivative. In one embodiment, the prodrug moiety can be at the 2', 5' positions linked together.

In one aspect of the invention, there is provided a compound having the Formula: 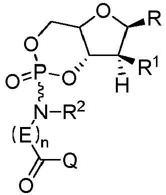 or a pharmaceutically acceptable salt thereof, wherein:
R is 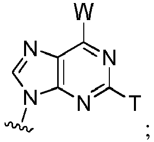
W is NH₂;
T is F or Cl;
R¹ is F;
Q is OR³;
E is CR⁴R⁵;
n is 1;
R² is hydrogen; R³ is alkyl; R⁴ is hydrogen and R⁵ is alkyl, or cycloalkyl; or R⁴ is alkyl or cycloalkyl, and R⁵ is hydrogen,
wherein alkyl is optionally substituted by halogen, oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate;
cycloalkyl is optionally substituted by halogen, oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate;
alkyl is to C₁₀ alkyl; and
cycloalkyl is C₃ to C₁₀ cycloalkyl.

In one embodiment, there is provided a compound of the following Formula:

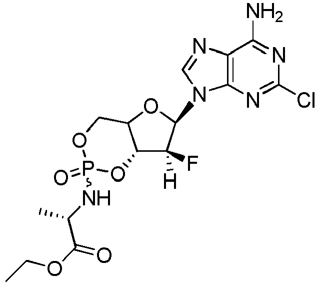

In a further embodiment, there is provided a compound selected from:
ethyl(2S)-2-[[(4aR,6R,7S,7aR)-6-(6-amino-2-chloro-purin-9-yl)-7-fluoro-2-oxo-4a,6,7,7a-tetrahydro-4H-furo[3,2-d][1,3,2]dioxaphosphinin-2-yl]amino]propanoate (diastereomer 1 and 2), or a pharmaceutically acceptable salt thereof, and;
ethyl (2S)-2-[[(4aR,6R,7S,7aS)-6-(6-amino-2-fluoro-purin-9-yl)-7-hydroxy-2-oxo-4a,6,7,7a-tetrahydro-4H-furo[3,2-d][1,3,2]dioxaphosphinin-2-yl]amino]propanoate (diastereomer 1 and 2), or a pharmaceutically acceptable salt thereof.

In one aspect of the invention, there is provided a pharmaceutical composition comprising a compound of the invention, or a pharmaceutically acceptable salt thereof and a pharmaceutically acceptable excipient, carrier, or diluents. In one embodiment, the composition is an oral formulation.
In one aspect of the invention, there is provided a compound of the invention, or a pharmaceutically acceptable salt thereof, for use in therapy.

In one aspect of the invention, there is provided a compound of the invention, or a pharmaceutically acceptable salt thereof for use in the treatment of a blood cancer. In one embodiment, the blood cancer is a leukemia cancer. In one embodiment, the leukemia is acute myelogenous leukemia (AML).
In one aspect of the invention, there is provided a combination comprising a compound of the invention, or a pharmaceutically acceptable salt thereof and a second chemotherapeutic agent.
In one aspect of the invention, there is provided a combination comprising a compound of the invention, or a pharmaceutically acceptable salt thereof and one, two, three or more other therapeutic agents.

In some embodiments, when the phosphoramidate is substituted with at least one amino acid group, the amino acid is in the L-configuration. In one embodiment, the amino acid is alanine. In one embodiment, the amino acid is leucine.
In some embodiments, the phosphoramidate compound provided herein possesses a chiral phosphorous center. In some embodiments, the phosphoramidate is diastereomerically enriched.

### Optically Active Compounds

It is appreciated that compounds provided herein have several chiral centers and may exist in and be isolated in optically active and racemic forms. Some compounds may exhibit polymorphism. It is to be understood that any racemic, optically-active, diastereomeric, polymorphic, or stereoisomeric form, or mixtures thereof, of a compound provided herein, which possess the useful properties described herein is within the scope of the invention. It is well known in the art how to prepare optically active forms (for example, by resolution of the racemic form by recrystallization techniques, by synthesis from optically-active starting materials, by chiral synthesis, or by chromatographic separation using a chiral stationary phase).

In particular, since the 1' and 4' carbons of a nucleoside are chiral, their nonhydrogen substituents (the base and the CHOR groups, respectively) can be either cis (on the same side) or trans (on opposite sides) with respect to the sugar ring system. The four optical isomers therefore are represented by the following configurations (when orienting the sugar moiety in a horizontal plane such that the oxygen atom is in the back): cis (with both groups "up", which corresponds to the configuration of naturally occurring β-D nucleosides), cis (with both groups "down", which is a nonnaturally occurring β-L configuration), trans (with the C2' substituent "up" and the C4' substituent "down"), and trans (with the C2' substituent "down" and the C4' substituent "up"). The "D-nucleosides" are cis nucleosides in a natural configuration and the "L-nucleosides" are cis nucleosides in the non-naturally occurring configuration.

Likewise, most amino acids are chiral (designated as L or D, wherein the L enantiomer is the naturally occurring configuration) and can exist as separate enantiomers.

Examples of methods to obtain optically active materials are known in the art, and include at least the following.
i) physical separation of crystals - a technique whereby macroscopic crystals of the individual enantiomers are manually separated. This technique can be used if crystals of the separate enantiomers exist, *i*.*e*., the material is a conglomerate, and the crystals are visually distinct;
ii) simultaneous crystallization - a technique whereby the individual enantiomers are separately crystallized from a solution of the racemate, possible only if the latter is a conglomerate in the solid state;
iii) enzymatic resolutions - a technique whereby partial or complete separation of a racemate by virtue of differing rates of reaction for the enantiomers with an enzyme;
iv) enzymatic asymmetric synthesis - a synthetic technique whereby at least one step of the synthesis uses an enzymatic reaction to obtain an enantiomerically pure or enriched synthetic precursor of the desired enantiomer;
v) chemical asymmetric synthesis - a synthetic technique whereby the desired enantiomer is synthesized from an achiral precursor under conditions that produce asymmetry (*i*.*e*., chirality) in the product, which may be achieved using chiral catalysts or chiral auxiliaries;
vi) diastereomer separations - a technique whereby a racemic compound is reacted with an enantiomerically pure reagent (the chiral auxiliary) that converts the individual enantiomers to diastereomers. The resulting diastereomers are then separated by chromatography or crystallization by virtue of their now more distinct structural differences and the chiral auxiliary later removed to obtain the desired enantiomer;
vii) first- and second-order asymmetric transformations - a technique whereby diastereomers from the racemate equilibrate to yield a preponderance in solution of the diastereomer from the desired enantiomer or where preferential crystallization of the diastereomer from the desired enantiomer perturbs the equilibrium such that eventually in principle all the material is converted to the crystalline diastereomer from the desired enantiomer. The desired enantiomer is then released from the diastereomer;
viii) kinetic resolutions - this technique refers to the achievement of partial or complete resolution of a racemate (or of a further resolution of a partially resolved compound) by virtue of unequal reaction rates of the enantiomers with a chiral, non-racemic reagent or catalyst under kinetic conditions;
ix) enantiospecific synthesis from non-racemic precursors - a synthetic technique whereby the desired enantiomer is obtained from non-chiral starting materials and where the stereochemical integrity is not or is only minimally compromised over the course of the synthesis;
x) chiral liquid chromatography - a technique whereby the enantiomers of a racemate are separated in a liquid mobile phase by virtue of their differing interactions with a stationary phase. The stationary phase can be made of chiral material or the mobile phase can contain an additional chiral material to provoke the differing interactions;
xi) chiral gas chromatography - a technique whereby the racemate is volatilized and enantiomers are separated by virtue of their differing interactions in the gaseous mobile phase with a column containing a fixed non-racemic chiral adsorbent phase;
xii) extraction with chiral solvents - a technique whereby the enantiomers are separated by virtue of preferential dissolution of one enantiomer into a particular chiral solvent;
xii) transport across chiral membranes - a technique whereby a racemate is placed in contact with a thin membrane barrier. The barrier typically separates two miscible fluids, one containing the racemate, and a driving force such as concentration or pressure differential causes preferential transport across the membrane barrier. Separation occurs as a result of the non-racemic chiral nature of the membrane which allows only one enantiomer of the racemate to pass through.

In some embodiments, compositions of the present disclosure are provided that are substantially free of an undesignated enantiomer of a nucleoside or nucleoside derivative. In one embodiment, in the methods and compounds of this invention, the compounds are substantially free of undesignated enantiomers. In some embodiments, the composition includes a compound that is at least 85 %, 90%, 95%, 98%, 99%, to 100% by weight of the designated compound, the remainder comprising other chemical species or enantiomers.

### Isotopically Enriched Compounds

Also provided herein are isotopically enriched compounds, including but not limited to isotopically enriched nucleoside derivatives.

Isotopic enrichment (for example, deuteration) of pharmaceuticals to improve pharmacokinetics ("PK"), pharmacodynamics ("PD"), and toxicity profiles, has been demonstrated previously with some classes of drugs. See, for example, Lijinsky et. al., Food Cosmet. Toxicol., 20: 393 (1982); Lijinsky et. al., J. Nat. Cancer Inst., 69: 1127 (1982); Mangold et. al., Mutation Res. 308: 33 (1994); Gordon et. al., Drug Metab. Dispos., 15: 589 (1987); Zello et. al., Metabolism, 43: 487 (1994); Gately et. al., J. Nucl. Med., 27: 388 (1986); Wade D, Chem. Biol. Interact. 117: 191 (1999).

Isotopic enrichment of a drug can be used, for example, to (1) reduce or eliminate unwanted metabolites, (2) increase the half-life of the parent drug, (3) decrease the number of doses needed to achieve a desired effect, (4) decrease the amount of a dose necessary to achieve a desired effect, (5) increase the formation of active metabolites, if any are formed, and/or (6) decrese the production of deleterious metabolites in specific tissues and/or create a more effective drug and/or a safer drug for combination therapy, whether the combination therapy is intentional or not.

Replacement of an atom for one of its isotopes often will result in a change in the reaction rate of a chemical reaction. This phenomenon is known as the Kinetic Isotope Effect ("KIE"). For example, if a C-H bond is broken during a rate-determining step in a chemical reaction (i.e. the step with the highest transition state energy), substitution of a deuterium for that hydrogen will cause a decrease in the reaction rate and the process will slow down. This phenomenon is known as the Deuterium Kinetic Isotope Effect ("DKIE"). (*See, e.g,* Foster et al., Adv. Drug Res., vol. 14, pp. 1-36 (1985); Kushner et al., Can. J. Physiol. Pharmacol., vol. 77, pp. 79-88 (1999)).

The magnitude of the DKIE can be expressed as the ratio between the rates of a given reaction in which a C-H bond is broken, and the same reaction where deuterium is substituted for hydrogen. The DKIE can range from about 1 (no isotope effect) to very large numbers, such as 50 or more, meaning that the reaction can be fifty, or more, times slower when deuterium is substituted for hydrogen. Without being limited to a particular theory, high DKIE values may be due in part to a phenomenon known as tunneling, which is a consequence of the uncertainty principle. Tunneling is ascribed to the small mass of a hydrogen atom, and occurs because transition states involving a proton can sometimes form in the absence of the required activation energy. Because deuterium has more mass than hydrogen, it statistically has a much lower probability of undergoing this phenomenon.

Tritium ("T") is a radioactive isotope of hydrogen, used in research, fusion reactors, neutron generators and radiopharmaceuticals. Tritium is a hydrogen atom that has 2 neutrons in the nucleus and has an atomic weight close to 3. It occurs naturally in the environment in very low concentrations, most commonly found as T₂O. Tritium decays slowly (half-life = 12.3 years) and emits a low energy beta particle that cannot penetrate the outer layer of human skin. Internal exposure is the main hazard associated with this isotope, yet it must be ingested in large amounts to pose a significant health risk. As compared with deuterium, a lesser amount of tritium must be consumed before it reaches a hazardous level. Substitution of tritium ("T") for hydrogen results in yet a stronger bond than deuterium and gives numerically larger isotope effects. Similarly, substitution of isotopes for other elements, including, but not limited to, ¹³C or ¹⁴C for carbon, ³³S, ³⁴S, or ³⁶S for sulfur, ¹⁵N for nitrogen, and ¹⁷O or ¹⁸O for oxygen, may lead to a similar kinetic isotope effect.

For example, the DKIE was used to decrease the hepatotoxicity of halothane by presumably limiting the production of reactive species such as trifluoroacetyl chloride. However, this method may not be applicable to all drug classes. For example, deuterium incorporation can lead to metabolic switching. The concept of metabolic switching asserts that xenogens, when sequestered by Phase I enzymes, may bind transiently and re-bind in a variety of conformations prior to the chemical reaction (e.g., oxidation). This hypothesis is supported by the relatively vast size of binding pockets in many Phase I enzymes and the promiscuous nature of many metabolic reactions. Metabolic switching can potentially lead to different proportions of known metabolites as well as altogether new metabolites. This new metabolic profile may impart more or less toxicity.

The animal body expresses a variety of enzymes for the purpose of eliminating foreign substances, such as therapeutic agents, from its circulation system. Examples of such enzymes include the cytochrome P450 enzymes ("CYPs"), esterases, proteases, reductases, dehydrogenases, and monoamine oxidases, to react with and convert these foreign substances to more polar intermediates or metabolites for renal excretion. Some of the most common metabolic reactions of pharmaceutical compounds involve the oxidation of a carbon-hydrogen (C-H) bond to either a carbon-oxygen (C-O) or carbon-carbon (C-C) pi-bond. The resultant metabolites may be stable or unstable under physiological conditions, and can have substantially different pharmacokinetic, pharmacodynamic, and acute and long-term toxicity profiles relative to the parent compounds. For many drugs, such oxidations are rapid. These drugs therefore often require the administration of multiple or high daily doses.

Therefore, isotopic enrichment at certain positions of a compound provided herein will produce a detectable KIE that will affect the pharmacokinetic, pharmacologic, and/or toxicological profiles of a compound provided herein in comparison with a similar compound having a natural isotopic composition.

### Preparation of Compounds

The compounds provided herein can be, in some instances, prepared, isolated, or obtained by any method apparent to those of skill in the art. Those skilled in the art will recognize that compounds provided herein may be designed or prepared by reaction, e.g., at a hydroxy group of said anti-cancer drug, for example, via condensation or dehydration.

In other instances, compounds provided herein can be prepared according to the Exemplary Preparation Schemes provided below. In certain embodiments, compounds provided herein can be prepared according to Schemes 1-6. Reaction conditions, steps, and reactants not provided in the Exemplary Preparation Schemes would be apparent to, and known by, those skilled in the art and, thus, are within the scope of this disclosure.

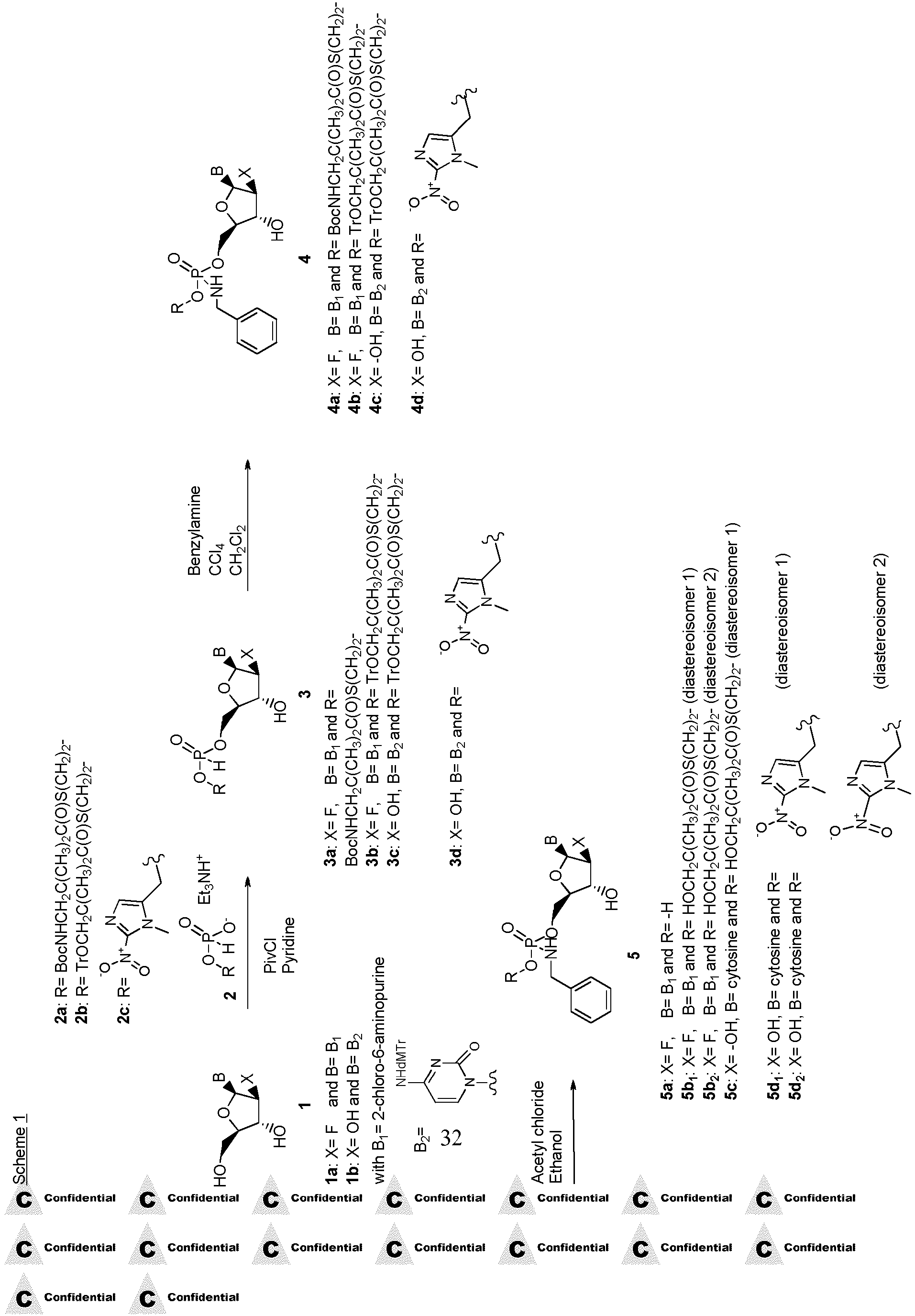

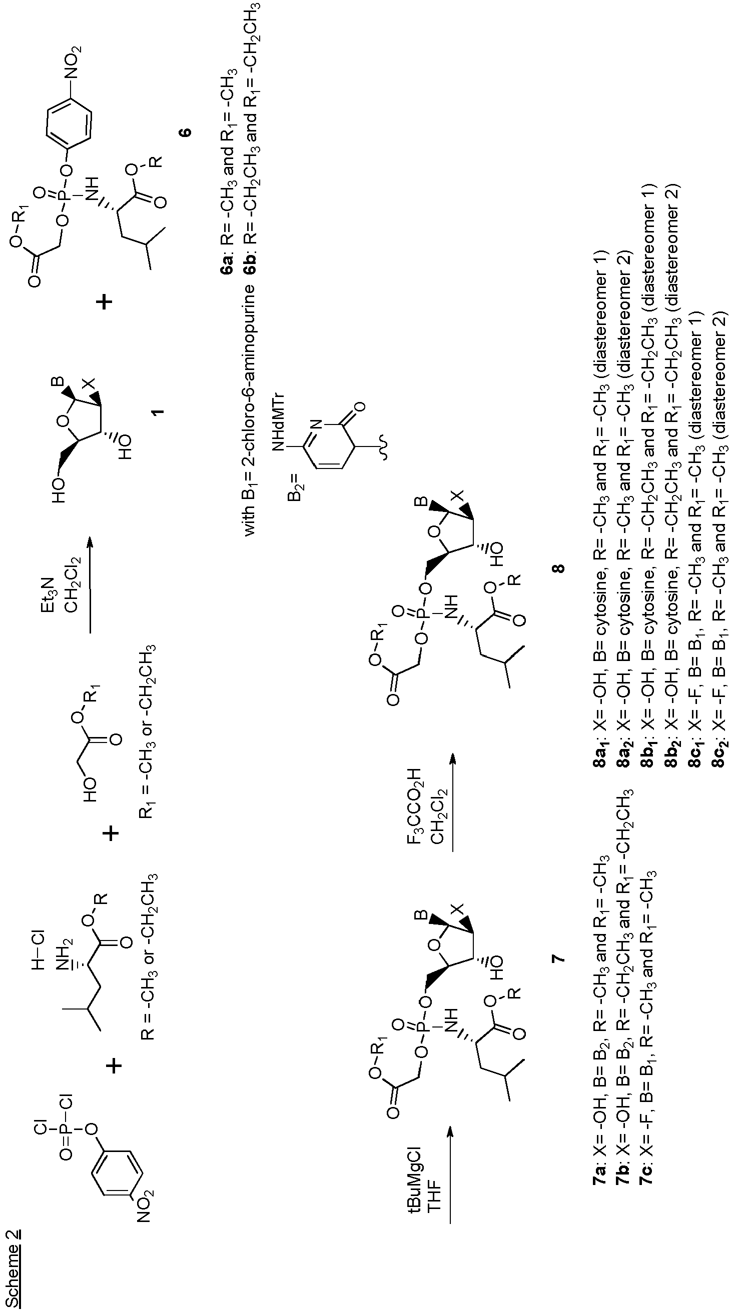

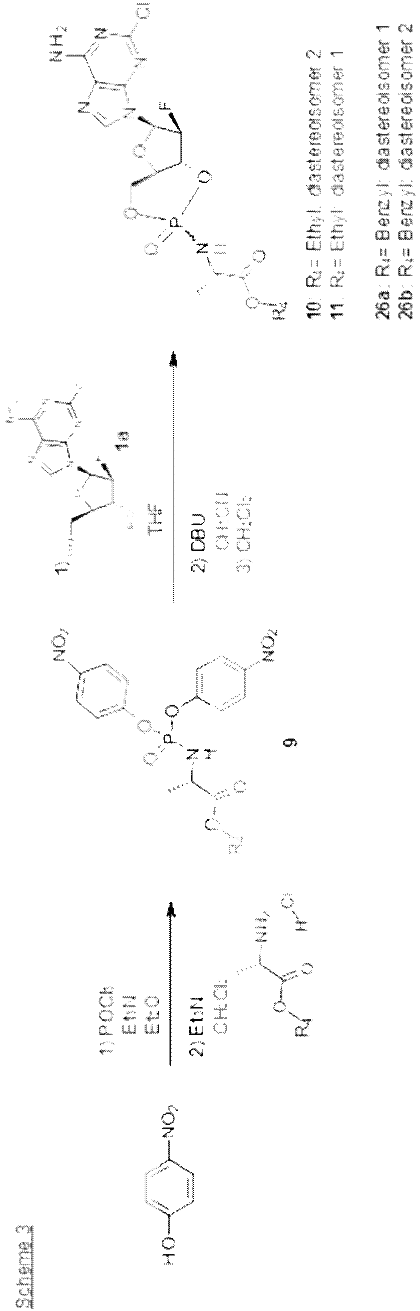

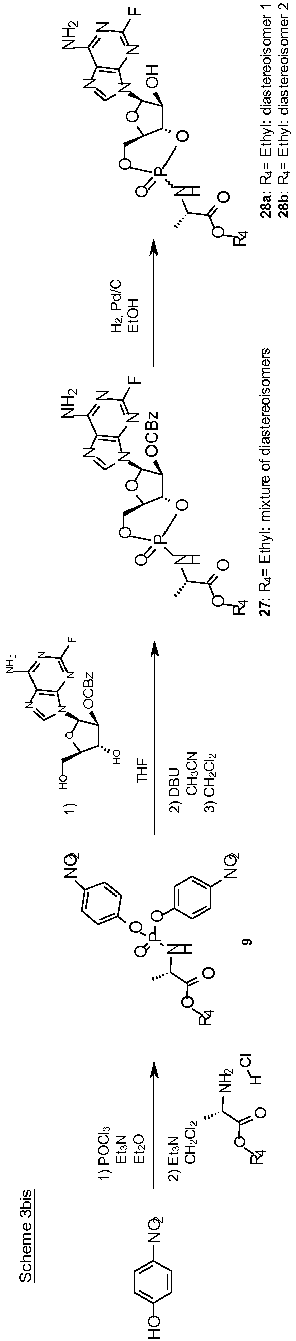

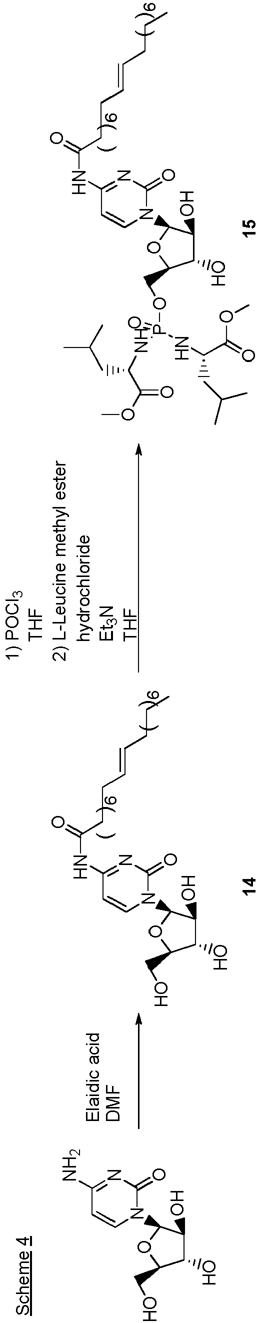

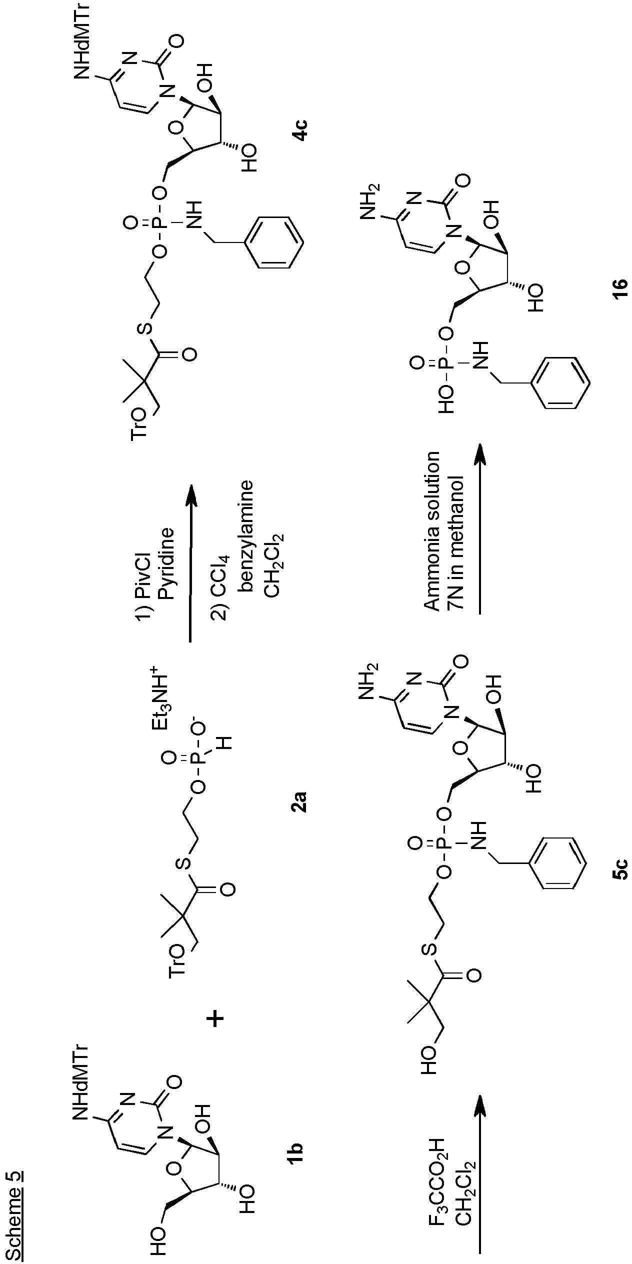

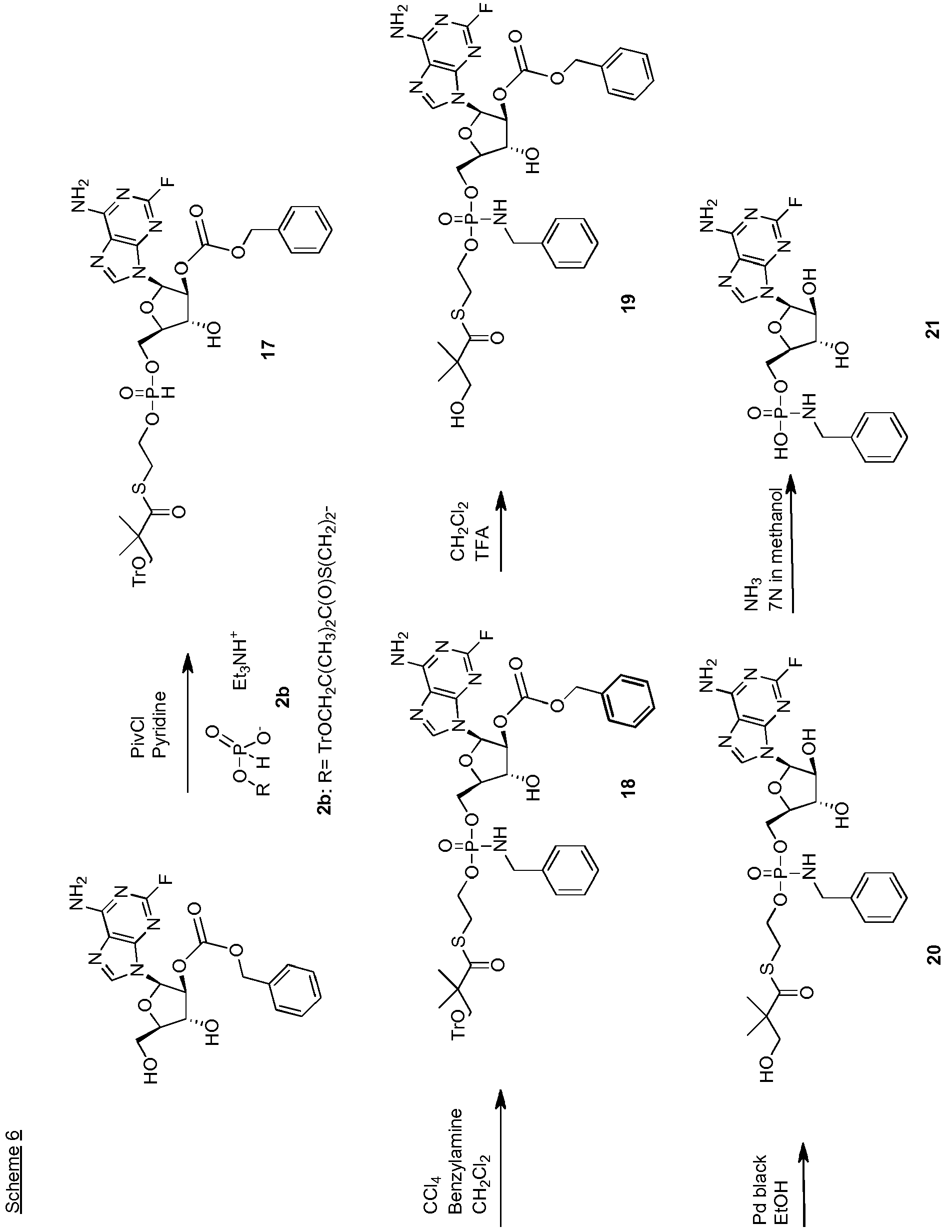

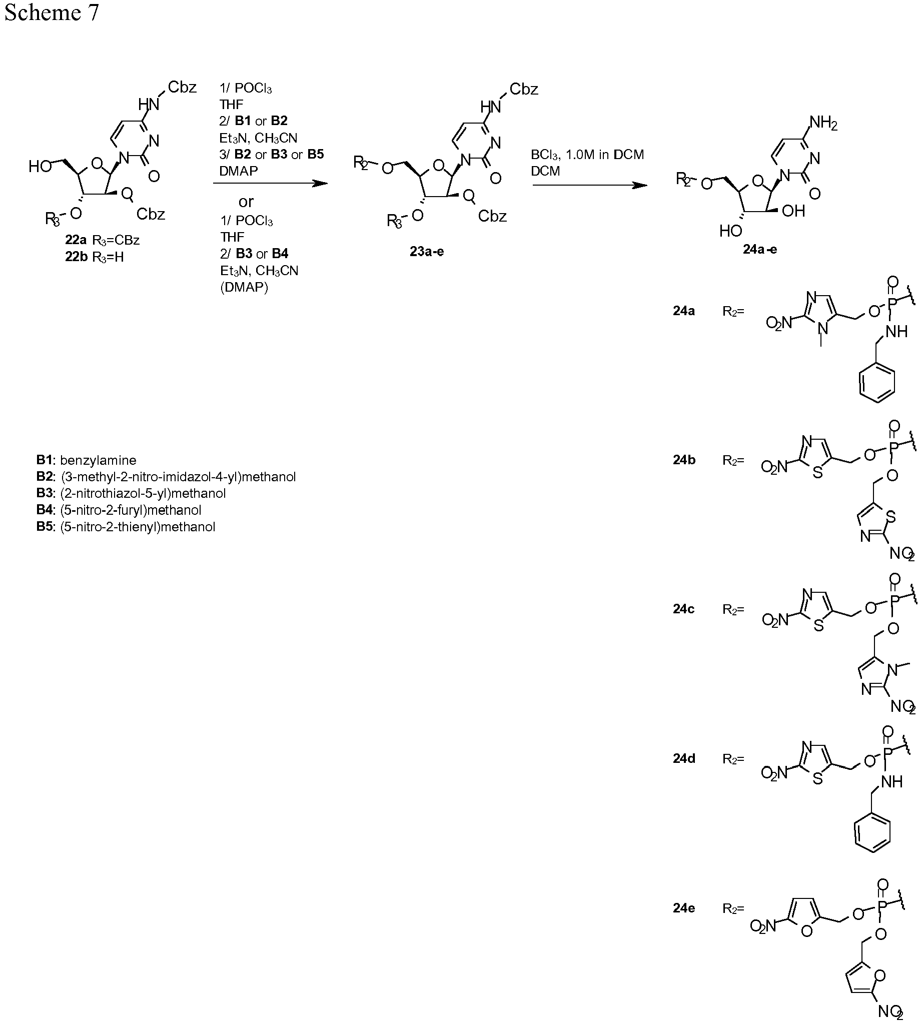

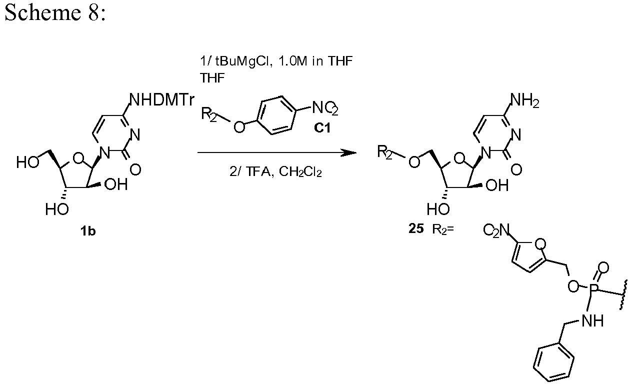

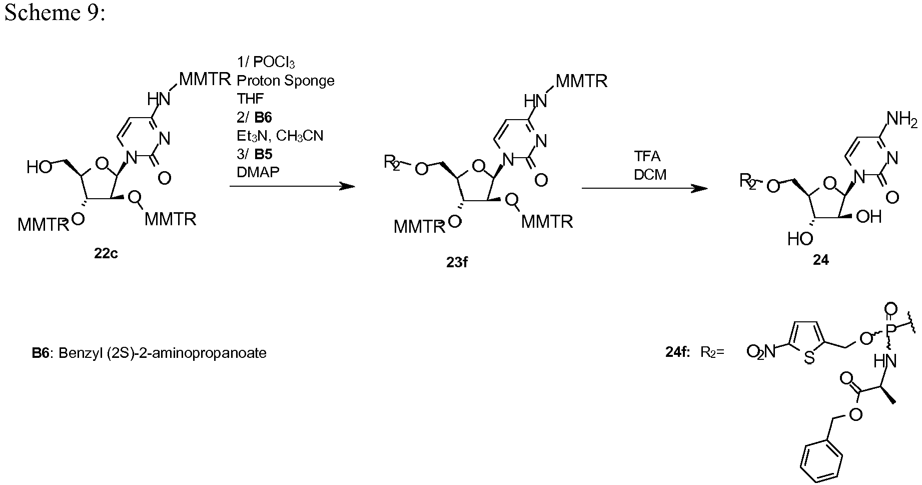

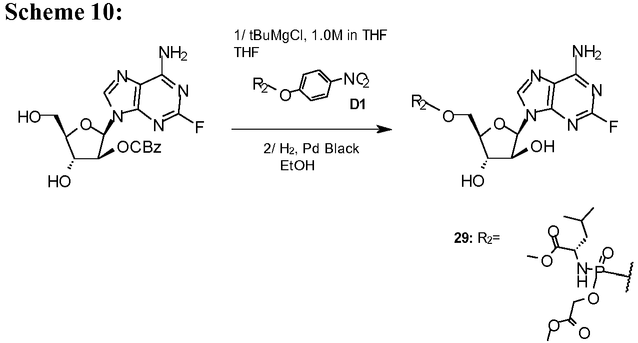

### General procedure A

Step 1: To a stirred solution of compound **22a** or **22b** (9.97mmol) in THF (10mL/mmol) at -10°C was added phosphorus oxychloride (11.96mmol). The reaction mixture was stirred from -10°C to room temperature overnight and then, a solution of reagent **B1** (9.97mmol) and triethylamine (49.85mmol) in acetonitrile (2.4mL/mmol) was added at 0°C. The reaction mixture was stirred for 1 hour at 0°C. Then, reagent **B2** (29.91mmol) and DMAP (29.91mmol) were added at 0°C and the reaction mixture was stirred from 0°C to room temperature for 2 hours. The reaction mixture was then diluted with ethyl acetate, washed with HCl 1N solution, water and brine. The organic layer was filtered and concentrated under reduced pressure. The crude residue was purified by flash chromatography on silica gel (DCM/methanol: 0 to 5%) to afford the expected compound.

Step 2: To a stirred suspension of compound **23a-c** (2.40mmol) in DCM (50mL/mmol) at -80°C under nitrogen was added a solution of boron trichloride, 1.0M in DCM (12.02mmol). The reaction mixture was stirred from -80°C to -40°C for 4 hours. Then MeOH (2mL) was added at -80°C and the reaction mixture was concentrated under reduced pressure at 25°C. The crude residue was purified by RP18 chromatography (H₂O/CH₃CN) to afford the expected compound as solid. In some cases, the mixture of diastereoisomers was purified by chiral HPLC to afford the expected diastereoisomer.

### General procedure B

Step 1: To a stirred solution of compound **22a** (1.86mmol) in THF (10mL/mmol) at -10°C under nitrogen was added phosphorus oxychloride (2.23mmol). The reaction mixture was stirred from -10°C to room temperature overnight and then, cooled down to 0°C. Then, a solution of reagent **B3** (3.72mmol) and triethylamine (9.31mmol) in acetonitrile (2.4mL/mmol) was added at 0°C. The reaction mixture was stirred at room temperature overnight. The reaction mixture was then diluted with ethyl acetate and washed with a saturated NH₄Cl solution. The organic layer was filtered and concentrated under reduced pressure. The crude residue was purified by flash chromatography on silica gel (DCM/methanol: 0 to 3%) to afford the expected compound.

Step 2: according to step 2 of general procedure A.

### General procedure C

Step 1: To a solution of compound **1b** (2.20mmol) in THF (10mL/mmol) at 0°C was added a solution of tert-butylmagnesium chloride, 1M in THF (4.60mmol). The reaction mixture was stirred for 1 hour at room temperature, then cooled to 0°C. To this reaction mixture was added a solution of the appropriate reagent **C1** (2.40mmol) in THF (10mL/mmol). The reaction mixture was stirred for 1 hour at 0°C and at room temperature overnight. The reaction mixture was diluted with ethyl acetate and washed with a saturated NH₄Cl solution, water and brine. The organic layer was dried, filtered and concentrated under reduced pressure. The crude residue was purified by flash chromatography on silica gel (DCM/methanol) to afford the expected intermediate as a mixture of diastereoisomers;

Step 2: To a solution of the previous intermediate (0.52mmol) in DCM (30mL/mmol) was added trifluoroacetic acid (10.27mmol). The reaction mixture was stirred at room temperature for 3 hours and purified by flash chromatography on silica gel (DCM/methanol: 0 to 20%) followed by RP18 chromatography ((H₂O/CH₃CN) to afford the expected compound as mixture of diastereoisomers. This mixture was purified by MS-preparative HPLC or by chiral HPLC to afford the 2 expected diastereoisomers as pure solid compounds.

### General procedure D

Step 1: To a stirred solution of 4-nitrophenol (29.36mmol) and phosphorus oxychloride (14.68mmol) in diethyl ether (10mL/mmol) at -80°C under nitrogen was added triethylamine (29.36mmol). The reaction mixture was stirred at room temperature overnight. Then, DCM (10mL/mmol) and the appropriate L-alanine ester hydrochloride (14.68mmol) were added at 0°C under nitrogen. To the reaction mixture was added dropwise at 0°C triethylamine (29.36mmol). The reaction mixture was stirred at room temperature overnight, and then, filtrated. The filtrate was concentrated under reduced pressure. The crude residue was purified by flash chromatography on silica gel (PE/Et₂O: 0 to 50%) to afford the expected intermediate 9.

Step 2: To a solution of compound 1a (1.55mmol) in THF (7mL/mmol) was added at room temperature under nitrogen a solution of 1,8-diazabicyclo[5.4.0]undec-7-ene (3.71mmol) in acetonitrile (12mL/mmol), followed by a solution of the appropriate compound 9 (1.86mmol) in DCM (12mL/mmol). The reaction mixture was stirred at room temperature overnight, and then, quenched on tampon phosphate pH=7. The reaction mixture was extracted with DCM and the organic layer was dried, filtered and concentrated under reduced pressure. The crude residue was purified by flash chromatography on silica gel (DCM/methanol: 0 to 10%) to afford the expected compound as a mixture of diastereoisomers. This mixture was purified by MS-Preparative HPLC to afford the pure diastereoisomer.

### Compounds 10 and 11

### (2 diastereomers)

### Ethyl (2S)-2-[[(4aR,6R,7S,7aR)-6-(6-amino-2-chloro-purin-9-yl)-7-fluoro-2-oxo-4a,6,7,7a-tetrahydro-4H-furo[3,2-d][1,3,2]dioxaphosphinin-2-yl]amino]propanoate

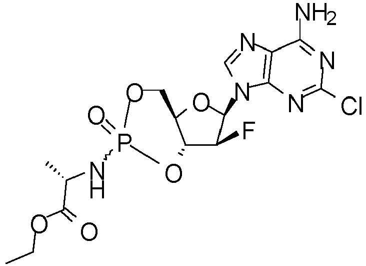

**Compounds 10** and **11** were synthesized from compound 1a according to scheme 3 and to the general procedure D.

### Reference Compound 21

### [(2R,3S,4S,5R)-5-(6-amino-2-fluoro-purin-9-yl)-3,4-dihydroxy-tetrahydrofuran-2-yl]methoxy-N-benzyl-phosphonamidic acid

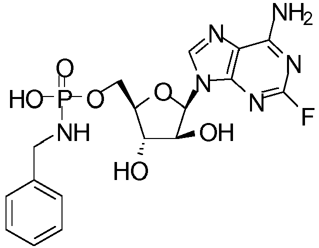

**Reference Compound 21** was synthesized according to scheme 6 and to the following general procedure:
To a solution of 2'OCBz fludarabine (3.38mmol) and 2-(2,2-dimethyl-3-trityloxypropanoyl)-sulfanylethoxyphosphinic acid (5.08mmol) in pyridine (12mL/mmol) at 0°C was slowly added pivaloyl chloride (6.77mmol). The reaction mixture was stirred for 1 hour at 0°C and for 2 hours at room temperature. The reaction was monitored by LC/MS. The reaction mixture was quenched with a 1M solution of NH₄Cl and extracted with ethyl acetate. The organic layer was dried, filtered and concentrated under reduced pressure to afford the expected intermediate 17.

To a solution of compound 17 (1.1mmol) in carbon tetrachloride (20mL/mmol) and DCM (15mL/mmol) were added benzylamine (5.5mmol) and triethylamine (6.6mmol) at room temperature. The reaction mixture was stirred overnight, and then, concentrated under reduced pressure and purified by flash chromatography on silica (DCM/methanol) to afford the expected intermediate 18.

To a solution of compound 18 (0.49mmol) in DCM (30mL/mmol) was added trifluoroacetic acid (7.4mmol) under nitrogen. The reaction mixture was stirred at room temperature overnight and purified by flash chromatography on silica (DCM/methanol: 0 to 20%) followed by MS-preparative HPLC to afford the mixture of diastereoisomers 19.

To a solution of compound 19 in EtOH (5mL/mmol) was added Palladium Black under nitrogen. After several flush N₂/vaccum and vaccum/H₂, the reaction mixture was stirred under H₂ Atmosphere for 6hours at room temperature. The reaction mixture was filtered on Celite and concentrated under reduced pressure to afford the expected intermediate 20.

The compound 20 (0.35mmol) was dissolved in a 7N ammonia solution in methanol (50mL/mmol) and the reaction mixture was stirred at room temperature for 4 hours. The reaction mixture was concentrated under reduced pressure and purified by RP-18 chromatography (H₂O+TEAB/CH₃CN) to afford the expected compound as solid.

### Reference Compound 24a

### (2 Diastereomers)

### 4-amino-1-[(2R,3S,4S,5R)-5-[[(benzylamino)-[(3-methyl-2-nitro-imidazol-4-yl)methoxy]phosphoryl]oxymethyl]-3,4-dihydroxy-tetrahydrofuran-2-yl]pyrimidin-2-one

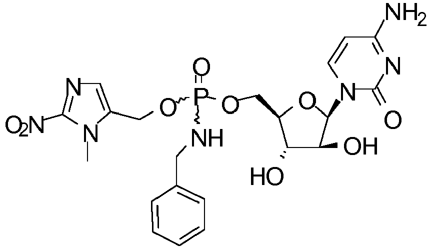

**Reference Compound 24a** was synthesized from compound 22a with reagent B1 and B2 according to scheme 7 and general procedure A.

**Reference Compound 24a:** (Diastereoisomer 1): ¹H NMR (DMSO-*d₆*, 400MHz) δ (ppm) 7.54 (d, *J*=7.51Hz, 1H), 7.32-7.27 (m, 4H), 7.26-7.21 (m, 1H), 7.20 (s, 1H), 7.11 (brs, 1H), 7.01 (brs, 1H), 6.09 (d, *J*=3.69Hz, 1H), 5.80 (dt, *J*=12.73Hz and 7.20Hz, 1H), 5.64 (d, *J*=7.51Hz, 1H), 5.59-5.53 (m, 2H), 5.05 (dd, *J*=13.26Hz and 7.79Hz, 1H), 5.00 (dd, *J*=13.26Hz and 7.34Hz, 1H), 4.14-3.88 (m, 7H), 3.86 (s, 3H); ³¹P NMR (DMSO-*d₆*, 162MHz) δ (ppm) 10.27 (s, 1P); MS (ESI) m/z = 550.1 (MH⁻).

**Reference Compound 24a:** (Diastereoisomer 2): ¹H NMR (DMSO-*d₆*, 400MHz) δ (ppm) 7.59 (d, *J*=7.43Hz, 1H), 7.323-7.29 (m, 4H), 7.26-7.20 (m, 1H), 7.19 (s, 1H), 7.11 (brs, 1H), 7.01 (brs, 1H), 6.10 (d, *J*=3.74Hz, 1H), 5.80 (dt, *J*=12.55Hz and 7.14Hz, 1H), 5.63 (d, *J*=7.43Hz, 1H), 5.58-5.56 (m, 2H), 5.05 (dd, *J*=13.24Hz and 7.83Hz, 1H), 4.99 (dd, *J*=13.24Hz and 7.31Hz, 1H), 4.16-4.09 (m, 1H), 4.06-3.91 (m, 5H), 3.90-3.88 (m, 1H), 3.85 (s, 3H); ³¹P NMR (DMSO-*d₆*, 162MHz) δ (ppm) 10.19 (s, 1P); MS (ESI) m/z = 552.1 (MH⁺).

### Reference Compound 24b

### [(2R,3S,4S,5R)-5-(4-amino-2-oxo-pyrimidin-1-yl)-3,4-dihydroxy-tetrahydrofuran-2-yl]methyl bis[(2-nitrothiazol-5-yl)methyl]phosphate

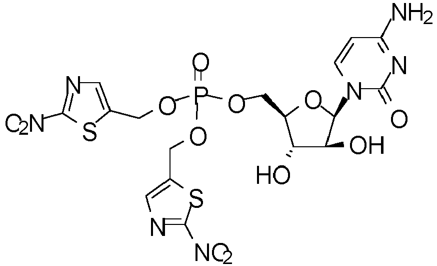

**Reference Compound 24b** was synthesized from compound 22a with reagent B3 according to scheme 7 and general procedure B.

**Reference Compound 24b:** ¹H NMR (DMSO-*d₆*, 400MHz) δ (ppm) 8.07 (s, 1H); 8.05 (s, 1H); 7.56 (d, *J*=7.46Hz, 1H), 7.11 (brs, 1H), 7.01 (brs, 1H), 6.11 (d, *J*=3.48Hz, 1H), 5.64-5.58 (m, 3H), 5.47-5.40 (m, 4H), 4.32-4.21 (m, 2H), 4.00-3.91 (m, 2H), 3.90-3.84 (m, 1H); ³¹P NMR (DMSO-*d₆*, 162MHz) δ (ppm) -1.37 (s, 1P); MS (ESI) m/z = 608.0 (MH⁺).

### Reference Compound 24c

### (Mixture of diastereomers 64/36)

### [(2R,3S,4S,5R)-5-(4-amino-2-oxo-pyrimidin-1-yl)-3,4-dihydroxy-tetrahydrofuran-2-yl]methyl (3-methyl-2-nitro-imidazol-4-yl)methyl(2-nitrothiazol-5-yl)methyl phosphate

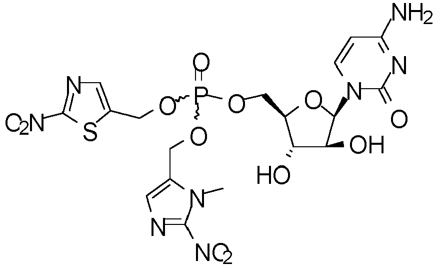

**Reference Compound 24c** was synthesized from compound 22a with reagents B2 and B3 according to scheme 7 and general procedure A.

**Reference Compound 24c:** ¹H NMR (DMSO-*d₆*, 400MHz) δ (ppm) 8.06 (s, 0.64H), 8.04 (s, 0.36H), 7.57 (d, *J*=7.49Hz, 0.64H), 7.56 (d, *J*=7.49Hz, 0.36H), 7.31-7.25 (m, 1H), 7.11 (brs, 1H), 7.02 (brs, 1H), 6.10 (d, *J*=3.48Hz, 1H), 5.65-5.57 (m, 3H), 5.44-5.37 (m, 2H), 5.28-5.21 (m, 2H), 4.28-4.18 (m, 2H), 3.98-3.83 (m, 6H); ³¹P NMR (DMSO-*d₆*, 162MHz) δ (ppm) -1.43 (s, 1P); MS (ESI) m/z = 605.2 (MH⁺).

### Reference Compound 24d

### (2 diastereomers)

### 4-amino-1-[(2R,3S,4S,5R)-5-[[(benzylamino)-[(5-nitro-2-thienyl)methoxy]phosphoryl]oxymethyl]-3,4-dihydroxy-tetrahydrofuran-2-yl]pyrimidin-2-one

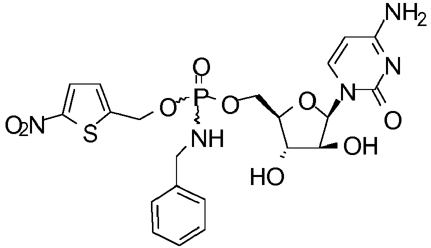

**Reference Compound 24d** was synthesized from compound 22b with reagents B1 and B5 according to scheme 7 and general procedure A.

**Reference Compound 24d:** (Diastereoisomer 1): ¹H NMR (DMSO-*d₆*, 400MHz) δ (ppm) 8.02 (d, *J*=4.21Hz, 1H), 7.54 (d, *J*=7.49Hz, 1H), 7.35-7.26 (m, 4H), 7.26-7.20 (m, 1H), 7.19 (d, *J*=4.21Hz, 1H), 7.10 (brs, 1H), 7.00 (brs, 1H), 6.09 (d, *J*=3.69Hz, 1H), 5.85 (dt, *J*=12.62Hz and 7.13Hz, 1H), 5.62 (d, *J*=7.50Hz, 1H), 5.58-5.53 (m, 2H), 5.17 (dd, *J*=13.81Hz and 8.51Hz, 1H), 5.11 (dd, *J*=13.81Hz and 8.51Hz, 1H), 4.15-3.87 (m, 7H); ³¹P NMR (DMSO-*d₆*, 162MHz) δ (ppm) 10.37 (s, 1P); MS (ESI) m/z = 554.2 (MH⁺).

**Reference Compound 24d:** (Diastereoisomer 2): ¹H NMR (DMSO-*d₆*, 400MHz) δ (ppm) 8.01 (d, *J*=4.20Hz, 1H), 7.59 (d, *J*=7.49Hz, 1H), 7.33-7.28 (m, 4H), 7.25-7.21 (m, 1H), 7.18 (d, *J*=4.18Hz, 1H), 7.09 (brs, 1H), 7.00 (brs, 1H), 6.10 (d, *J*=3.66Hz, 1H), 5.89-5.82 (m, 1H), 5.61 (d, *J*=7.51Hz, 1H), 5.57-5.55 (m, 2H), 5.17 (dd, *J*=13.90Hz and 8.62Hz, 1H), 5.11 (dd, J=13.90Hz and 8.43Hz, 1H), 4.17-4.11 (m, 1H), 4.07-3.92 m, 5H), 3.89-3.88 (m, 1H); ³¹P NMR (DMSO-*d₆*, 162MHz) δ (ppm) 10.28 (s, 1P); MS (ESI) m/z = 554.2 (MH⁺).

### Reference Compound 24e

### [(2R,3S,4S,5R)-5-(4-amino-2-oxo-pyrimidin-1-yl)-3,4-dihydroxy-tetrahydrofuran-2-yl]methyl bis[(5-nitro-2-furyl)methyl] phosphate

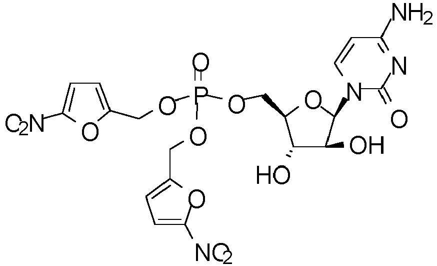

**Reference Compound 24e** was synthesized from compound 22a with reagent B4 according to scheme 7 and general procedure B.

**Reference Compound 24e:** ¹H NMR (DMSO-*d₆*, 400MHz) δ (ppm) 7.65 (d, *J*=3.77Hz, 1H), 7.64 (d, *J*=3.77Hz, 1H), 7.54 (d, *J*=7.48Hz, 1H), 7.11 (brs, 1H), 7.02 (brs, 1H), 6.97 (d, *J*=3.77Hz, 1H), 6.94 (d, *J*=3.77Hz, 1H), 6.09 (d, *J*=3.48Hz, 1H), 5.63 (d, *J*=7.48Hz, 1H), 5.60 (d, *J*=3.92Hz, 1H), 5.58 (d, *J*=4.82Hz, 1H), 5.21-5.14 (m, 4H), 4.27-4.21 (m, 2H), 3.98-3.90 (m, 2H), 3.89-3.84 (m, 1H); ³¹P NMR (DMSO-*d₆*, 162MHz) δ (ppm) -1.27 (s, 1P); MS (ESI) m/z = 574.2 (MH⁺).

### Reference Compound 24f

### (2 diastereomers)

### Benzyl (2S)-2-[[[(2R,3S,4S,5R)-5-(4-amino-2-oxo-pyrimidin-1-yl)-3,4-dihydroxy-tetrahydrofuran-2-yl]methoxy-[(5-nitro-2-thienyl)methoxy]phosphoryl]amino]propanoate

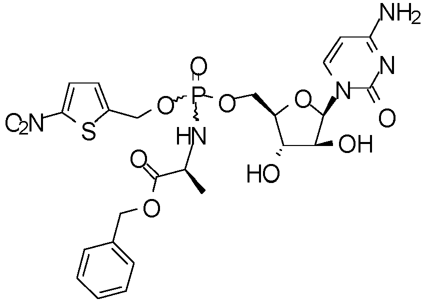

**Reference Compound 24f** was synthesized from compound 22c with reagents B6 and B5 according to scheme 9 and general procedure A step 1. In this case, the addition of phosphorus oxychloride was followed by the addition of Proton sponge. Step 2 was done according to step 2 of general procedure C.

**Reference Compound 24f:** (Diastereoisomer 1): ¹H NMR (DMSO-*d₆*, 400MHz) δ (ppm) 8.03 (d, *J*=4.21Hz, 1H), 7.53 (d, *J*=7.41Hz, 1H), 7.38-7.28 (m, 5H), 7.21 (d, *J*=4.21Hz, 1H), 7.11 (brs, 1H), 7.02 (brs, 1H), 6.08 (d, *J*=3.69Hz, 1H), 5.90 (dd, *J*=9.96Hz and 12.07Hz, 1H), 5.64 (d, *J*=7.41Hz, 1H), 5.58-5.54 (m, 2H), 5.20-5.15 (m, 2H), 5.15-5.07 (m, 2H), 4.13-4.03 (m, 2H), 3.99-3.80 (m, 4H), 1.31 (d, *J*=7.17Hz, 3H); ³¹P NMR (DMSO-*d₆*, 162MHz) δ (ppm) 8.57 (s, 1P); MS (ESI) m/z = 626.2 (MH⁺).

**Reference Compound 24f:** (Diastereoisomer 2): ¹H NMR (DMSO-*d₆*, 400MHz) δ (ppm) 8.02 (d, *J*=4.21Hz, 1H), 7.57 (d, *J*=7.41Hz, 1H), 7.38-7.28 (m, 5H), 7.19 (d, *J*=4.21Hz, 1H), 7.10 (brs, 1H), 7.01 (brs, 1H), 6.10 (d, *J*=3.69Hz, 1H), 5.95 (dd, *J*=10.15Hz and 12.64Hz, 1H), 5.61 (d, *J*=7.41Hz, 1H), 5.59-5.55 (m, 2H), 5.20-5.06 (m, 4H), 4.19-4.03 (m, 2H), 3.99-3.91 (m, 2H), 3.90-3.78 (m, 2H), 1.31 (d, *J*=7.17Hz, 3H); ³¹P NMR (DMSO-*d₆*, 162MHz) δ (ppm) 8.16 (s, 1P); MS (ESI) m/z = 626.2 (MH⁺).

### Reference Compound 25

### (2 diastereomers)

### 4-amino-1-[(2R,3S,4S,5R)-5-[[(benzylamino)-[(5-nitro-2-furyl)methoxy]phosphoryl]oxymethyl]-3,4-dihydroxy-tetrahydrofuran-2-yl]pyrimidin-2-one

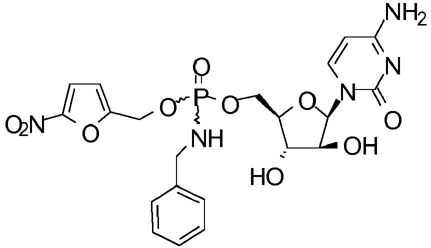

**Reference Compound 25** was synthesized with reagent C1 according to scheme 8 and general procedure C.

**Reference Compound 25:** (Diastereoisomer 1): ¹H NMR (DMSO-*d₆*, 400MHz) δ (ppm) 7.66 (d, *J*=3.74Hz, 1H), 7.54 (d, *J*=7.40Hz, 1H), 7.33-7.28 (m, 4H), 7.25-7.21 (m, 1H), 7.11 (brs, 1H), 7.03 (brs, 1H), 6.88 (d, *J*=3.74Hz, 1H), 6.09 (d, *J*=3.69Hz, 1H), 5.81(dt, *J*=12.52Hz and 7.15Hz, 1H), 5.63 (d, *J*=7.40Hz, 1H), 5.58-5.56 (m, 2H), 5.04-4.94 (m, 2H), 4.10-4.05 (m, 2H), 4.02-3.88 (m, 5H); ³¹P NMR (DMSO-*d₆*, 162MHz) δ (ppm) 10.49 (s, 1P); MS (ESI) m/z = 538 (MH⁺).

**Reference Compound 25:** (Diastereoisomer 2): ¹H NMR (DMSO-*d₆*, 400MHz) δ (ppm) 7.65 (d, *J*=3.78Hz, 1H), 7.58 (d, *J*=7.43Hz, 1H), 7.33-7.28 (m, 4H), 7.25-7.21 (m, 1H), 7.10 (brs, 1H), 7.02 (brs, 1H), 6.87 (d, *J*=3.78Hz, 1H), 6.09 (d, *J*=3.65Hz, 1H), 5.82(dt, *J*=12.45Hz and 7.17Hz, 1H), 5.63 (d, *J*=7.43Hz, 1H), 5.58-5.56 (m, 2H), 5.04-4.93 (m, 2H), 4.15-4.09 (m, 1H), 4.06-3.91 (m, 5H), 3.89-3.87 (m, 1H); ³¹P NMR (DMSO-*d₆*, 162MHz) δ (ppm) 10.39 (s, 1P); MS (ESI) m/z = 538 (MH⁺).

### Reference Compound 26:

### (2 diastereomers)

### Benzyl (2S)-2-[[(4aR,6R,7S,7aR)-6-(6-amino-2-chloro-purin-9-yl)-7-fluoro-2-oxo-4a,6,7,7a-tetrahydro-4H-furo[3,2-d][1,3,2]dioxaphosphinin-2-yl]amino]propanoate

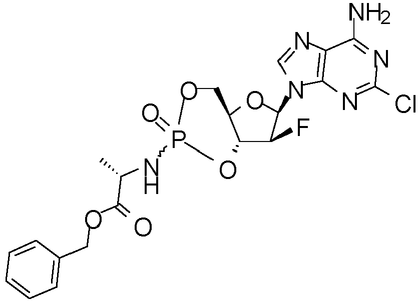

**Reference Compound 26** was synthesized from compound 1a according to scheme 3 and to the following procedure D.

**Reference Compound 26a** (Diastereoisomer 1): ¹H NMR (DMSO-*d₆*, 400MHz) δ (ppm) 8.35 (d, *J*=1.43Hz, 1H), 7.97 (brs, 2H), 7.41-7.29 (m, 5H), 6.60 (dd, *J*=5.14Hz and 6.61Hz, 1H), 6.14 (dd, *J*=10.15Hz and 13.40Hz, 1H), 5.80 (td, *J*=55.34Hz and 7.34Hz, 1H), 5.39-5.26 (m, 1H), 5.18 (d, *J*=12.62Hz, 1H), 5.14 (d, *J*=12.62Hz, 1H), 4.63-4.52 (m, 1H), 4.52-4.42 (m, 1H), 4.28-4.18 (m, 1H), 4.00-3.87 (m, 1H), 1.37 (d, *J*=7.15Hz, 3H); ³¹P NMR (DMSO-*d₆*, 162MHz) δ (ppm) 2.75 (s, 1P); ¹⁹F NMR (DMSO-*d₆*, 376MHz) δ (ppm) -199.64 (s, IF); MS (ESI) m/z = 527.0 (MH⁺).

**Reference Compound 26a** (Diastereoisomer 2): ¹H NMR (DMSO-*d₆*, 400MHz) δ (ppm) 8.55 (d, *J*=1.41Hz, 1H), 7.95 (brs, 2H), 7.40-7.34 (m, 5H), 6.60-6.57 (m, 1H), 6.30 (dd, *J*=13.69Hz and 9.94Hz, 1H), 5.75 (td, *J*=55.52Hz and 7.13Hz, 1H), 5.48-5.39 (m, 1H), 5.19-5.12 (m, 2H), 4.56-4.51 (m, 1H), 4.42-4.34 (m, 1H), 4.08-4.02 (m, 1H), 3.96-3.86 (m, 1H), 1.37 (d, *J*=7.10Hz, 3H); ³¹P NMR (DMSO-*d₆*, 162MHz) δ (ppm) 4.82 (s, 1P); ¹⁹F NMR (DMSO-*d₆*, 376MHz) δ (ppm) - 199.46 (s, IF); MS (ESI) m/z = 527.0 (MH⁺).

### Compound 28a and 28b

### (2 diastereomers)

### Ethyl (2S)-2-[[(4aR,6R,7S,7aS)-6-(6-amino-2-fluoro-purin-9-yl)-7-hydroxy-2-oxo-4a,6,7,7a-tetrahydro-4H-furo[3,2-d][1,3,2]dioxaphosphinin-2-yl]amino]propanoate

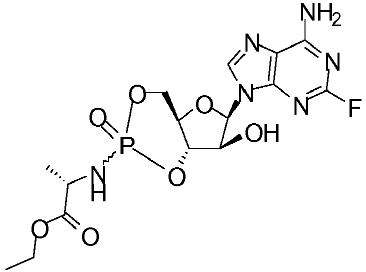

**Compound 28** was synthesized from 2'OCBz-fludarabine according to scheme 3bis and to the procedure D. In this case, additional step of deprotection using H₂, Pd/C 5% in ethanol was used to afford the 2 expected diastereoisomers.

**Compound 28a** (Diastereoisomer 1): ³¹P NMR (DMSO-*d₆*, 162MHz) δ (ppm) 3.01 (s, 1P); MS (ESI) m/z = 447 (MH⁺).

**Compound 28b** (Diastereoisomer 2): ³¹P NMR (DMSO-*d₆*, 162MHz) δ (ppm) 4.97 (s, 1P); MS (ESI) m/z = 447 (MH⁺).

### Reference Compound 29

### (Mixture of diastereomers)

### Methyl (2S)-2-[[[(2R,3S,4S,5R)-5-(6-amino-2-fluoro-purin-9-yl)-3,4-dihydroxy-tetrahydrofuran-2-yl]methoxy-(2-methoxy-2-oxo-ethoxy)phosphoryl]amino]-4-methyl-pentanoate

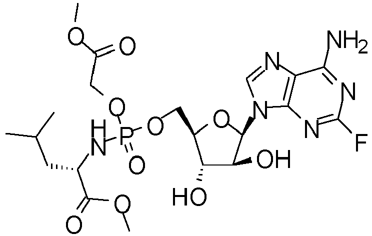

**Reference Compound 29** was synthesized according to scheme 10 and to the following procedure.

To a solution of 2'OCBz-fludarabine (1.19mmol) in THF (10mL/mmol) at 0°C was added a solution of tert-butylmagnesium chloride, 1M in THF (3.70mmol). The reaction mixture was stirred at room temperature for 1hour, and then cooled down to 0°C. The appropriate reagent **D1** (1.31mmol) in THF (10mL/mmol) was added, and the reaction mixture was stirred at 0°C for 1hour and for 3days at room temperature. The reaction mixture was diluted with ethyl acetate and washed with a saturated solution of NH₄Cl, water and brine. The combined organic layers were dried, filtered and concentrated under reduced pressure. The crude residue was purified by flash chromatography on silica (DCM/ethanol or DCM/methanol) to afford the expected intermediate;

To a solution of this intermediate (0.07mmol) in EtOH (5mL/mmol) was added Palladium Black (0.28mmol) under nitrogen. After several flush N₂/vaccum and vaccum/H₂, the reaction mixture was stirred under H₂ Atmosphere for 1h at room temperature. The reaction mixture was filtered on Celite and concentrated under reduced pressure. The crude residue was triturated in a DCM/pentane mixture, and then purified by flash chromatography on silica gel (DCM/methanol: 0 to 5%) to afford the expected compound as solid as mixture of diastereoisomers.

**Reference Compound 29:** ¹H NMR (DMSO-*d₆*, 400MHz) δ (ppm) 8.13 (s, 0.4H), 8.11 (s, 0.6H), 7.83 (brs, 2H), 6.18-6.16 (m, 1H), 5.78-5.65 (m, 2H), 4.52-4.44 (m, 2H), 4.26-4.09 (m, 4H), 3.99-3.93 (m, 1H), 3.76-3.69 (m, 1H), 3.68 (s, 1.8H), 3.66 (s, 1.2H), 3.62 (s, 1.2H), 3.60 (s, 1.8H), 1.73-1.65 (m, 1H), 1.52-1.22 (m, 2H), 0.88-0.82 (m, 6H); ³¹P NMR (DMSO-*d₆*, 162MHz) δ (ppm) 8.68 (s, 0.6P), 8.51 (s, 0.4P); ¹⁹F NMR (DMSO-*d₆*, 376MHz) δ (ppm) -52.38 (m, IF); MS (ESI) m/z = 565 (MH⁺).

Characterization data of the compounds provided herein are described in Table 1:

| Structure | LC/MS | NMR |
|---|---|---|
| 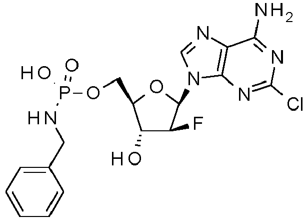 | MS (ESI) m/z = 473 (MH⁺). | ¹H NMR (400 MHz, DMSO-*d₆*) δ (ppm) 8.24 (s, 1H), 7.88 (brs, 2H), 7.32-7.30 (m, 2H), 7.26-7.22 (m, 2H), 7.16-7.13 (m, 1H), 6.30 (dd, *J*=5.0 Hz and 12.14 Hz, 1H), 5.26 (td, *J*=4.67 Hz and 53.0 Hz, 1H), 4.48 (td, *J*=4.94 Hz and 19.75 Hz, 1H), 3.95-3.87 (m, 5H), 2.87 (brs, 4H), 1.10 (t, *J*=6.95 Hz, 6H). |
| | | ³¹P NMR (162 MHz, DMSO-*d₆*) δ (ppm) 6.59 (1P, s). |
| | | ¹⁹F NMR (376 MHz, DMSO-*d₆*) δ (ppm) - 198.12 (s, 1F). |
| 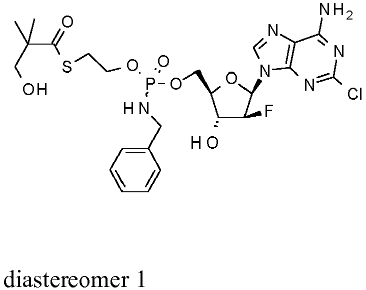 | MS (ESI) m/z = 633.2 (MH⁺). | ¹H NMR (400 MHz, DMSO-*d₆*) δ (ppm) 8.19 (d, *J*=2.10 Hz, 1H), 7.89 (brs, 2H), 7.32-7.25 (m, 4H), 7.23-7.17 (m, 1H), 6.35 (dd, *J*=4.37 Hz and 15.3 Hz, 1H), 6.13 (d, *J*=5.1 Hz, 1H), 5.68-5.62 (m, 1H), 5.24 (td, *J*=4.0 Hz and 52.34 Hz, 1H), 4.93-4.90 (m, 1H), 4.51-4.43 (m, 1H), 4.19-4.01 (m, 3H), 3.97-3.82 (m, 4H), 3.42 (d, *J*=5.4 Hz, 2H), 3.03-3.0 (m, 2H), 1.09 (s, 6H). |
| | | ³¹P NMR (162 MHz, DMSO-*d₆*) δ (ppm) 9.93 (1P, s). |
| 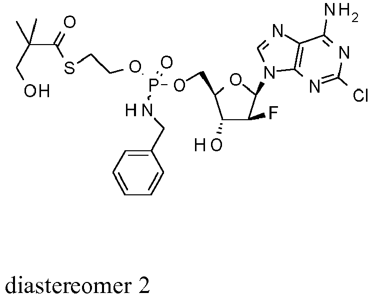 | MS (ESI) m/z = 633.2 (MH⁺). | ¹H NMR (400 MHz, DMSO-*d₆*) δ (ppm) 8.21 (d, *J*=2.19 Hz, 1H), 7.89 (brs, 2H), 7.31-7.28 (m, 4H), 7.24-7.19 (m, 1H), 6.36 (dd, *J*=4.4 Hz and 15.41 Hz, 1H), 6.12 (d, *J*=5.1 Hz, 1H), 5.70-5.64 (m, 1H), 5.23 (td, *J*=3.93 Hz and 52.23 Hz, 1H), 4.92-4.89 (m, 1H), 4.50-4.42 (m, 1H), 4.14-4.11 (m, 2H), 4.05-4.01 (m, 1H), 3.98-3.80 (m, 4H), 3.41 (d, *J*=5.42 Hz, 2H), 3.02-2.99 (m, 2H), 1.08 (s, 6H). |
| | | ³¹P NMR (162 MHz, DMSO-*d₆*) δ (ppm) 9.84 (s, 1P). |
| 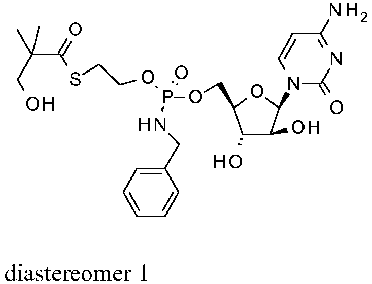 | MS (ESI) m/z = 573.2 (MH⁺). | ¹H NMR (400 MHz, DMSO-*d₆*) δ (ppm) 7.56 (d, *J*=7.41 Hz, 1H), 7.34-7.28 (m, 4H), 7.25-7.20 (m, 1H), 7.08 (brs, 1H), 6.99 (brs, 1H), 6.08 (d, *J*=3.76 Hz, 1H), 5.66-5.59 (m, 2H), 5.54-5.52 (m, 2H), 4.92 (t, *J*=5.53 Hz, 1H), 4.09-3.80 (m, 9H), 3.45-3.40 (m, 2H), 3.02 (t, *J*=6.61 Hz, 2H), 1.10 (s, 6H). |
| | | ³¹P NMR (162 MHz, DMSO-*d₆*) δ (ppm) 9.89 (s, 1P). |
| 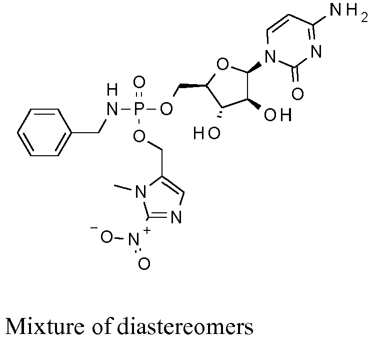 | MS (ESI) m/z = 552.2 (MH⁺). | ¹H NMR (400 MHz, MeOD) δ (ppm) 7.78 (d, *J*=7.58 Hz, 0.55H), 7.73 (d, *J*=7.58 Hz, 0.45H), 7.34-7.28 (m, 4H), 7.26-7.21 (m, 1H), 7.15 (s, 0.45H), 7.14 (s, 0.55H), 6.23 (d, *J*=3.67 Hz, 0.55H), 6.21 (d, *J*=3.67 Hz, 0.45H), 5.79 (d, *J*=7.56 Hz, 0.45H), 5.78 (d, *J*=7.49 Hz, 0.55H), 5.15-5.03 (m, 2H), 4.35-4.00 (m, 7H), 3.93 (s, 1.35H), 3.92 (s, 1.65H). |
| | | ³¹P NMR (162 MHz, MeOD) δ (ppm) 10.24 (s, 0.45P), 10.06 (s, 0.55P). |
| 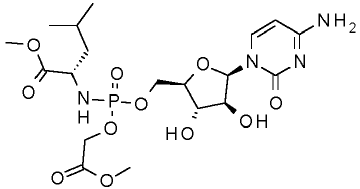 | MS (ESI) m/z = 523 (MH⁺). | ¹H NMR (400 MHz, MeOD) δ (ppm) 8.53 (brs, 0.65H), 7.83-7.79 (m, 1H), 6.24 (d, *J*=3.74 Hz, 0.57H), 6.22 (d, *J*=3.74 Hz, 0.43H), 5.89 (d, *J*=7.49 Hz, 0.57H), 5.88 (d, *J*=7.40 Hz, 0.43H), 4.62-4.55 (m, 2H), 4.41 - 4.21 (m, 2H), 4.20-4.18 (m, 1H), 4.11-4.06 (m, 1H), 4.04-4.03 (m, 1H), 3.95-3.86 (m, 1H), 3.77 (s, 1.29H), 3.76 (s, 1.71H), 3.71 (s, 3H), 1.84-1.74 (m, 1H), 1.57-1.53 (m, 2H), 0.95-0.92 (m, 6H). |
| | | ³¹P NMR (162 MHz, DMSO-*d₆*) δ (ppm) 8.98 (s, 0.43P), 8.59 (s, 0.57P). |
| 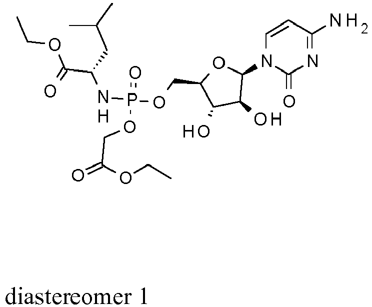 | MS (ESI) m/z = 551.0 (MH⁺). | ¹H NMR (400 MHz, DMSO-*d₆*) δ (ppm) 7.53 (d, J= 7.43Hz, 1H), 7.13 (brs, 1H), 7.02 (brs, 1H), 6.08 (d, J= 3.67Hz, 1H), 5.68-5.64 (m, 2H), 5.55-5.54 (m, 2H), 4.50 (d, J= 9.71Hz, 2H), 4.15 (q, J= 7.14Hz, 2H), 4.12-4.04 (m, 4H), 3.97-3.95 (m, 1H), 3.93-3.89 (m, 1H), 3.87-3.85 (m, 1H), 3.76-3.68 (m, 1H), 1.75-1.68 (m, 1H), 1.53-1.39 (m, 2H), 1.20 (dt, J= 10.96Hz and 7.14Hz, 6H), 0.87 (t, J= 6.89Hz, 6H); |
| | | ³¹P NMR (162 MHz, DMSO-*d₆*) δ (ppm) 8.82 (s, 1P). |
| 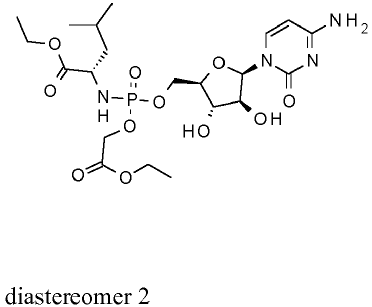 | MS (ESI) m/z = 551.0 (MH⁺). | ¹H NMR (400 MHz, DMSO-*d₆*) δ (ppm) 7.57 (d, *J*= 7.44Hz, 1H), 7.13 (brs, 1H), 7.02 (brs, 1H), 6.10 (d, *J*= 3.70Hz, 1H), 5.67 (dd, *J*= 13.07Hz and 10.23Hz, 1H), 5.65 (d, *J*= 7.44Hz, 1H), 5.57 (d, *J*= 6.01Hz, 1H), 5.56 (d, *J*= 6.85Hz, 1H), 4.50-4.39 (m, 2H), 4.19-4.04 (m, 6H), 3.97-3.91 (m, 2H), 3.87-3.85 (m, 1H), 3.72-3.64 (m, 1H), 1.76-1.66 (m, 1H), 1.53-1.38 (m, 2H), 1.22-1.16 (m, 6H), 0.87 (t, *J*= 5.98Hz, 6H). |
| | | ³¹P NMR (162 MHz, DMS0-*d₆*) δ (ppm) 8.52 (s, 1P). |
| 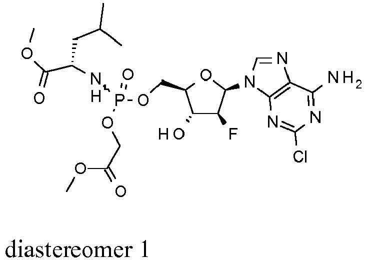 | MS (ESI) m/z = 583.04 (MH⁺). | ¹H NMR (400 MHz, CDCl₃) δ (ppm) 8.075 (d, *J*=2.4 Hz, 1H), 6.47 (dd, *J*=4 Hz and 17.2 Hz, 1H), 5.77 (brs, 2H), 5.18 (td, *J*=3.4 Hz and 51.6 Hz, 1H), 4.75-4.25 (m, 5H), 4.18-4.12 (m, 1H), 3.98-3.90 (m, 1H), 3.81 (s, 3H), 3.74 (s, 3H), 3.37 (t, *J*=10.6 Hz, 1H), 1.79-1.70 (m, 1H), 1.65-1.48 (m, 2H), 1.32-1.26 (m, 1H), 0.95-0.93 (m, 6H). |
| | | ³¹P NMR (162 MHz, DMSO-*d₆*) δ (ppm) 8.54 (s, 1P). |
| | | ¹⁹F NMR (376 MHz, DMSO-*d₆*) δ (ppm) -197.48 (s, 1F). |
| 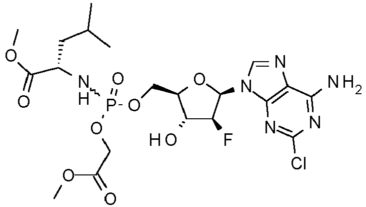 | MS (ESI) m/z = 583.04 (MH⁺). | ¹H NMR (400 MHz, CDCl₃) δ (ppm) 8.044 (d, *J*=3.2 Hz, 1H), 6.46 (dd, *J*=3.6 Hz and 18.4 Hz, 1H), 5.84 (brs, 2H), 5.14 (td, *J*=2.8 Hz and 51.6 Hz, 1H), 4.67-4.52 (m, 3H), 4.42-4.27 (m, 2H), 4.20-4.15 (m, 1H), 4-3.92 (m, 1H), 3.81 (s, 3H), 3.74 (s, 3H), 3.52 (t, *J*=10.4 Hz, 1H), 1.80-1.72 (m, 1H), 1.63-1.46 (m, 2H), 1.32-1.26 (m, 1H), 0.95-0.93 (m, 6H). |
| | | ³¹P NMR (162 MHz, DMSO-*d₆*) δ (ppm) 8.68 (s, 1P). |
| | | ¹⁹F NMR (376 MHz, DMSO-*d₆*) δ (ppm) -197.62 (s, 1F). |
| 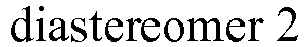 | | |
| 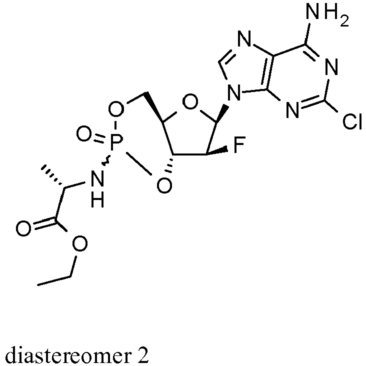 | MS (ESI) m/z = 487.1 (MNa⁺). | ¹H NMR (400 MHz, DMSO-*d₆*) δ (ppm) 8.34 (d, *J*=1.38 Hz, 1H), 7.94 (brs, 2H), 6.58 (dd, *J*=5.07 Hz and 6.61 Hz, 1H), 6.02 (dd, *J*=10.13 Hz and 13.30 Hz, 1H), 5.78 (td, *J*=7.0 Hz and 55.3 Hz, 1H), 5.35-5.26 (m, 1H), 4.62-4.55 (m, 1H), 4.25-4.18 (m, 1H), 4.15-4.07 (m, 2H), 3.88-3.78 (m, 1H), 1.33 (d, *J*=7.14 Hz, 3H), 1.18 (t, *J*=7.1 Hz, 3H). |
| | | ³¹P NMR (162 MHz, DMSO-*d₆*) δ (ppm) 2.68 (s, 1P). |
| | | ¹⁹F NMR (376 MHz, DMSO-*d₆*) d (ppm) - 199.81 (s, 1F). |
| 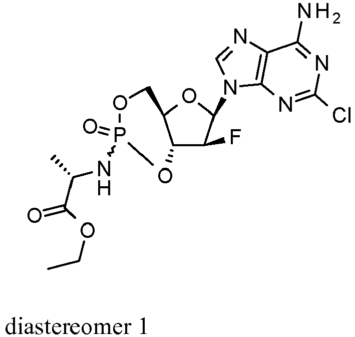 | MS (ESI) m/z = 487.1 (MNa⁺). | ¹H NMR (400 MHz, DMSO-*d₆*) δ (ppm) 8.56 (d, *J*=1.57 Hz, 1H), 7.95 (brs, 2H), 6.60 (dd, *J*=5.56 Hz and 6.59 Hz, 1H), 6.22 (dd, *J*=9.92 Hz and 13.60 Hz, 1H), 5.75 (td, *J*=7.16 Hz and, 55.49 Hz, 1H), 5.49-5.39 (m, 1H), 4.59-4.44 (m, 2H), 4.13-4.03 (m, 1H), 4.12 (d, *J*=7.02 Hz, 2H), 3.87-3.77 (m, 1H), 1.30 (d, *J*=7.14 Hz, 3H), 1.21 (t, *J*=7.06 Hz, 3H). |
| | | ³¹P NMR (162 MHz, DMSO-*d₆*) δ (ppm) 4.89 (s, 1P). |
| | | ¹⁹F NMR (376 MHz, DMSO-*d₆*) δ (ppm) - 199.49 (s, 1F). |
| 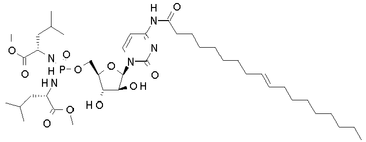 | MS (ESI) m/z = 842.4 (MH⁺). | ¹H NMR (400 MHz, DMSO-*d₆*) δ (ppm) 10.79 (brs, 1H), 8.11-8.08 (m, 1H), 7.23-7.20 (m, 1H), 6.11-6.08 (m, 1H), 5.64-5.59 (m, 2H), 5.38-5.32 (m, 2H), 4.77-4.69 (m, 2H), 4.11-4.03 (m, 2H), 4-3.86 (m, 3H), 3.75-3.68 (m, 2H), 3.61-3.56 (m, 6H), 2.40-2.34 (m, 2H), 1.96-1.89 (m, 4H), 1.75-1.64 (m, 2H), 1.57-1.35 (m, 6H), 1.32-1.19 (m, 20H), 0.89-0.81 (m, 15H). |
| | | ³¹P NMR (162 MHz, DMSO-*d₆*) δ (ppm) 13.05 (s, 1P). |
| 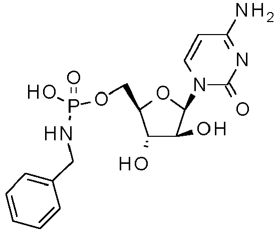 | MS (ESI) m/z = 413.2 (MH⁺). | ¹H NMR (400 MHz, DMSO-*d₆*) δ (ppm) 7.61 (d, *J*=7.41 Hz, 1H), 7.34-7.31 (m, 2H), 7.27-7.23 (m, 2H), 7.18-7.13 (m, 2H), 6.93 (brs, 1H), 6.01 (d, *J*=4.27 Hz, 1H), 5.96 (d, *J*=2.82 Hz, 1H), 5.87 (d, *J*=5.72 Hz, 1H), 5.63 (d, *J*=7.38 Hz, 1H), 4.09-4.06 (m, 1H), 3.91-3.86 (m, 3H), 3.82-3.73 (m, 3H), 3.53-3.36 (m, 1H), 3.13-3.09 (m, 1H). |
| | | ³¹P NMR (162 MHz, DMSO-*d₆*) δ (ppm) 6.44 (s, 1P). |
| 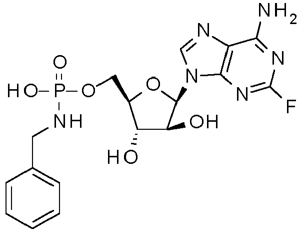 | MS (ESI) m/z = 455 (MH⁺). | ¹H NMR (400 MHz, DMSO-*d₆*) δ (ppm) 8.23 (s, 1H), 7.78 (brs, 2H), 7.31-7.28 (m, 2H), 7.23-7.17 (m, 2H), 7.14-7.09 (m, 1H), 6.41 (brs, 1H), 6.14 (brs, 1H), 6.08 (d, *J*=5.20 Hz, 1H), 4.30-4.25 (m, 1H), 4.14-4.11 (m, 1H), 4.01-3.93 (m, 1H), 3.91-3.83 (m, 4H). |
| | | ³¹P NMR (162 MHz, DMSO) δ (ppm) 5.38 (s, 1P). |
| | | ¹⁹F NMR (376 MHz, DMSO-*d₆*) d (ppm) - 52.47 (s, 1F). |
| 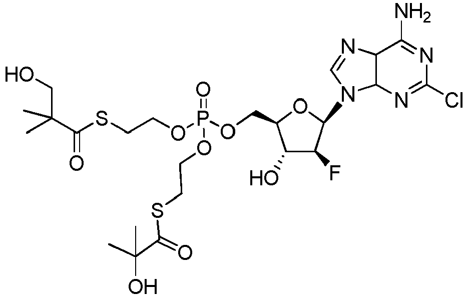 | MS (ESI) m/z = 704.1 (MH⁺). | ¹H NMR (400 MHz, DMSO-*d₆*) δ (ppm) 8.21 (d, *J*= 2.22Hz, 1H), 7.88 (brs, 2H), 6.37 (dd, *J*= 15.70Hz and 4.45Hz, 1H), 6.14 (d, *J*= 5.14Hz, 1H), 5.32-5.17 (m, 1H), 4.91-4.88 (m, 2H), 4.50-4.42 (m, 1H), 4.29-4.25 (m, 2H), 4.08-3.97 (m, 5H), 3.41 (t, *J*= 5.04Hz, 4H), 3.07 (q, *J*= 6.48Hz, 4H), 1.09 (d, *J*= 6.24Hz, 12H). |
| | | ³¹P NMR (162 MHz, DMSO-*d₆*) δ (ppm) - 1.76 (s, 1P). |

Using similar procedures, the following additional compounds were also prepared.

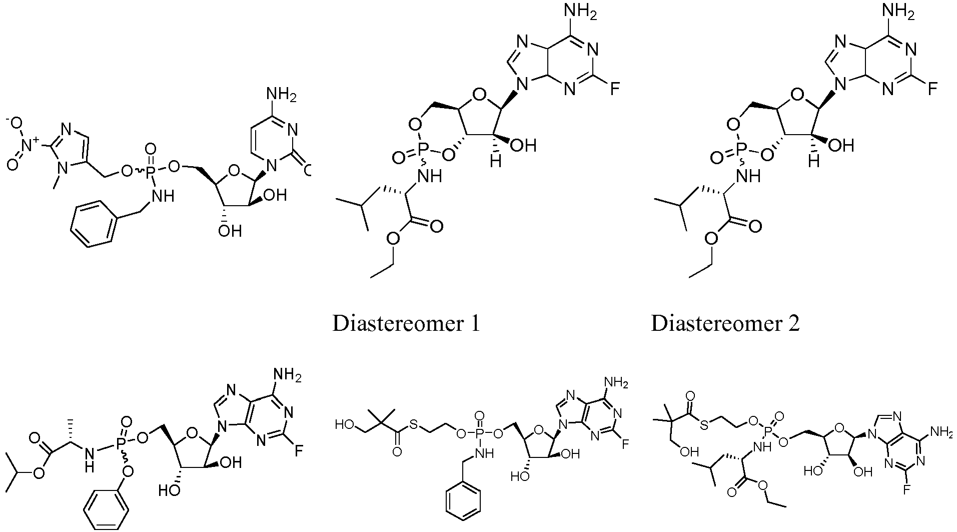

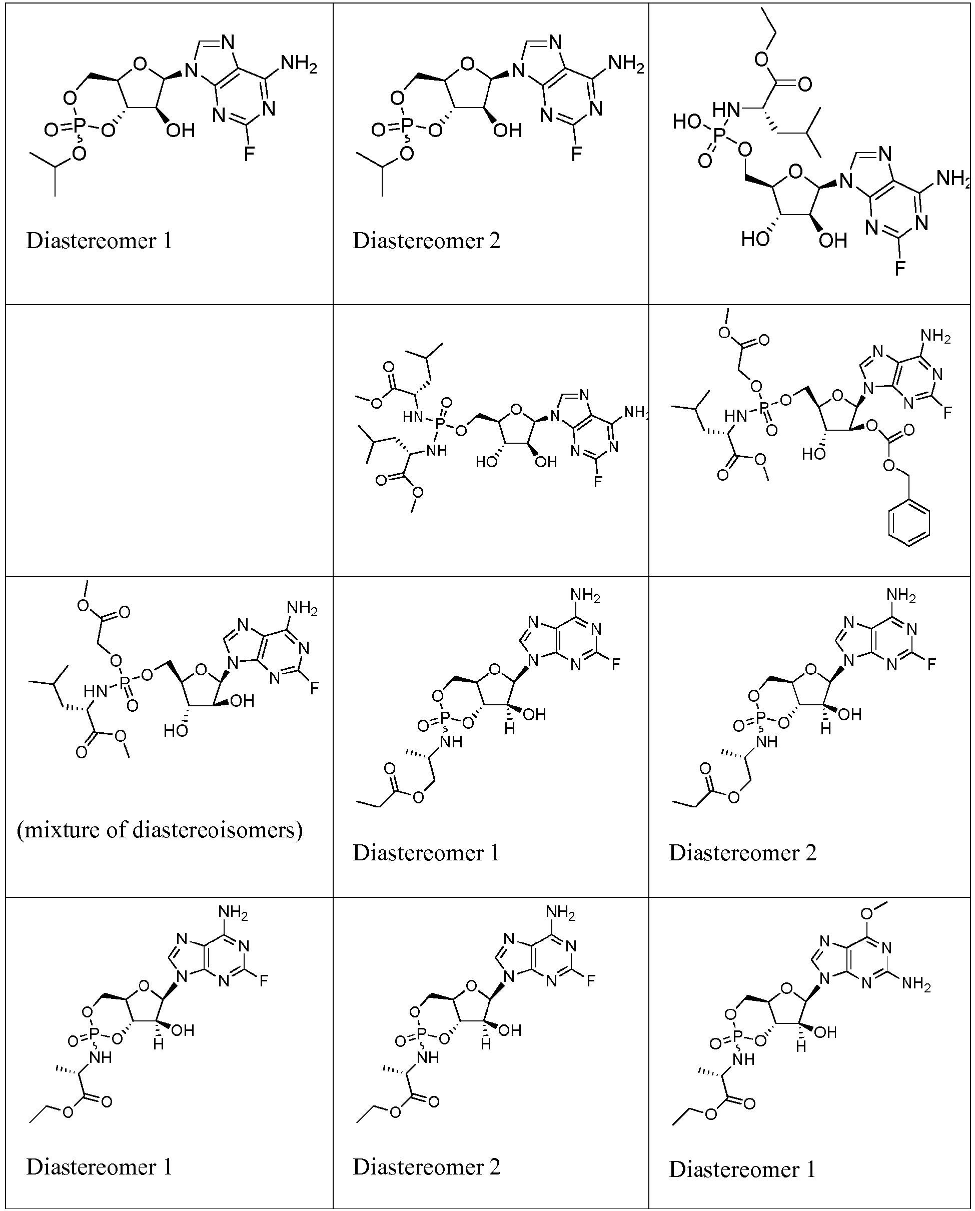

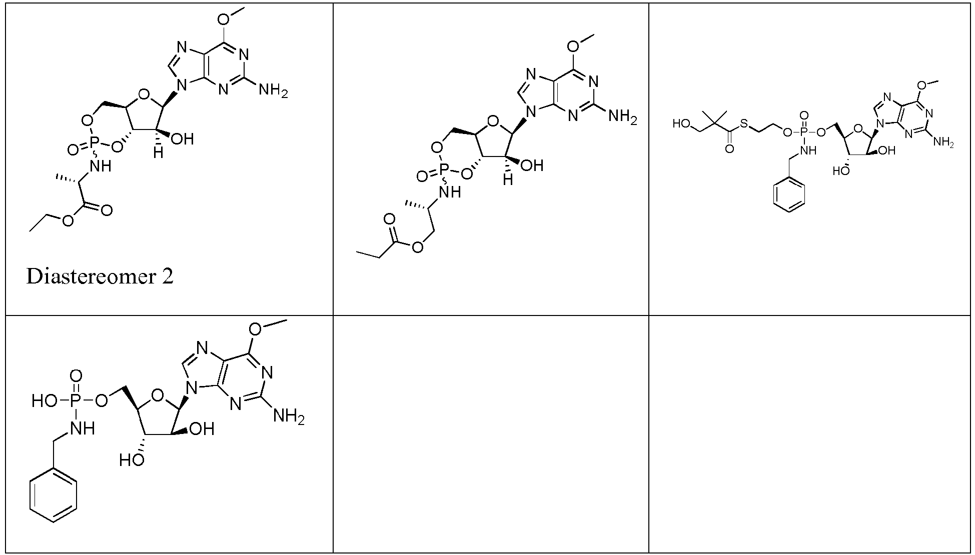

Using similar procedures, the following additional compounds were also prepared:

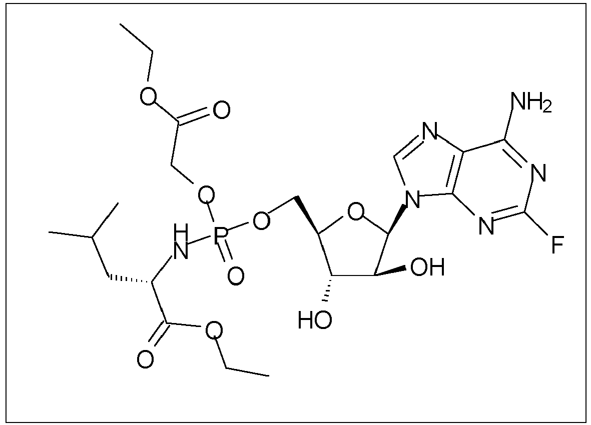

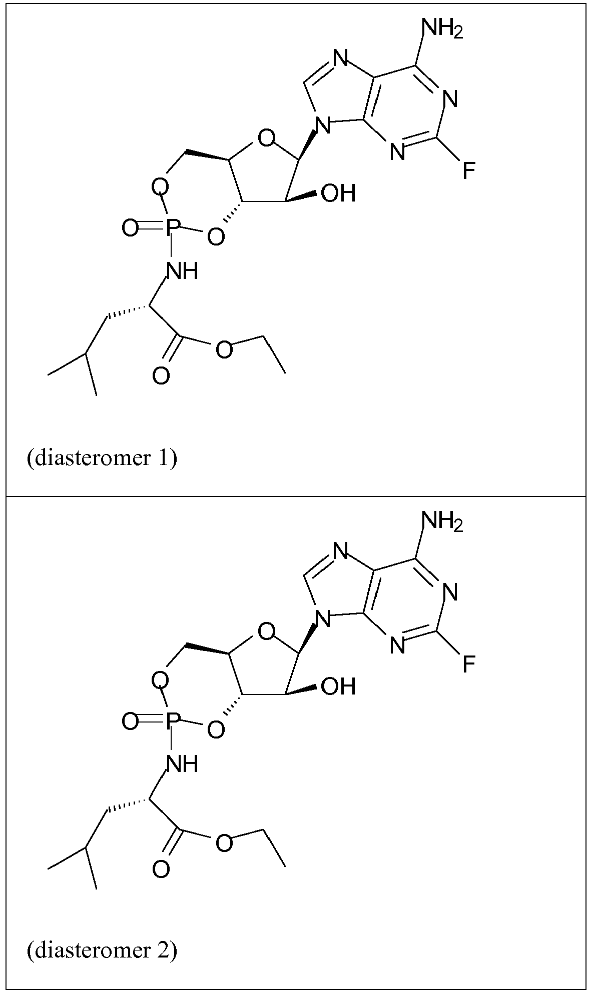

### Pharmaceutical Compositions and Methods of Administration

Derivatives of a variety of therapeutic agents can be formulated into pharmaceutical compositions using methods available in the art and those disclosed herein. Therapeutic agents that can be derivatized to phosphate derivatives include any anti-cancer agent that includes, or has been derivatized to include a reactive group for attachment of the phosphate moiety, including but not limited to nucleosides and nucleoside analogues including acyclic nucleosides. Any of the compounds disclosed herein can be provided in the appropriate pharmaceutical composition and be administered by a suitable route of administration.

The methods provided herein encompass administering pharmaceutical compositions containing at least one compound as described herein, if appropriate in the salt form, either used alone or in the form of a combination with one or more compatible and pharmaceutically acceptable carriers, such as excipients, diluents or adjuvants, or with another anti-cancer agent.

In certain embodiments, the second agent can be formulated or packaged with the compound provided herein. Of course, the second agent will only be formulated with the compound provided herein when, according to the judgment of those of skill in the art, such co-formulation should not interfere with the activity of either agent or the method of administration. In certain embodiments, the compound provided herein and the second agent are formulated separately. They can be packaged together, or packaged separately, for the convenience of the practitioner of skill in the art.

In clinical practice the active agents provided herein may be administered by any conventional route, in particular orally, parenterally, rectally or by inhalation (*e.g*. in the form of aerosols). In certain embodiments, the compound provided herein is administered orally.

Use may be made, as solid compositions for oral administration, of tablets, pills, hard gelatin capsules, powders or granules. In these compositions, the active product is mixed with one or more inert diluents or adjuvants, such as sucrose, lactose, or starch.

These compositions can comprise substances other than diluents, for example a lubricant, such as magnesium stearate, or a coating intended for controlled release.

Use may be made, as liquid compositions for oral administration, of solutions which are pharmaceutically acceptable, suspensions, emulsions, syrups, and elixirs containing inert diluents, such as water or liquid paraffin. These compositions can also comprise substances other than diluents, for example wetting, sweetening or flavoring products.

The compositions for parenteral administration can be emulsions or sterile solutions. Use may be made, as solvent or vehicle, of propylene glycol, a polyethylene glycol, vegetable oils, in particular olive oil, or injectable organic esters, for example ethyl oleate. These compositions can also contain adjuvants, in particular wetting, isotonizing, emulsifying, dispersing, and stabilizing agents. Sterilization can be carried out in several ways, for example using a bacteriological filter, by radiation or by heating. They can also be prepared in the form of sterile solid compositions which can be dissolved at the time of use in sterile water or any other injectable sterile medium.

The compositions for rectal administration are suppositories or rectal capsules which contain, in addition to the active principle, excipients such as cocoa butter, semi-synthetic glycerides or polyethylene glycols.

The compositions can also be aerosols. For use in the form of liquid aerosols, the compositions can be stable sterile solutions or solid compositions dissolved at the time of use in apyrogenic sterile water, in saline or any other pharmaceutically acceptable vehicle. For use in the form of dry aerosols intended to be directly inhaled, the active principle is finely divided and combined with a water-soluble solid diluent or vehicle, for example dextran, mannitol, or lactose.

In one embodiment, a composition provided herein is a pharmaceutical composition or a single unit dosage form. Pharmaceutical compositions and single unit dosage forms provided herein comprise a prophylactically or therapeutically effective amount of one or more prophylactic or therapeutic agents (*e.g*., a compound provided herein, or other prophylactic or therapeutic agent), and a typically one or more pharmaceutically acceptable carriers or excipients. In a specific embodiment and in this context, the term "pharmaceutically acceptable" means approved by a regulatory agency of the Federal or a state government or listed in the U.S. Pharmacopeia or other generally recognized pharmacopeia for use in animals, and more particularly in humans. The term "carrier" includes a diluent, adjuvant (*e.g*., Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water can be used as a carrier when the pharmaceutical composition is administered intravenously. Saline solutions and aqueous dextrose and glycerol solutions can also be employed as liquid carriers, particularly for injectable solutions. Examples of suitable pharmaceutical carriers are described in "*Remington's Pharmaceutical Sciences"* by E.W. Martin.

Typical pharmaceutical compositions and dosage forms comprise one or more excipients. Suitable excipients are well-known to those skilled in the art of pharmacy, and non-limiting examples of suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. Whether a particular excipient is suitable for incorporation into a pharmaceutical composition or dosage form depends on a variety of factors well known in the art including, but not limited to, the way in which the dosage form will be administered to a subject and the specific active ingredients in the dosage form. The composition or single unit dosage form, if desired, can also contain minor amounts of wetting or emulsifying agents, or pH buffering agents.

Lactose free compositions provided herein can comprise excipients that are well known in the art and are listed, for example, in the U.S. Pharmocopia (USP) SP (XXI)/NF (XVI). In general, lactose free compositions comprise an active ingredient, a binder/filler, and a lubricant in pharmaceutically compatible and pharmaceutically acceptable amounts. Exemplary lactose free dosage forms comprise an active ingredient, microcrystalline cellulose, pre gelatinized starch, and magnesium stearate.

Further encompassed herein are anhydrous pharmaceutical compositions and dosage forms comprising active ingredients, since water can facilitate the degradation of some compounds. For example, the addition of water (*e.g*., 5%) is widely accepted in the pharmaceutical arts as a means of simulating long term storage in order to determine characteristics such as shelf life or the stability of formulations over time. *See*, *e.g*., Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms provided herein can be prepared using anhydrous or low moisture containing ingredients and low moisture or low humidity conditions. Pharmaceutical compositions and dosage forms that comprise lactose and at least one active ingredient that comprises a primary or secondary amine can be anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions can be packaged using materials known to prevent exposure to water such that they can be included in suitable formulary kits. Examples of suitable packaging include, but are not limited to, hermetically sealed foils, plastics, unit dose containers (*e.g*., vials), blister packs, and strip packs.

Further provided are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

The pharmaceutical compositions and single unit dosage forms can take the form of solutions, suspensions, emulsion, tablets, pills, capsules, powders, sustained-release formulations, and the like. Oral formulation can include standard carriers such as pharmaceutical grades of mannitol, lactose, starch, magnesium stearate, sodium saccharine, cellulose, magnesium carbonate, etc. Such compositions and dosage forms will contain a prophylactically or therapeutically effective amount of a prophylactic or therapeutic agent, in certain embodiments, in purified form, together with a suitable amount of carrier so as to provide the form for proper administration to the subject. The formulation should suit the mode of administration. In a certain embodiment, the pharmaceutical compositions or single unit dosage forms are sterile and in suitable form for administration to a subject, for example, an animal subject, such as a mammalian subject, for example, a human subject.

A pharmaceutical composition is formulated to be compatible with its intended route of administration. Examples of routes of administration include, but are not limited to, parenteral, *e.g*., intravenous, intradermal, subcutaneous, intramuscular, subcutaneous, oral, buccal, sublingual, inhalation, intranasal, transdermal, topical, transmucosal, intra-tumoral, intra-synovial, and rectal administration. In a specific embodiment, the composition is formulated in accordance with routine procedures as a pharmaceutical composition adapted for intravenous, subcutaneous, intramuscular, oral, intranasal, or topical administration to human beings. In an embodiment, a pharmaceutical composition is formulated in accordance with routine procedures for subcutaneous administration to human beings. Typically, compositions for intravenous administration are solutions in sterile isotonic aqueous buffer. Where necessary, the composition may also include a solubilizing agent and a local anesthetic such as lignocamne to ease pain at the site of the injection.

Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (*e.g.*, nasal sprays or inhalers); gels; liquid dosage forms suitable for oral or mucosal administration to a subject, including suspensions (*e.g*., aqueous or non aqueous liquid suspensions, oil in water emulsions, or a water in oil liquid emulsions), solutions, and elixirs; liquid dosage forms suitable for parenteral administration to a subject; and sterile solids (*e.g*., crystalline or amorphous solids) that can be reconstituted to provide liquid dosage forms suitable for parenteral administration to a subject.

The composition, shape, and type of dosage forms provided herein will typically vary depending on their use. For example, a dosage form used in the initial treatment of viral infection may contain larger amounts of one or more of the active ingredients it comprises than a dosage form used in the maintenance treatment of the same infection. Similarly, a parenteral dosage form may contain smaller amounts of one or more of the active ingredients it comprises than an oral dosage form used to treat the same disease or disorder. These and other ways in which specific dosage forms encompassed herein will vary from one another will be readily apparent to those skilled in the art. *See*, *e.g.*, Remington's Pharmaceutical Sciences, 20th ed., Mack Publishing, Easton PA (2000).

Generally, the ingredients of compositions are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Typical dosage forms comprise a compound provided herein, or a pharmaceutically acceptable salt, solvate or hydrate thereof lie within the range of from about 0.1 mg to about 1000 mg per day, given as a single once-a-day dose in the morning or as divided doses throughout the day taken with food. Particular dosage forms can have about 0.1, 0.2, 0.3, 0.4, 0.5, 1.0, 2.0, 2.5, 5.0, 10.0, 15.0, 20.0, 25.0, 50.0, 100, 200, 250, 500, or 1000 mg of the active compound.

### Oral Dosage Forms

Pharmaceutical compositions that are suitable for oral administration can be presented as discrete dosage forms, such as, but are not limited to, tablets (*e.g*., chewable tablets), caplets, capsules, and liquids (*e.g*., flavored syrups). Such dosage forms contain predetermined amounts of active ingredients, and may be prepared by methods of pharmacy well known to those skilled in the art. *See* generally, Remington's Pharmaceutical Sciences, 20th ed., Mack Publishing, Easton PA (2000).

In certain embodiments, the oral dosage forms are solid and prepared under anhydrous conditions with anhydrous ingredients, as described in detail in the sections above. However, the scope of the compositions provided herein extends beyond anhydrous, solid oral dosage forms. As such, further forms are described herein.

Typical oral dosage forms are prepared by combining the active ingredient(s) in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g*., powders, tablets, capsules, and caplets) include, but are not limited to, starches, sugars, micro crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free flowing form such as powder or granules, optionally mixed with an excipient. Molded tablets can be made by molding in a suitable machine a mixture of the powdered compound moistened with an inert liquid diluent.

Examples of excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre gelatinized starch, hydroxypropyl methyl cellulose, (*e.g*., Nos. 2208, 2906, 2910), microcrystalline cellulose, and mixtures thereof.

Examples of fillers suitable for use in the pharmaceutical compositions and dosage forms disclosed herein include, but are not limited to, talc, calcium carbonate (*e.g*., granules or powder), microcrystalline cellulose, powdered cellulose, dextrates, kaolin, mannitol, silicic acid, sorbitol, starch, pre gelatinized starch, and mixtures thereof. The binder or filler in pharmaceutical compositions is typically present in from about 50 to about 99 weight percent of the pharmaceutical composition or dosage form.

Suitable forms of microcrystalline cellulose include, but are not limited to, the materials sold as AVICEL PH 101, AVICEL PH 103 AVICEL RC 581, AVICEL PH 105 (available from FMC Corporation, American Viscose Division, Avicel Sales, Marcus Hook, PA), and mixtures thereof. A specific binder is a mixture of microcrystalline cellulose and sodium carboxymethyl cellulose sold as AVICEL RC 581. Suitable anhydrous or low moisture excipients or additives include AVICEL PH 103™ and Starch 1500 LM.

Disintegrants are used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms. The amount of disintegrant used varies based upon the type of formulation, and is readily discernible to those of ordinary skill in the art. Typical pharmaceutical compositions comprise from about 0.5 to about 15 weight percent of disintegrant, specifically from about 1 to about 5 weight percent of disintegrant.

Disintegrants that can be used in pharmaceutical compositions and dosage forms include, but are not limited to, agar agar, alginic acid, calcium carbonate, microcrystalline cellulose, croscarmellose sodium, crospovidone, polacrilin potassium, sodium starch glycolate, potato or tapioca starch, pre gelatinized starch, other starches, clays, other algins, other celluloses, gums, and mixtures thereof.

Lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g*., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Plano, TX), CAB O SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

### Delayed Release Dosage Forms

Active ingredients such as the compounds provided herein can be administered by controlled release means or by delivery devices that are well known to those of ordinary skill in the art. Examples include, but are not limited to, those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; and 6,699,500; each of which is incorporated herein by reference in its entirety. Such dosage forms can be used to provide slow or controlled release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled release formulations known to those of ordinary skill in the art, including those described herein, can be readily selected for use with the active ingredients provided herein. Thus encompasseed herein are single unit dosage forms suitable for oral administration such as, but not limited to, tablets, capsules, gelcaps, and caplets that are adapted for controlled release.

All controlled release pharmaceutical products have a common goal of improving drug therapy over that achieved by their non controlled counterparts. Ideally, the use of an optimally designed controlled release preparation in medical treatment is characterized by a minimum of drug substance being employed to cure or control the condition in a minimum amount of time. Advantages of controlled release formulations include extended activity of the drug, reduced dosage frequency, and increased subject compliance. In addition, controlled release formulations can be used to affect the time of onset of action or other characteristics, such as blood levels of the drug, and can thus affect the occurrence of side (*e.g.*, adverse) effects.

Most controlled release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled release of an active ingredient can be stimulated by various conditions including, but not limited to, pH, temperature, enzymes, water, or other physiological conditions or compounds.

In certain embodiments, the drug may be administered using intravenous infusion, an implantable osmotic pump, a transdermal patch, liposomes, or other modes of administration. In one embodiment, a pump may be used (*see,* Sefton, CRC Crit. Ref. Biomed. Eng. 14:201 (1987**)**; Buchwald et al., Surgery 88:507 (1980); Saudek et al., N. Engl. J. Med. 321:574 (1989)). In another embodiment, polymeric materials can be used. In yet another embodiment, a controlled release system can be placed in a subject at an appropriate site determined by a practitioner of skill, *i.e.*, thus requiring only a fraction of the systemic dose (*see, e.g.*, Goodson, Medical Applications of Controlled Release, vol. 2, pp. 115-138 (1984)). Other controlled release systems are discussed in the review by Langer (Science 249:1527-1533 (1990)). The active ingredient can be dispersed in a solid inner matrix, *e.g*., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, *e.g*., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient in such parenteral compositions is highly dependent on the specific nature thereof, as well as the needs of the subject.

### Parenteral Dosage Forms

In one embodiment, provided are parenteral dosage forms. Parenteral dosage forms can be administered to subjects by various routes including, but not limited to, subcutaneous, intravenous (including bolus injection), intramuscular, and intraarterial. Because their administration typically bypasses subjects' natural defenses against contaminants, parenteral dosage forms are typically sterile or capable of being sterilized prior to administration to a subject. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. In certain embodiments, examples of a suitable vehicle include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water miscible vehicles such as, but not limited to, ethyl alcohol, polyethylene glycol, and polypropylene glycol; and non aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms.

### Transdermal, Topical & Mucosal Dosage Forms

Also provided are transdermal, topical, and mucosal dosage forms. Transdermal, topical, and mucosal dosage forms include, but are not limited to, ophthalmic solutions, sprays, aerosols, creams, lotions, ointments, gels, solutions, emulsions, suspensions, or other forms known to one of skill in the art. See, *e.g*., Remington's Pharmaceutical Sciences, 16th, 18th and 20th eds., Mack Publishing, Easton PA (1980, 1990 & 2000); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels. Further, transdermal dosage forms include "reservoir type" or "matrix type" patches, which can be applied to the skin and worn for a specific period of time to permit the penetration of a desired amount of active ingredients.

Suitable excipients (*e.g*., carriers and diluents) and other materials that can be used to provide transdermal, topical, and mucosal dosage forms encompassed herein are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane -1,3- diol, isopropyl myristate, isopropyl palmitate, mineral oil, and mixtures thereof to form lotions, tinctures, creams, emulsions, gels or ointments, which are non toxic and pharmaceutically acceptable. Moisturizers or humectants can also be added to pharmaceutical compositions and dosage forms if desired. Examples of such additional ingredients are well known in the art. See, *e.g*., Remington's Pharmaceutical Sciences, 16th, 18th and 20th eds., Mack Publishing, Easton PA (1980, 1990 & 2000).

Depending on the specific tissue to be treated, additional components may be used prior to, in conjunction with, or subsequent to treatment with active ingredients provided. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery enhancing or penetration enhancing agent. Different salts, hydrates or solvates of the active ingredients can be used to further adjust the properties of the resulting composition.

### Method of Use

In one embodiment, provided herein is the use in the treatment of a proliferative disease in a subject, which comprises administering to the subject a therapeutically effective amount of a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof.

In certain embodiments, the therapeutically effective amount is ranging from about 0.1 to about 100 mg/kg/day, from about 0.1 to about 50 mg/kg/day, from about 0.1 to about 40 mg/kg/day, from about 0.1 to about 30 mg/kg/day, from about 0.1 to about 25 mg/kg/day, from about 0.1 to about 20 mg/kg/day, from about 0.1 to about 15 mg/kg/day, from about 0.1 to about 10 mg/kg/day, or from about 0.1 to about 5 mg/kg/day. In one embodiment, the therapeutically effective amount is ranging from about 0.1 to about 100 mg/kg/day. In another embodiment, the therapeutically effective amount is ranging from about 0.1 to about 50 mg/kg/day. In yet another embodiment, the therapeutically effective amount is ranging from about 0.1 to about 40 mg/kg/day. In yet another embodiment, the therapeutically effective amount is ranging from about 0.1 to about 30 mg/kg/day. In yet another embodiment, the therapeutically effective amount is ranging from about 0.1 to about 25 mg/kg/day. In yet another embodiment, the therapeutically effective amount is ranging from about 0.1 to about 20 mg/kg/day. In yet another embodiment, the therapeutically effective amount is ranging from about 0.1 to about 15 mg/kg/day. In yet another embodiment, the therapeutically effective amount is ranging from about 0.1 to about 10 mg/kg/day. In still another embodiment, the therapeutically effective amount is ranging from about 0.1 to about 5 mg/kg/day.

It is understood that the administered dose can also be expressed in units other than mg/kg/day. For example, doses for parenteral administration can be expressed as mg/m²/day. One of ordinary skill in the art would readily know how to convert doses from mg/kg/day to mg/m²/day to given either the height or weight of a subject or both (*see,* www.fda.gov/cder/cancer/animalframe.htm). For example, a dose of 1 mg/m²/day for a 65 kg human is approximately equal to 38 mg/kg/day.

In certain embodiments, the subject is a mammal. In certain embodiments, the subject is a human.

In one embodiment is a compound or a pharmaceutically acceptable salt, or stereoisomeric, tautomeric, or polymorphic form thereof for use in therapy.

In certain embodiments, the proliferative disease is a carcinoma, including, but not limited to, Kit-mediated carcinomas, adenocarcinoma, squamous cell carcinoma, adenosquamous carcinoma, teratocarcinoma, head and neck cancer, brain cancer, intracranial carcinoma, glioblastoma (including PDGFR-mediated glioblastoma), glioblastoma multiforme (including PDGFR-mediated glioblastoma multiforme), neuroblastoma, cancer of the larynx, multiple endocrine neoplasias 2A and 2B (MENS 2A and MENS 2B) (including RET-mediated MENS), thyroid cancer (including sporadic and familial medullary thyroid carcinoma), papillary thyroid carcinoma, parathyroid carcinoma (including any RET-mediated thyroid carcinoma), follicular thyroid cancer, anaplastic thyroid cancer, bronchial carcinoid, oat cell carcinoma, lung cancer, small-cell lung cancer (including FLT3 and/or Kit-mediated small cell lung cancer), non-small-cell lung cancer, stomach/gastric cancer, gastrointestinal cancer, gastrointestinal stromal tumors (GIST) (including Kit-mediated GIST and PDGFR α-mediated GIST), colon cancer, colorectal cancer, pancreatic cancer, islet cell carcinoma, hepatic/liver cancer, metastases to the liver, bladder cancer, renal cell cancer (including PDGFR-mediated renal cell cancer), cancers of the genitourinary tract, ovarian cancer (including Kit-mediated and/or PDGFR-mediated ovarian cancer), endometrial cancer (including CSF-1R-mediated endometrial cancer), cervical cancer, breast cancer (including FLT3-mediated and/or PDGFR-mediated breast cancer), prostate cancer (including Kit-mediated prostate cancer), germ cell tumors (including Kit-mediated germ cell tumors), seminomas (including Kit-mediated seminomas), dysgerminomas (including Kit-mediated dysgerminomas), melanoma (including PDGFR-mediated melanoma), metastases to the bone (including CSF-1R-mediated bone metastases), metastatic tumors (including VEGFR-mediated tumors), stromal tumors, neuroendocrine tumors, tumor angiogenesis (including VEGFR-mediated tumor angiogenesis), and mixed mesodermal tumors.

In certain embodiments, the proliferative disease is sarcomas, including, but not limited to, PDGFR-mediated sarcomas, osteosarcoma, osteogenic sarcoma, bone cancer, glioma (including PDGFR-mediated and/or CSF-1R-mediated glioma), astrocytoma, vascular tumors (including VEGFR-mediated vascular tumors), Kaposi's sarcoma, carcinosarcoma, hemangiosarcomas (including VEGFR3-mediated hemangiosarcomas), and lymphangiosarcoma (including VEGFR3-mediated lymphangiosarcoma).

In certain embodiments, the proliferative disease is a hematologic malignancy. In certain embodiments, the proliferative disease is a relapsed hematologic malignancy. In certain embodiments, the proliferative disease is a refractory hematologic malignancy. In certain embodiments, the proliferative disease is a drug-resistant hematologic malignancy. In certain embodiments, the proliferative disease is a multidrug-resistant hematologic malignancy. In certain embodiments, the proliferative disease is a Bcr-Abl kinase inhibitor-resistant hematologic malignancy. In certain embodiments, the proliferative disease is an imatinib-resistant hematologic malignancy. In certain embodiments, the proliferative disease is a dasatinib-resistant hematologic malignancy. In certain embodiments, the proliferative disease is a nilatinib-resistant hematologic malignancy. In certain embodiments, the proliferative disease is a bosutinib-resistant hematologic malignancy. In certain embodiments, the proliferative disease is a cytarabine-resistant hematologic malignancy.

In certain embodiments, the hematologic malignancy is myeloma, leukemia, myeloproliferative diseases, acute myeloid leukemia (AML) (including FLT3 mediated and/or KIT-mediated and/or CSF1R-mediated acute myeloid leukemia), chronic myeloid leukemias (CML) (including FLT3-mediated and/or PDGFR-mediated chronic myeloid leukemia), myelodysplastic leukemias (including FLT3-mediated myelodysplastic leukemia), myelodysplastic syndrome (including FLT3 mediated and/or Kit-mediated myelodysplastic syndrome), idiopathic hypereosinophilic syndrome (HES) (including PDGFR-mediated HES), chronic eosinophilic leukemia (CEL) (including PDGFR-mediated CEL), chronic myelomonocytic leukemia (CMML), mast cell leukemia (including Kit-mediated mast cell leukemia), or systemic mastocytosis (including Kit-mediated systemic mastocytosis).

In certain embodiments, the hematologic malignancy is lymphoma, lymphoproliferative diseases, acute lymphoblastic leukemia (ALL), B-cell acute lymphoblastic leukemias, T-cell acute lymphoblastic leukemias, chronic lymphocytic leukemia (CLL), natural killer (NK) cell leukemia, B-cell lymphoma, T-cell lymphoma, or natural killer (NK) cell lymphoma.

In an embodiment, the hematologic malignancy is myelodysplastic syndrome (MDS).

In certain embodiments, the hematologic malignancy is Langerhans cell histiocytosis (including CSF-1R-mediated and/or FLT3-mediated Langerhans cell histiocytosis), mast cell tumors, or mastocytosis.

In certain embodiments, the hematologic malignancy is leukemia. In certain embodiments, the hematologic malignancy is relapsed leukemia. In certain embodiments, the hematologic malignancy is refractory leukemia. In certain embodiments, the hematologic malignancy is drug-resistant leukemia. In certain embodiments, the hematologic malignancy is multidrug-resistant leukemia. In certain embodiments, the hematologic malignancy is a Bcr-Abl kinase inhibitor-resistant leukemia. In certain embodiments, the hematologic malignancy is imatinib-resistant leukemia. In certain embodiments, the hematologic malignancy is dasatinib-resistant leukemia. In certain embodiments, the hematologic malignancy is nilatinib-resistant leukemia. In certain embodiments, the hematologic malignancy is bosutinib-resistant leukemia. In certain embodiments, the hematologic malignancy is cytarabine-resistant leukemia.

In certain embodiments, the leukemia is acute leukemia. In certain embodiments, the leukemia is relapsed acute leukemia. In certain embodiments, the leukemia is refractory acute leukemia. In certain embodiments, the leukemia is drug-resistant acute leukemia. In certain embodiments, the leukemia is multidrug-resistant acute leukemia. In certain embodiments, the leukemia is a Bcr-Abl kinase inhibitor-resistant acute leukemia. In certain embodiments, the leukemia is imatinib-resistant acute leukemia. In certain embodiments, the leukemia is dasatinib-resistant acute leukemia. In certain embodiments, the leukemia is nilatinib-resistant acute leukemia. In certain embodiments, the leukemia is bosutinib-resistant acute leukemia. In certain embodiments, the leukemia is cytarabine-resistant acute leukemia. In certain embodiments, the leukemia is a hereditary leukemia. In certain embodiments, the hereditary leukemia is severe congenital neutropenia (SCN). In certain embodiments, the hereditary leukemia is familial platelet disorder with acute myelogenous leukemia (FDP/AML). In certain embodiments, the leukemia is caused by LEF1. In certain embodiments, the leukemia is mediated by LEF1. In certain embodiments, the leukemia is caused by GSK3.

In certain embodiments, the leukemia is ALL. In certain embodiments, the leukemia is relapsed ALL. In certain embodiments, the leukemia is refractory ALL. In certain embodiments, the leukemia is drug-resistant ALL. In certain embodiments, the leukemia is multidrug-resistant ALL. In certain embodiments, the leukemia is a Bcr-Abl kinase inhibitor-resistant ALL. In certain embodiments, the leukemia is imatinib-resistant ALL. In certain embodiments, the leukemia is dasatinib-resistant ALL. In certain embodiments, the leukemia is nilatinib-resistant ALL. In certain embodiments, the leukemia is bosutinib-resistant ALL. In certain embodiments, the leukemia is cytarabine-resistant ALL.

In one embodiment, ALL is leukemia that originates in the blast cells of the bone marrow (B-cells), thymus (T-cells), or lymph nodes. ALL is categorized according to the French-American-British (FAB) Morphological Classification Scheme as L1 - mature-appearing lymphoblasts (T-cells or pre-B-cells), L2 - immature and pleomorphic (variously shaped) lymphoblasts (T-cells or pre-B-cells), and L3 - lymphoblasts (B-cells; Burkitt's cells). In another embodiment, ALL originates in the blast cells of the bone marrow (B-cells). In yet another embodiment, ALL originates in the thymus (T-cells). In yet another embodiment, ALL originates in the lymph nodes. In yet another embodiment, ALL is L1 type characterized by mature-appearing lymphoblasts (T-cells or pre-B-cells). In yet another embodiment, ALL is L2 type characterized by immature and pleomorphic (variously shaped) lymphoblasts (T-cells or pre-B-cells). In still another embodiment, ALL is L3 type characterized by lymphoblasts (B-cells; Burkitt's cells).

In certain embodiments, the leukemia is AML. In certain embodiments, the leukemia is relapsed AML. In certain embodiments, the leukemia is refractory AML. In certain embodiments, the leukemia is drug-resistant AML. In certain embodiments, the leukemia is multidrug-resistant AML. In certain embodiments, the leukemia is a Bcr-Abl kinase inhibitor-resistant AML. In certain embodiments, the leukemia is imatinib-resistant AML. In certain embodiments, the leukemia is dasatinib-resistant AML. In certain embodiments, the leukemia is nilatinib-resistant AML. In certain embodiments, the leukemia is bosutinib-resistant AML. In certain embodiments, the leukemia is cytarabine-resistant AML. In certain embodiments, AML has a RAS mutation. In certain embodiments, the RAS mutation is NRAS, KRAS, or HRAS. In certain embodiments, the RAS mutation is NRAS. In certain embodiments, the RAS mutation is KRAS. In certain embodiments, the RAS mutation is HRAS.

In certain embodiments, AML is undifferentiated AML (M0), myeloblastic leukemia (M1), myeloblastic leukemia (M2), promyelocytic leukemia (M3 or M3 variant [M3V]), myelomonocytic leukemia (M4 or M4 variant with eosinophilia [M4E]), monocytic leukemia (M5), erythroleukemia (M6), or megakaryoblastic leukemia (M7). In one embodiment, AML is undifferentiated AML (M0). In another embodiment, AML is myeloblastic leukemia (M1). In yet another embodiment, AML is myeloblastic leukemia (M2). In yet another embodiment, AML is promyelocytic leukemia (M3 or M3 variant [M3V]). In yet another embodiment, AML is myelomonocytic leukemia (M4 or M4 variant with eosinophilia [M4E]). In yet another embodiment, AML is monocytic leukemia (M5). In yet another embodiment, AML is erythroleukemia (M6). In still another embodiment, AML is megakaryoblastic leukemia (M7). In certain embodiments, the leukemia is chronic leukemia. In certain embodiments, the leukemia is relapsed chronic leukemia. In certain embodiments, the leukemia is refractory chronic leukemia. In certain embodiments, the leukemia is drug-resistant chronic leukemia. In certain embodiments, the leukemia is multidrug-resistant chronic leukemia. In certain embodiments, the leukemia is a Bcr-Abl kinase inhibitor-resistant chronic leukemia. In certain embodiments, the leukemia is imatinib-resistant chronic leukemia. In certain embodiments, the leukemia is dasatinib-resistant chronic leukemia. In certain embodiments, the leukemia is nilatinib-resistant chronic leukemia. In certain embodiments, the leukemia is bosutinib-resistant chronic leukemia. In certain embodiments, the leukemia is cytarabine-resistant chronic leukemia.

In certain embodiments, the leukemia is CLL. In certain embodiments, the leukemia is relapsed CLL. In certain embodiments, the leukemia is refractory CLL. In certain embodiments, the leukemia is drug-resistant CLL. In certain embodiments, the leukemia is multidrug-resistant CLL. In certain embodiments, the leukemia is a Bcr-Abl kinase inhibitor-resistant CLL. In certain embodiments, the leukemia is imatinib-resistant CLL. In certain embodiments, the leukemia is dasatinib-resistant CLL. In certain embodiments, the leukemia is nilatinib-resistant CLL. In certain embodiments, the leukemia is bosutinib-resistant CLL. In certain embodiments, the leukemia is cytarabine-resistant CLL.

In certain embodiments, the leukemia is CML. In certain embodiments, the leukemia is relapsed CML. In certain embodiments, the leukemia is refractory CML. In certain embodiments, the leukemia is drug-resistant CML. In certain embodiments, the leukemia is multidrug-resistant CML. In certain embodiments, the leukemia is a Bcr-Abl kinase inhibitor-resistant CML. In certain embodiments, the leukemia is imatinib-resistant CML. In certain embodiments, the leukemia is dasatinib-resistant CML. In certain embodiments, the leukemia is nilatinib-resistant CML. In certain embodiments, the leukemia is bosutinib-resistant CML. In certain embodiments, the leukemia is cytarabine-resistant CML. In certain embodiments, the leukemia is juvenile CML. In certain embodiments, the leukemia is juvenile CML with one or more NF-1 mutations.

In certain embodiments, the leukemia is T-cell leukemia. In one embodiment, the T-cell leukemia is peripheral T-cell leukemia, T-cell lymphoblastic leukemia, cutaneous T-cell leukemia, and adult T-cell leukemia. In another embodiment, the T-cell leukemia is peripheral T-cell leukemia. In yet another embodiment, the T-cell leukemia is T-cell lymphoblastic leukemia. In yet another embodiment, the T-cell leukemia is cutaneous T-cell leukemia. In still another embodiment, the T-cell leukemia is adult T-cell leukemia.

In certain embodiments, the leukemia is Philadelphia positive. In one embodiment, the Philadelphia positive leukemia is Philadelphia positive AML, including, but not limited to, undifferentiated AML (M0), myeloblastic leukemia (M1), myeloblastic leukemia (M2), promyelocytic leukemia (M3 or M3 variant [M3V]), myelomonocytic leukemia (M4 or M4 variant with eosinophilia [M4E]), monocytic leukemia (M5), erythroleukemia (M6), or megakaryoblastic leukemia (M7). In another embodiment, the Philadelphia positive leukemia is Philadelphia positive ALL.

In certain embodiments, the proliferative disease is cancer, including, but not limited to, head and neck cancer (originating lip, oral cavity, oropharynx, hypopharynx, larynx, nasopharynx, nasal cavity, paranasal sinuses, or salivary glands), lung cancer (including small cell lung cancer and non-small cell lung cancer), gastrointestinal tract cancers (including esophageal cancer), gastric cancer, colorectal cancer, anal cancer, pancreatic cancer, liver cancer, gallbladder cancer, extrahepatic bile duct cancer, cancer of the ampulla of vater, breast cancer, gynecologic cancers (including cancer of uterine cervix), cancer of the uterine body, vaginal cancer, vulvar cancer, ovarian cancer, gestational trophoblastic cancer neoplasia, testicular cancer, urinary tract cancers (including renal cancer), urinary blader cancer, prostate cancer, penile cancer, urethral cancer, neurologic tumors, endocrine neoplasms (including carcinoid and islet cell tumors), pheochromocytoma, adrenal cortical carcinoma, parathyroid carcinoma, and metastases to endocrine glands.

Further examples of cancers are basal cell carcinoma, squamous cell carcinoma, chondrosarcoma (a cancer arising in cartilage cells), mesenchymal-chondrosarcoma, soft tissue sarcomas (including malignant tumors that may arise in any of the mesodermal tissues (muscles, tendons, vessels that carry blood or lymph, joints and fat)), soft tissue sarcomas (include alveolar soft-part sarcoma), angiosarcoma, fibrosarcoma, leiomyosarcoma, liposarcoma, malignant fibrous histiocytoma, hemangiopericytoma, mesenchymoma, schwannoma, peripheral neuroectodermal tumours, rhabdomyosarcoma, synovial sarcoma, gestational trophoblastic tumor (malignancy in which the tissues formed in the uterus following conception become cancerous), Hodgkin's lymphoma, and laryngeal cancer.

In certain embodiments, the proliferative disease is a nonmalignant proliferation disease, including, but not limited to, atherosclerosis (including PDGFR-mediated atherosclerosis), restenosis following vascular angioplasty (including PDGFR-mediated restenosis), and fibroproliferative disorders (including obliterative bronchiolitis and idiopathic myelofibrosis).

In certain embodiments, the proliferative disease is an inflammatory disease or disorder related to immune dysfunction, immunodeficiency, or immunomodulation, including, but not limited to, autoimmune diseases, tissue transplant rejection, graft-versus-host disease, wound healing, kidney disease, multiple sclerosis, thyroiditis, type 1 diabetes, sarcoidosis, allergic rhinitis, inflammatory bowel diseases (including Crohn's disease and ulcerative colitis (UC)), systemic lupus erythematosis (SLE), arthritis, osteoarthritis, rheumatoid arthritis, osteoporosis, asthma, and chronic obstructive pulmonary disease (COPD).

In certain embodiments, the proliferative disease is an infectious disease. In certain embodiments, the infectious disease is fungal infection. In certain embodiments, the infectious disease is a superficial mycose (*e.g*., Tinea versicolor). In certain embodiments, the infectious disease is a cutaneous mycose (*e.g*., epidermis). In certain embodiments, the infectious disease is a subcutaneous mycose. In certain embodiments, the infectious disease is a systemic mycose.

In certain embodiments, the proliferative disease is leukemia, adult T-cell leukemia, promyelocytic leukemia, pre-B cell leukemia, lymphoma, Mantle cell lymphoma, breast cancer, pancreatic cancer, prostate cancer, head and neck cancer, ovarian cancer, melanoma, giloma, liver cancer, renal cancer, colorectal cancer, rhabdomyosarcoma, tongue cancer, stomach cancer, multiple myeloma, bladder cancer, thyroid cancer, epidermoid carcinoma, lung cancer, NSC lung cancer, or large cell lung cancer.

In certain embodiments, the proliferative disease is adult T-cell leukemia, promyelocytic leukemia, pre-B cell leukemia, lymphoma, mantle cell lymphoma, pancreatic cancer, prostate cancer, head and neck cancer, ovarian cancer, melanoma, giloma, liver cancer, renal cancer, colorectal cancer, rhabdomyosarcoma, tongue cancer, stomach cancer, multiple myeloma, bladder cancer, thyroid cancer, epidermoid carcinoma, NSC lung cancer, or large cell lung cancer.

In certain embodiments, the proliferative disease is leukemia, adult T-cell leukemia, promyelocytic leukemia, pre-B cell leukemia, lymphoma, mantle cell lymphoma, breast cancer, head and neck cancer, ovarian cancer, colorectal cancer, tongue cancer, multiple myeloma, or large cell lung cancer.

In an embodiment, the cancers which can be treated by the compounds described herein include, but are not limited to, Acute Lymphoblastic Leukemia; Acute Myeloid Leukemia; Adrenocortical Carcinoma; AIDS-Related Lymphoma; AIDS-Related Malignancies; Anal Cancer; Astrocytoma; Bile Duct Cancer; Bladder Cancer; Bone Cancer, Osteosarcoma/Malignant Fibrous Histiocytoma; Brain Stem Glioma; Brain Tumor, Cerebellar Astrocytoma; Brain Tumor, Cerebral Astrocytoma/Malignant Glioma; Brain Tumor, Ependymoma; Brain Tumor, Medulloblastoma; Brain Tumor, Supratentorial Primitive Neuroectodermal Tumors; Brain Tumor, Visual Pathway and Hypothalamic Glioma; Breast Cancer; Bronchial Adenomas/Carcinoids; Carcinoid Tumor; Carcinoid Tumor, Gastrointestinal; Carcinoma, Adrenocortical; Carcinoma, Islet Cell; Central Nervous System Lymphoma, Primary; Cerebral Astrocytoma/Malignant Glioma; Cervical Cancer; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer; Cutaneous T-Cell Lymphoma; Endometrial Cancer; Ependymoma; Epithelial Cancer, Ovarian; Esophageal Cancer; Esophageal Cancer; Ewing's Family of Tumors; Extracranial Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma, Childhood Brain Stem; Glioma, Childhood Visual Pathway and Hypothalamic; Hairy Cell Leukemia; Head and Neck Cancer; Hepatocellular (Liver) Cancer; Hodgkin's Lymphoma; Hypopharyngeal Cancer; Hypothalamic and Visual Pathway Glioma; Intraocular Melanoma; Islet Cell Carcinoma (Endocrine Pancreas); Kaposi's Sarcoma; Kidney Cancer; Laryngeal Cancer; Leukemia, Acute Lymphoblastic; Leukemia, Acute Myeloid; Leukemia, Chronic Lymphocytic; Leukemia, Chronic Myelogenous; Leukemia, Hairy Cell; Lip and Oral Cavity Cancer; Liver Cancer; Lung Cancer, Non-Small Cell; Lung Cancer, Small Cell; Lymphoblastic Leukemia; Lymphoma, AIDS- Related; Lymphoma, Central Nervous System (Primary); Lymphoma, Cutaneous T-Cell; Lymphoma, Hodgkin's; Lymphoma, Hodgkin's During Pregnancy; Lymphoma, Non-Hodgkin's; Lymphoma, Primary Central Nervous System; Macroglobulinemia, Waldenstrom's; Male Breast Cancer; Malignant Mesothelioma; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplastic Syndromes; Myelogenous Leukemia, Chronic; Myeloid Leukemia; Myeloma, Multiple; Myeloproliferative Disorders, Chronic; Nasal Cavity and Paranasal Sinus Cancer; Nasopharyngeal Cancer; Neuroblastoma; Non-Hodgkin's Lymphoma; Non-Small Cell Lung Cancer; Oral Cancer; Oral Cavity and Lip Cancer; Oropharyngeal Cancer; steosarcoma/Malignant Fibrous Histiocytoma of Bone; Ovarian Epithelial Cancer; Ovarian Germ Cell Tumor; Ovarian Low Malignant Potential Tumor; Pancreatic Cancer; Paranasal Sinus and Nasal Cavity Cancer; Parathyroid Cancer; Penile Cancer; Pheochromocytoma; Pineal and Supratentorial Primitive Neuroectodermal Tumors; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer; Prostate Cancer; Rectal Cancer; Renal Cell (Kidney) Cancer; Renal Pelvis and Ureter, Transitional Cell Cancer; Retinoblastoma; Rhabdomyosarcoma; Salivary Gland Cancer; Sarcoma, Ewing's Family of Tumors; Sarcoma, Kaposi's; Sarcoma (Osteosarcoma)/Malignant Fibrous Histiocytoma of Bone; Sarcoma, Soft Tissue; Sezary Syndrome; Skin Cancer; Skin Cancer (Melanoma); Skin Carcinoma, Merkel Cell; Small Cell Lung Cancer; Small Intestine Cancer; Soft Tissue Sarcoma; Squamous Neck Cancer with Occult Primary, Metastatic; Stomach (Gastric) Cancer; Supratentorial Primitive Neuroectodermal Tumors; T- Cell Lymphoma, Cutaneous; Testicular Cancer; Thymoma, Malignant; Thyroid Cancer; Transitional Cell Cancer of the Renal Pelvis and Ureter; Trophoblastic Tumor, Gestational; Ureter and Renal Pelvis, Transitional Cell Cancer; Urethral Cancer; Uterine Sarcoma; Vaginal Cancer; Visual Pathway and Hypothalamic Glioma; Vulvar Cancer; Waldenstrom's Macro globulinemia; and Wilms' Tumor.

In one embodiment, a cancer potentially associated with mutant IDH enzyme activity is brain cancer, such as an astrocytic tumor (e.g., pilocytic astrocytoma, subependymal giant-cell astrocytoma, diffuse astrocytoma, pleomorphic xanthoastrocytoma, anaplastic astrocytoma, astrocytoma, giant cell glioblastoma, glioblastoma, secondary glioblastoma, primary adult glioblastoma, and primary pediatric glioblastoma); oligodendroglial tumor (e.g., oligodendroglioma, and anaplastic oligodendroglioma); oligoastrocytic tumor (e.g., oligoastrocytoma, and anaplastic oligoastrocytoma); ependymoma (e.g., myxopapillary ependymoma, and anaplastic ependymoma); medulloblastoma; primitive neuroectodermal tumor, schwannoma, meningioma, meatypical meningioma, anaplastic meningioma; and pituitary adenoma. In another embodiment, the brain cancer is glioma, glioblastoma multiforme, paraganglioma, or suprantentorial primordial neuroectodermal tumors (sPNET).

In another embodiment, a cancer potentially associated with mutant IDH enzyme activity is leukemia, such as acute myeloid leukemia (AML), myelodysplastic syndrome (MDS), chronic myelogenous leukemia (CML), myeloproliferative neoplasm (MPN), post-MPN AML, post-MDS AML, del(5q)-associated high risk MDS or AML, blast-phase chronic myelogenous leukemia, angioimmunoblastic lymphoma and acute lymphoblastic leukemia.

In one embodiment, a cancer potentially associated with mutant IDH enzyme activity is skin cancer, including melanoma. In another embodiment, a cancer potentially associated with mutant IDH enzyme activity is prostate cancer, breast cancer, thyroid cancer, colon cancer, or lung cancer. In another embodiment, a cancer potentially associated with mutant IDH enzyme activity is sarcoma, including central chondrosarcoma, central and periosteal chondroma, and fibrosarcoma. In another embodiment, a cancer potentially associated with mutant IDH enzyme activity is cholangiocarcinoma.

Also provided are compounds described herein for use in the treatments described herein.

Also provided are uses of compounds described herein for the manufacture of medicaments for the treatments described herein.

In certain embodiments, the subject to be treated with one of the uses provided herein has not been treated with anticancer therapy for the proliferative disease to be treated prior to the administration of a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof.

In certain embodiments, the subject to be treated with one of the uses provided herein has been treated with anticancer therapy for the proliferative disease to be treated prior to the administration of a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof.

In certain embodiments, the subject to be treated with one of the uses provided herein has developed drug resistance to the anticancer therapy.

The uses provided herein encompass treating a subject regardless of patient's age, although some diseases or disorders are more common in certain age groups. Further provided herein is a the use in the treatment of a subject who has undergone surgery in an attempt to treat the disease or condition at issue, as well as the one who have not. Because the subjects with cancer have heterogeneous clinical manifestations and varying clinical outcomes, the treatment given to a particular subject may vary, depending on his/her prognosis. The skilled clinician will be able to readily determine without undue experimentation, specific secondary agents, types of surgery, and types of non-drug based standard therapy that can be effectively used to treat an individual subject with cancer.

Depending on the disease to be treated and the subject's condition, a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, may be administered by oral, parenteral (*e.g*., intramuscular, intraperitoneal, intravenous, CIV, intracistemal injection or infusion, subcutaneous injection, or implant), inhalation, nasal, vaginal, rectal, sublingual, or topical (*e.g*., transdermal or local) routes of administration. A compound provided herein, *e.g*., an enantiomer, a mixture of enantiomers, a diastereomer, a mixture of two or more diastereomers, a tautomer, a mixture of two or more tautomers, or an isotopic variant thereof; or a pharmaceutically acceptable salt thereof, may be formulated, alone or together, in suitable dosage unit with pharmaceutically acceptable excipients, carriers, adjuvants and vehicles, appropriate for each route of administration.

In one embodiment, a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, is administered orally. In another embodiment, a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, is administered parenterally. In yet another embodiment, a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, is administered intravenously. In yet another embodiment, a compound provided herein, *e.g.*, a compound of a diastereomer, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, is administered intramuscularly. In yet another embodiment, a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, is administered subcutaneously. In still another embodiment, a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, is administered topically.

A compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, can be delivered as a single dose such as, *e.g*., a single bolus injection, or oral tablets or pills; or over time such as, *e.g*., continuous infusion over time or divided bolus doses over time. The compound provided herein can be administered repetitively if necessary, for example, until the patient experiences stable disease or regression, or until the patient experiences disease progression or unacceptable toxicity. For example, stable disease for solid tumors generally means that the perpendicular diameter of measurable lesions has not increased by 25% or more from the last measurement. Response Evaluation Criteria in Solid Tumors (RECIST) Guidelines, Journal of the National Cancer Institute 92(3): 205-216 (2000). Stable disease or lack thereof is determined by methods known in the art such as evaluation of patient symptoms, physical examination, visualization of the tumor that has been imaged using X-ray, CAT, PET, or MRI scan and other commonly accepted evaluation modalities.

A compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, can be administered once daily (QD), or divided into multiple daily doses such as twice daily (BID), and three times daily (TID). In addition, the administration can be continuous, *i.e*., every day, or intermittently. The term "intermittent" or "intermittently" as used herein is intended to mean stopping and starting at either regular or irregular intervals. For example, intermittent administration of a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, is administration for one to six days per week, administration in cycles (*e.g.*, daily administration for two to eight consecutive weeks, then a rest period with no administration for up to one week), or administration on alternate days.

In certain embodiments, a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, is cyclically administered to a patient. Cycling therapy involves the administration of an active agent for a period of time, followed by a rest for a period of time, and repeating this sequential administration. Cycling therapy can reduce the development of resistance to one or more of the therapies, avoid or reduce the side effects of one of the therapies, and/or improves the efficacy of the treatment.

In one embodiment is a combination comprising an effective amount of a compound as provided herein, including an enantiomer, a mixture of enantiomers, a diastereomer, or a mixture of diastereomers thereof; or a pharmaceutically acceptable salt thereof, or a tautomeric, or polymorphic form thereof and one, two, three or more other therapeutic agents, *e.g.* anti-cancer agents.

A compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof, can also be combined or used in combination with other therapeutic agents useful in the treatment and/or prevention of a disease described herein.

As used herein, the term "in combination" includes the use of more than one therapy (*e.g*., one or more prophylactic and/or therapeutic agents). However, the use of the term "in combination" does not restrict the order in which therapies (*e.g*., prophylactic and/or therapeutic agents) are administered to a subject with a disease or disorder. A first therapy (*e.g*., a prophylactic or therapeutic agent such as a compound provided herein) can be administered prior to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.*, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (*e.g*., a prophylactic or therapeutic agent) to the subject. Triple therapy is also contemplated herein.

The route of administration of a compound provided herein, an enantiomer, a mixture of enantiomers, a diastereomer, or a mixture of diastereomers thereof; or a pharmaceutically acceptable salt thereof, is independent of the route of administration of a second therapy. In one embodiment, a compound provided herein, including an enantiomer, a mixture of enantiomers, a diastereomer, or a mixture of diastereomers thereof; or a pharmaceutically acceptable salt, or solvate thereof, is administered orally. In another embodiment, a compound provided herein, including an enantiomer, a mixture of enantiomers, a diastereomer, or a mixture of diastereomers thereof; or a pharmaceutically acceptable salt thereof, is administered intravenously. Thus, in accordance with these embodiments, a compound provided herein, including an enantiomer, a mixture of enantiomers, a diastereomer, or a mixture of diastereomers thereof; or a pharmaceutically acceptable salt thereof, is administered orally or intravenously, and the second therapy can be administered orally, parenterally, intraperitoneally, intravenously, intraarterially, transdermally, sublingually, intramuscularly, rectally, transbuccally, intranasally, liposomally, via inhalation, vaginally, intraoccularly, via local delivery by catheter or stent, subcutaneously, intraadiposally, intraarticularly, intrathecally, or in a slow release dosage form. In one embodiment, a compound provided herein, including an enantiomer, a mixture of enantiomers, a diastereomer, or a mixture of diastereomers thereof; or a pharmaceutically acceptable salt thereof, and a second therapy are administered by the same mode of administration, orally or by IV. In another embodiment, a compound provided herein, including an enantiomer, a mixture of enantiomers, a diastereomer, or a mixture of diastereomers thereof; or a pharmaceutically acceptable salt thereof, is administered by one mode of administration, *e.g*., by IV, whereas the second agent (an anticancer agent) is administered by another mode of administration, *e.g*., orally.

In certain embodiments, each use provided herein may independently, further comprise the step of administering a second therapeutic agent. In one embodiment, the second therapeutic agent is an anticancer agent. In another embodiment, the anticancer agent is an antimetabolite, including, but not limited to, 5-fluoro uracil, methotrexate, cytarabine (also known as cytosine arabinoside or Ara-C), and HDAC (high dose cytarabine) and fludarabine. In yet another embodiment, the anticancer agent is an antimicrotubule agent, including, but not limited to, vinca alkaloids (*e.g*., vincristine and vinblastine) taxanes (*e.g*., paclitaxel and docetaxel), and epothilones and their derivatives (*e.g*., ixabepilone). In yet another embodiment, the anticancer agent is an alkylating agent, including, but not limited to, cyclophosphamide, melphalan, carmustine, and nitrosoureas (*e.g*., bischloroethylnitrosurea and hydroxyurea). In yet another embodiment, the anticancer agent is a platinum agent, including, but not limited to, cisplatin, carboplatin, oxaliplatin, satraplatin (JM-216), and CI-973. In yet another embodiment, the anticancer agent is an anthracycline, including, but not limited to, doxrubicin and daunorubicin. In yet another embodiment, the anticancer agent is an antitumor antibiotic, including, but not limited to, mitomycin, idarubicin, adriamycin, and daunomycin (also known as daunorubicin). In yet another embodiment, the anticancer agent is a topoisomerase inhibitor, *e.g*., etoposide and camptothecins. In yet another embodiment, the anticancer agent is selected from the group consisting of adriamycin, busulfan, cytarabine, cyclophosphamide, dexamethasone, fludarabine, fluorouracil, hydroxyurea, interferons, oblimersen, platinum derivatives, taxol, topotecan, and vincristine.

In another embodiment, the anticancer agent is a Bcr-Abl kinase inhibitor. In one embodiment, the Bcr-Abl kinase inhibitor is imatinib, BMS354825 (dasatinib), AMN107 (nilotinib), AP23464, AZD0530, CGP76030, ON012380, INN-0406 (NS-187), SKI-606 (bosutinib), VX-680, or pyrrolo[2,3-d]pyrimidines including PD166326, PD173955 and PD180970. In another embodiment, the Bcr-Abl kinase inhibitor is imatinib. In yet another embodiment, the Bcr-Abl kinase inhibitor is dasatinib. In yet another embodiment, the Bcr-Abl kinase inhibitor is nilotinib. In yet another embodiment, the Bcr-Abl kinase inhibitor is AP23464. In yet another embodiment, the Bcr-Abl kinase inhibitor is AZD0530. In yet another embodiment, the Bcr-Abl kinase inhibitor is CGP76030. In yet another embodiment, the Bcr-Abl kinase inhibitor is SKI-606. In yet another embodiment, the Bcr-Abl kinase inhibitor is ON012380. In yet another embodiment, the Bcr-Abl kinase inhibitor is INN-0406 (NS-187). In yet another embodiment, the Bcr-Abl kinase inhibitor is a pyrrolo[2,3-d]pyrimidine. In another embodiment, the Bcr-Abl kinase inhibitor is VX-680. In another embodiment, the Bcr-Abl kinase inhibitor is PD166326. In yet another embodiment, the Bcr-Abl kinase inhibitor is PD173955. In still another embodiment, the Bcr-Abl kinase inhibitor is PD180970.

In still another embodiment, the anticancer agent is a FLT3 kinase inhibitor. In one embodiment, the FLT3 kinase inhibitor is PKC 412, MLN 578, CEP-701, CT 53518, CT-53608, CT-52923, D-64406, D-65476, AGL-2033, AG1295, AG1296, KN-1022, PKC-412, SU5416, SU5614, SU11248, L-00021649, or CHIR-258. In another embodiment, the FLT3 kinase inhibitor is PKC 412. In yet another embodiment, the FLT3 kinase inhibitor is MLN 578. In yet another embodiment, the FLT3 kinase inhibitor is CEP-701. In yet another embodiment, the FLT3 kinase inhibitor is CT 53518. In yet another embodiment, the FLT3 kinase inhibitor is CT-53608. In yet another embodiment, the FLT3 kinase inhibitor is CT-52923. In yet another embodiment, the FLT3 kinase inhibitor is D-64406. In yet another embodiment, the FLT3 kinase inhibitor is D-65476. In yet another embodiment, the FLT3 kinase inhibitor is AGL-2033. In yet another embodiment, the FLT3 kinase inhibitor is AG1295. In yet another embodiment, the FLT3 kinase inhibitor is AG1296. In yet another embodiment, the FLT3 kinase inhibitor is KN-1022. In yet another embodiment, the FLT3 kinase inhibitor is KN-1022. In yet another embodiment, the FLT3 kinase inhibitor is SU5416. In yet another embodiment, the FLT3 kinase inhibitor is SU5614. In yet another embodiment, the FLT3 kinase inhibitor is SU11248. In yet another embodiment, the FLT3 kinase inhibitor is L-00021649. In still another embodiment, the FLT3 kinase inhibitor is CHIR-258.

Other therapies or anticancer agents that may be used in combination with a compound provided herein, including an enantiomer, a mixture of enantiomers, a diastereomer, or a mixture of diastereomers thereof; or a pharmaceutically acceptable salt thereof, include surgery, radiotherapy (*e.g*., gamma-radiation, neutron beam radiotherapy, electron beam radiotherapy, proton therapy, brachytherapy, and systemic radioactive isotopes), endocrine therapy, biologic response modifiers (*e.g*., interferons, interleukins, and tumor necrosis factor (TNF)), hyperthermia and cryotherapy, agents to attenuate any adverse effects (*e.g*., antiemetics), and other approved chemotherapeutic drugs, including, but not limited to, alkylating drugs (mechlorethamine, chlorambucil, cyclophosphamide, melphalan, and ifosfamide), antimetabolites (cytarabine (also known as cytosine arabinoside or Ara-C), HDAC (high dose cytarabine), and methotrexate), purine antagonists and pyrimidine antagonists (6-mercaptopurine, 5-fluorouracil, cytarbine, and gemcitabine), spindle poisons (vinblastine, vincristine, vinorelbine, and paclitaxel), podophyllotoxins (etoposide, irinotecan, and topotecan), antibiotics (daunorubicin, doxorubicin, bleomycin, and mitomycin), nitrosoureas (carmustine and lomustine), inorganic ions (cisplatin and carboplatin), enzymes (asparaginase), and hormones (tamoxifen, leuprolide, flutamide, and megestrol), imatinib, adriamycin, dexamethasone, and cyclophosphamide. For a more comprehensive discussion of updated cancer therapies see, http://www.nci.nih.gov/, a list of the FDA approved oncology drugs at http://www.fda.gov, and The Merck Manual, Seventeenth Ed. 1999, the entire contents of which are hereby incorporated by reference.

In one embodiment, the other anticancer agent is selected from the group consisting of vascular endothelial growth factor (VEGF) receptor inhibitors, topoisomerase II inhibitors, smoothen inhibitors, alkylating agents, anti-tumor antibiotics, anti-metabolites, retinoids, immunomodulatory agents including but not limited to anti-cancer vaccines, CTLA-4, LAG-3, PD-1 antagonists and BET bromodomain inhibitors.

Examples of vascular endothelial growth factor (VEGF) receptor inhibitors include, but are not limited to, bevacizumab (sold under the trademark AVASTIN by Genentech/Roche), axitinib, (N-methyl-2-[[3-[([pound])-2-pyridin-2-ylethenyl]-1H-indazol-6-yl]sulfanyl]benzamide, also known as AG013736, and described in PCT Publication No. WO 01 /002369), Brivanib Alaninate ((S)-((R)-1-(4-(4-Fluoro-2-methyl-1H-indol-5-yloxy)-5-methylpyrrolo[2,1-f][1,2,4]triazin-6-yloxy)propan-2-yl)2-aminopropanoate, also known as BMS-582664), motesanib (N-(2,3-dihydro-3,3-dimethyl-1H-indoi-6-y!)-2-[(4-pyridinyimethyj)amino]-3-pyfidinecarboxamide. and described in PCT Publication No. WO 02/068470), pasireotide (also known as SO 230, and described in PCT Publication No. WO 02/010192), and sorafenib (sold under the tradename NEXAVAR).

Examples of topoisomerase II inhibitors, include but are not limited to, etoposide (also known as VP-16 and Etoposide phosphate, sold under the tradenames TOPOSAR, VEPESID and ETOPOPHOS), and teniposide (also known as VM-26, sold under the tradename VUMON).

Examples of alkylating agents, include but are not limited to, 5-azacytidine (sold under the trade name VIDAZA), decitabine (sold under the trade name of DECOGEN), temozolomide (sold under the trade names TEMODAR and TEMODAL by Schering-Plough/Merck), dactinomycin (also known as actinomycin-D and sold under the tradename COSMEGEN), melphalan (also known as L-PAM, L-sarcolysin, and phenylalanine mustard, sold under the tradename ALKERAN), altretamine (also known as hexamethylmelamine (HMM), sold under the tradename HEXALEN), carmustine (sold under the tradename BCNU), bendamustine (sold under the tradename TREANDA), busulfan (sold under the tradenames Busulfex(R) and Myleran(R)), carboplatin (sold under the tradename Paraplatin(R)), lomustine (also known as CCNU, sold under the tradename CeeNU(R)), cisplatin (also known as CDDP, sold under the tradenames Platinol(R) and Platinol(R)-AQ), chlorambucil (sold under the tradename Leukeran(R)), cyclophosphamide (sold under the tradenames Cytoxan(R) and Neosar(R)), dacarbazine (also known as DTIC, DIC and imidazole carboxamide, sold under the tradename DTIC-Dome(R)), altretamine (also known as hexamethylmelamine (HMM) sold under the tradename Hexalen(R)), ifosfamide (sold under the tradename Ifex(R)), procarbazine (sold under the tradename Matulane(R)), mechlorethamine (also known as nitrogen mustard, mustine and mechloroethamine hydrochloride, sold under the tradename Mustargen(R)), streptozocin (sold under the tradename Zanosar(R)), thiotepa (also known as thiophosphoamide, TESPA and TSPA, and sold under the tradename Thioplex(R).

Examples of anti-tumor antibiotics include, but are not limited to, doxorubicin (sold under the tradenames Adriamycin(R) and Rubex(R)), bleomycin (sold under the tradename lenoxane(R)), daunorubicin (also known as dauorubicin hydrochloride, daunomycin, and rubidomycin hydrochloride, sold under the tradename Cerubidine(R)), daunorubicin liposomal (daunorubicin citrate liposome, sold under the tradename DaunoXome(R)), mitoxantrone (also known as DHAD, sold under the tradename Novantrone(R)), epirubicin (sold under the tradename Ellence(TM)), idarubicin (sold under the tradenames Idamycin(R), Idamycin PFS(R)), and mitomycin C (sold under the tradename Mutamycin(R)).

Examples of anti-metabolites include, but are not limited to, claribine (2-chlorodeoxyadenosine, sold under the tradename leustatin(R)), 5-fluorouracil (sold under the tradename Adrucil(R)), 6-thioguanine (sold under the tradename Purinethol(R)), pemetrexed (sold under the tradename Alimta(R)), cytarabine (also known as arabinosylcytosine (Ara-C), sold under the tradename Cytosar-U(R)), cytarabine liposomal (also known as Liposomal Ara-C, sold under the tradename DepoCyt(TM)), decitabine (sold under the tradename Dacogen(R)), hydroxyurea (sold under the tradenames Hydrea(R), Droxia(TM) and Mylocel(TM)), fludarabine (sold under the tradename Fludara(R)), floxuridine (sold under the tradename FUDR(R)), cladribine (also known as 2-chlorodeoxyadenosine (2-CdA) sold under the tradename Leustatin(TM)), methotrexate (also known as amethopterin, methotrexate sodium (MTX), sold under the tradenames Rheumatrex(R) and Trexall(TM)), and pentostatin (sold under the tradename Nipent(R)).

Examples of retinoids include, but are not limited to, alitretinoin (sold under the tradename Panretin(R)), tretinoin (all-trans retinoic acid, also known as ATRA, sold under the tradename Vesanoid(R)), Isotretinoin (13-c/s-retinoic acid, sold under the tradenames Accutane(R), Amnesteem(R), Claravis(R), Clarus(R), Decutan(R), Isotane(R), Izotech(R), Oratane(R), Isotret(R), and Sotret(R)), and bexarotene (sold under the tradename Targretin(R)).

"PD-1 antagonist" means any chemical compound or biological molecule that blocks binding of PD-L1 expressed on a cancer cell to PD-1 expressed on an immune cell (T cell, B cell or NKT cell) and preferably also blocks binding of PD-L2 expressed on a cancer cell to the immune-cell expressed PD-1. Alternative names or synonyms for PD-1 and its ligands include: PDCD1, PD1, CD279 and SLEB2 for PD-1; PDCD1L1, PDL1, B7H1, B7-4, CD274 and B7-H for PD-L1; and PDCD1L2, PDL2, B7-DC, Btdc and CD273 for PD-L2. In any of the uses in treatment, medicaments and uses of the present invention in which a human individual is being treated, the PD-1 antagonist blocks binding of human PD-L1 to human PD-1, and preferably blocks binding of both human PD-L1 and PD-L2 to human PD-1. Human PD-1 amino acid sequences can be found in NCBI Locus No.: NP_005009. Human PD-L1 and PD-L2 amino acid sequences can be found in NCBI Locus No.: NP_054862 and NP_079515, respectively.

PD-1 antagonists useful in any of the uses in treatment, medicaments and uses of the present invention include a monoclonal antibody (mAb), or antigen binding fragment thereof, which specifically binds to PD-1 or PD-L1, and preferably specifically binds to human PD-1 or human PD-L1. The mAb may be a human antibody, a humanized antibody or a chimeric antibody, and may include a human constant region. In some embodiments the human constant region is selected from the group consisting of IgG1, IgG2, IgG3 and IgG4 constant regions, and in preferred embodiments, the human constant region is an IgG1 or IgG4 constant region. In some embodiments, the antigen binding fragment is selected from the group consisting of Fab, Fab'-SH, F(ab')₂, scFv and Fv fragments.

Examples of mAbs that bind to human PD-1, and useful in the uses in treatment, medicaments and uses of the present invention, are described in US7488802, US7521051, US8008449, US8354509, US8168757, WO2004/004771, WO2004/072286, WO2004/056875, and US2011/0271358.

Examples of mAbs that bind to human PD-L1, and useful in the uses in treatment, medicaments and uses of the present invention, are described in WO2013/019906, WO2010/077634 A1 and US8383796. Specific anti-human PD-L1 mAbs useful as the PD-1 antagonist in the uses in treatment, medicaments and uses of the present invention include MPDL3280A, BMS-936559, MEDI4736, MSB0010718C and an antibody which comprises the heavy chain and light chain variable regions of SEQ ID NO:24 and SEQ ID NO:21, respectively, of WO2013/019906.

Other PD-1 antagonists useful in any of the uses in treatment, medicaments and uses of the present invention include an immunoadhesin that specifically binds to PD-1 or PD-L1, and preferably specifically binds to human PD-1 or human PD-L1, e.g., a fusion protein containing the extracellular or PD-1 binding portion of PD-L1 or PD-L2 fused to a constant region such as an Fc region of an immunoglobulin molecule. Examples of immunoadhesion molecules that specifically bind to PD-1 are described in WO2010/027827 and WO2011/066342. Specific fusion proteins useful as the PD-1 antagonist in the uses in treatment, medicaments and uses of the present invention include AMP-224 (also known as B7-DCIg), which is a PD-L2-FC fusion protein and binds to human PD-1.

Examples of other cytotoxic agents include, but are not limited to, arsenic trioxide (sold under the tradename Trisenox(R)), asparaginase (also known as L-asparaginase, and Erwinia L-asparaginase, sold under the tradenames Elspar(R) and Kidrolase(R)).

In an embodiment, the other anticancer agent is a BET bromodomain inhibitor. Examples of BET bromodomain inhibitor include the compounds described in U.S. Patent No. 5712274, WO1994006802, U.S. Patent No. 8476260 and WO2009/084693.

The compounds provided herein can also be provided as an article of manufacture using packaging materials well known to those of skill in the art. *See*, *e.g*., U.S. Pat. Nos. 5,323,907; 5,052,558; and 5,033,252. Examples of pharmaceutical packaging materials include, but are not limited to, blister packs, bottles, tubes, inhalers, pumps, bags, vials, containers, syringes, and any packaging material suitable for a selected formulation and intended mode of administration and treatment.

### Kits

In certain embodiments, provided herein also are kits which, when used by the medical practitioner, can simplify the administration of appropriate amounts of active ingredients to a subject. In certain embodiments, the kit provided herein includes a container and a dosage form of a compound or composition provided herein, including a single enantiomer, a mixture of enantiomers, a diastereomer, or a mixture of diastereomers thereof; or a pharmaceutically acceptable salt, or solvate thereof.

In certain embodiments, the kit includes a container comprising a dosage form of the compound provided herein, including a single enantiomer, a mixture of enantiomers, a diastereomer, or a mixture of diastereomers thereof; or a pharmaceutically acceptable salt, or solvate thereof, in a container comprising one or more other therapeutic agent(s) described herein.

Kits provided herein can further include devices that are used to administer the active ingredients. Examples of such devices include, but are not limited to, syringes, needle-less injectors drip bags, patches, and inhalers. The kits provided herein can also include condoms for administration of the active ingredients.

Kits provided herein can further include pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: aqueous vehicles, including, but not limited to, Water for Injection USP, Sodium Chloride Injection, Ringer's Injection, Dextrose Injection, Dextrose and Sodium Chloride Injection, and Lactated Ringer's Injection; water-miscible vehicles, including, but not limited to, ethyl alcohol, polyethylene glycol, and polypropylene glycol; and non-aqueous vehicles, including, but not limited to, corn oil, cottonseed oil, peanut oil, sesame oil, ethyl oleate, isopropyl myristate, and benzyl benzoate. Disclosed herein is a method of inhibiting the growth of a cell, comprising the step of contacting the cell with a compound provided herein, including an enantiomer, a mixture of enantiomers, a diastereomer, a mixture of two or more diastereomers, a tautomer, a mixture of two or more tautomers, or an isotopic variant thereof; or a pharmaceutically acceptable salt thereof.

In one disclosure, the effective amount of the compound provided herein ranges from about 1 pM to about 1 mM, from about 10 pM to about 10 µM, from about 100 pM to about 2 µM, or from about 1 nM to about 1 µM.

In a further disclosure, the cell is a mammalian cell. In a further disclosure, the mammal cell is a human cell. In a further disclosure, the cell is a tumor cell. In a further disclosure, the cell is a mammalian tumor cell. In a further disclosure, the cell is a human tumor cell. In a further disclosure, the cell is a cancerous cell. In a further disclosure, the cell is a mammalian cancerous cell. In a further disclosure, the cell is a human cancerous cell. In a further disclosure, the cancerous cell is a metastatic cancerous cell. In a further disclosure, the cancerous cell is a metastatic microbial cell. In a further disclosure, the cancerous cell is a metastatic bacterial cell. In a further disclosure, the cancerous cell is a metastatic fungal cell.

In a further disclosure, the cell is a hematologic malignancy cell. In a further disclosure, the cell is a leukemia cell. In a further disclosure, the cell is a relapsed leukemia cell. In a further disclosure, the cell is a refractory leukemia cell. In a further disclosure, the cell is a drug-resistant leukemia cell. In a further disclosure, the cell is a multidrug-resistant leukemia cell. In a further disclosure, the cell is a Bcr-Abl kinase inhibitor-resistant leukemia cell. In a further disclosure, the cell is an imatinib-resistant leukemia cell. In a further disclosure, the cell is a dasatinib-resistant leukemia cell. In a further disclosure, the cell is a nilatinib-resistant leukemia cell. In a further disclosure, the cell is a bosutinib-resistant leukemia cell. In a further disclosure, the cell is a cytarabine-resistant leukemia cell.

In a further disclosure, the cell is a leukemia stem cell. In a further disclosure, the cell is a relapsed leukemia stem cell. In a further disclosure, the cell is a refractory leukemia stem cell. In a further disclosure, the cell is a drug-resistant leukemia stem cell. In a further disclosure, the cell is a multidrug-resistant leukemia stem cell. In a further disclosure, the cell is a Bcr-Abl kinase inhibitor-resistant leukemia stem cell. In a further disclosure, the cell is an imatinib-resistant leukemia stem cell. In a further disclosure, the cell is a dasatinib-resistant leukemia stem cell. In a further disclosure, the cell is a nilatinib-resistant leukemia stem cell. In a further disclosure, the cell is a bosutinib-resistant leukemia stem cell. In a further disclosure, the cell is a cytarabine-resistant leukemia stem cell.

In a further disclosure, the cell is an acute leukemia cell. In a further disclosure, the cell is a relapsed acute leukemia cell. In a further disclosure, the cell is a refractory acute leukemia cell. In a further disclosure, the cell is a drug-resistant acute leukemia cell. In a further disclosure, the cell is a multidrug-resistant acute leukemia cell. In a further disclosure, the cell is a Bcr-Abl kinase inhibitor-resistant acute leukemia cell. In a further disclosure, the cell is an imatinib-resistant acute leukemia cell. In a further disclosure, the cell is a dasatinib-resistant acute leukemia cell. In a further disclosure, the cell is a nilatinib-resistant acute leukemia cell. In a further disclosure, the cell is a bosutinib-resistant acute leukemia cell. In a further disclosure, the cell is a cytarabine-resistant acute leukemia cell.

In a further disclosure, the cell is an ALL cell. In a further disclosure, the cell is a relapsed ALL cell. In a further disclosure, the cell is a refractory ALL cell. In a further disclosure, the cell is a drug-resistant ALL cell. In a further disclosure, the cell is a multidrug-resistant ALL cell. In a further disclosure, the cell is a Bcr-Abl kinase inhibitor-resistant ALL cell. In a further disclosure, the cell is an imatinib-resistant ALL cell. In a further disclosure, the cell is a dasatinib-resistant ALL cell. In a further disclosure, the cell is a nilatinib-resistant ALL cell. In a further disclosure, the cell is a bosutinib-resistant ALL cell. In a further disclosure, the cell is a cytarabine-resistant ALL cell.

In a further disclosure, the cell is an AML cell. In a further disclosure, the cell is a relapsed AML cell. In a further disclosure, the cell is a refractory AML cell. In a further disclosure, the cell is a drug-resistant AML cell. In a further disclosure, the cell is a multidrug-resistant AML cell. In a further disclosure, the cell is a Bcr-Abl kinase inhibitor-resistant AML cell. In a further disclosure, the cell is an imatinib-resistant AML cell. In a further disclosure, the cell is a dasatinib-resistant AML cell. In a further disclosure, the cell is a nilatinib-resistant AML cell. In a further disclosure, the cell is a bosutinib-resistant AML cell. In a further disclosure, the cell is a cytarabine-resistant AML cell.

In a further disclosure, the cell is a chronic leukemia cell. In a further disclosure, the cell is a relapsed chronic leukemia cell. In a further disclosure, the cell is a refractory chronic leukemia cell. In a further disclosure, the cell is a drug-resistant chronic leukemia cell. In a further disclosure, the cell is a multidrug-resistant chronic leukemia cell. In a further disclosure, the cell is a Bcr-Abl kinase inhibitor-resistant chronic leukemia cell. In a further disclosure, the cell is an imatinib-resistant chronic leukemia cell. In a further disclosure, the cell is a dasatinib-resistant chronic leukemia cell. In a further disclosure, the cell is a nilatinib-resistant chronic leukemia cell. In a further disclosure, the cell is a bosutinib-resistant chronic leukemia cell. In a further disclosure, the cell is a cytarabine-resistant chronic leukemia cell.

In a further disclosure, the cell is a CLL cell. In a further disclosure, the cell is a relapsed CLL cell. In a further disclosure, the cell is a refractory CLL cell. In a further disclosure, the cell is a drug-resistant CLL cell. In a further disclosure, the cell is a multidrug-resistant CLL cell. In a further disclosure, the cell is a Bcr-Abl kinase inhibitor-resistant CLL cell. In a further disclosure, the cell is an imatinib-resistant CLL cell. In a further disclosure, the cell is a dasatinib-resistant CLL cell. In a further disclosure, the cell is a nilatinib-resistant CLL cell. In a further disclosure, the cell is a bosutinib-resistant CLL cell. In a further disclosure, the cell is a cytarabine-resistant CLL cell.

In a further disclosure, the cell is a CML cell. In a further disclosure, the cell is a relapsed CML cell. In a further disclosure, the cell is a refractory CML cell. In a further disclosure, the cell is a drug-resistant CML cell. In a further disclosure, the cell is a multidrug-resistant CML cell. In a further disclosure, the cell is a Bcr-Abl kinase inhibitor-resistant CML cell. In a further disclosure, the cell is an imatinib-resistant CML cell. In a further disclosure, the cell is a dasatinib-resistant CML cell. In a further disclosure, the cell is a nilatinib-resistant CML cell. In a further disclosure, the cell is a bosutinib-resistant CML cell. In a further disclosure, the cell is a cytarabine-resistant CML cell.

In a further disclosure, the cell is Philadelphia positive leukemia cell. In one embodiment, the cell is a Philadelphia positive ALL cell. In another embodiment, the cell is a Philadelphia positive AML cell. In yet another embodiment, the cell is a Philadelphia positive CLL cell. In still another embodiment, the cell is a Philadelphia positive CML cell.

The inhibition of cell growth can be gauged by, *e.g*., counting the number of cells contacted with a compound of interest, comparing the cell proliferation with otherwise identical cells not contacted with the compound, or determining the size of the tumor that encompasses the cells. The number of cells, as well as the size of the cells, can be readily assessed using any method known in the art (*e.g*., trypan blue exclusion and cell counting, measuring incorporation of ³H-thymidine into nascent DNA in a cell).

### Assay Methods

Patient samples were obtained from a non-interventional and prospective study. The study included samples from adult patients over 18 years of age who were diagnosed with Acute Myeloid Leukemia (AML).

In one embodiment, the method of data acquisition was performed as follows: on day 1 bone marrow (BM) or peripheral blood (PB) patient sample was received. A small part was separated from the rest of the sample for validation, while the majority of the sample was diluted with culture media and plated into 96-well plates previously prepared with the desired compounds and compound combinations, *e.g.,* a compound or compound combination described herein. The number of live leukemic cells seeded in each well was fixed between 8000 and 32000, depending on the percentage of leukemic cells for each sample. These plates were incubated for 72 hours and analyzed on day 4. Antibodies were added to identify leukemic cells using a gating strategy based on FSC/SSC and expression or lack of expression of different surface markers. The monoclonal antibodies selection was performed to optimize the identification of leukemic cell in each sample.

In one embodiment, non-limiting examples of biomarkers such as the biomarkers CD34, CD45, CD117, and HLADR known as "backbone markers" for AML (van Dongen, J.J. and A. Orfao, EuroFlow: Resetting leukemia and lymphoma immunophenotyping. Basis for companion diagnostics and personalized medicine, Leukemia, 2012, 26, 1899-907) were included in the combination.

In one embodiment, antibody combinations such as CD117/CD45, CD34/CD45, and HLADR/CD45 were used. Live leukemic cells were identified by their light scatter properties (FSC^{int/hi}/ SSC^{int}) in the absence of Annexin-V-FITC staining. FSC/SSC selection was performed to exclude debris. The average percentage of cell viability upon receipt of the sample was 80% and samples were only processed if the viability was greater than 50%.

In one embodiment, sample validation was performed as follows: BM and PB samples were extracted under sterile conditions and received in the laboratory within 24 hours of extraction. Initial analysis evaluated the number of pathological cells and their viability. Different volumes of sample (1 µL, 3 µL, 5 µL and 7 µL) were aliquoted in duplicate into a 96-well plate. To lyse red blood cells, 180 µL of ammonium chloride lysis solution was added to each well (2g KHCO₃, 16.58g NH₄Cl, 0.074g Na₂EDTA-₂H₂O, H₂O to 1L). Following a 10 min incubation period at 4°C, plate was centrifuged for 5 min at 1200 rpm and the supernatant removed. The lysis step was performed twice. To analyze, 20 µL of a combination of Annexin V-FITC (Immunostep, Salamanca, Spain), binding buffer (BB, 2.4g HEPES, 8.19g NaCl, 0.37g Cl₂Ca, H₂O to 1L), and the following monoclonal antibodies (mAb) were added to each well: CD117 (clone 104D2)-PE (Becton Dickinson, San Jose, CA, US), CD34 (clone 581)-PerCP (BioLegend, San Diego, CA, US), HLADR (clone L243)-PB (BioLegend) and CD45 (HI30)-PO (Life Technologies, Carlsbad, CA, US) (van Dongen, J.J., et al., EuroFlow antibody panels for standardized n-dimensional flow cytometric immunophenotyping of normal, reactive and malignant leukocytes, Leukemia, 2012, 26, 1908-75). After 15 min of incubation at room temperature in the dark, a wash step was performed using binding buffer solution. The pellet was resuspended in 30 µL BB for analysis in Vivia's ExviTech© platform. Cell count and viability upon arrival were computed and the optimal volume of sample to use per well was determined.

In one embodiment, the assay was performed according to following method: the whole sample was diluted with RPMI 1640, supplemented with 20% (v/v) FBS (Thermo Scientific, Waltham, MA, US), 2% HEPES, 1% antibiotic (Zell Shield, Labclinics, Barcelona, Spain) and 1% L-glutamine 200mM (Lonza, Hopkinton, MA, US) to a final volume of 60 µL per well. The mixture was dispensed into 96-well plates containing a compound described herein with a Multidrop Combi Smart (Thermo Scientific, Waltham, MA, US). Drug plates were previously prepared using an Echo 550 Liquid Handler (LabCyte, Sunnyvale, CA, US). Five or eight concentrations were used for each compound tested, adjusted to cover the range of activities across patients. The compounds tested were also tested against the corresponding parent as a control. The plates were incubated for 48 hours or 72 hours at 37°C in humidified air containing 5% CO₂.

In one embodiment, data analysis was conducted with Summit software (Beckman Coulter). Identification of pathological cells was performed using a gating strategy based on FSC/SSC and expression or lack of expression of the different mAb markers. Depletion was measured as the difference in the number of live cells in a well with the compounds described herein vs the control wells without the compounds. Annexin V was then used to exclude dying cells and measure only the number of live cells in the drug wells and in the control wells. Those cells without Annexin V staining and appropriate FSC/SSC were considered as live cells (Koopman, G., et al., Annexin V for flow cytometric detection of phosphatidylserine expression on B cells undergoing apoptosis, Blood, 1994, 84, 1415-20). Using the above parameters, FCS Analyzer was used to determine the effect of each of the individual compound. Data were transfered to ActivityBase (IDBS, Guildford, UK) for final analysis.

The disclosure will be further understood by the following non-limiting examples.

The *ex-vivo* effect of the compounds on AML samples when the plates were incubated for 72 hours is illustrated in Table 2:

| Compound | EC50 abs. (µM) | Emax (%) |
|---|---|---|
| 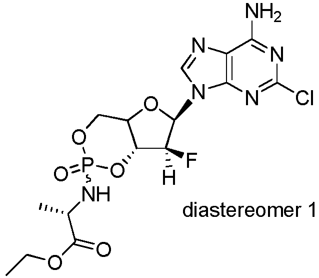 | A | A |
| 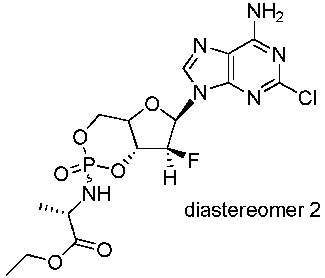 | C | B |
| 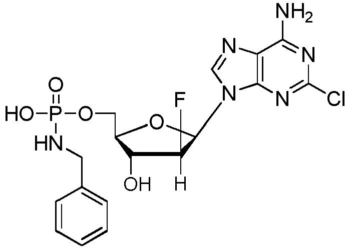 | A | A |
| 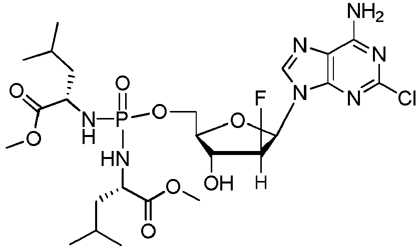 | B | C |
| 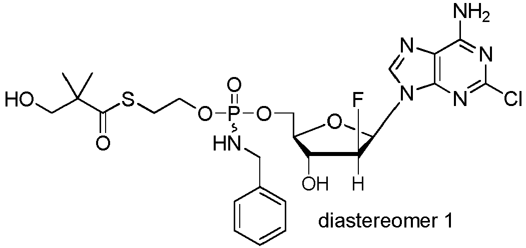 | A | B |
| 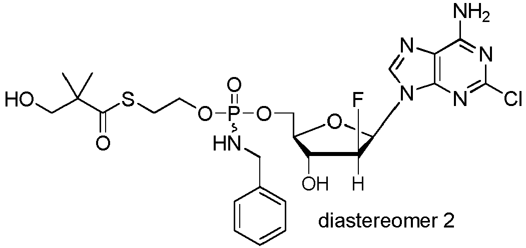 | B | B |
| 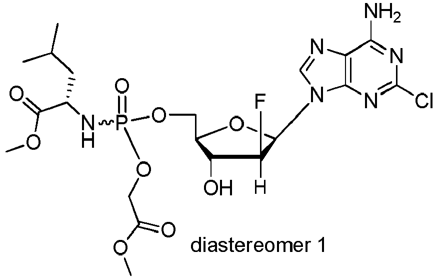 | B | C |
| 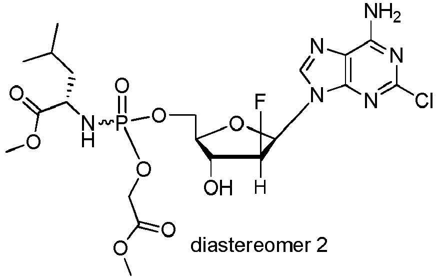 | B | B |
| 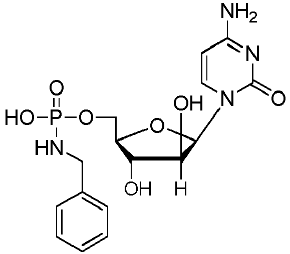 | C | A |
| 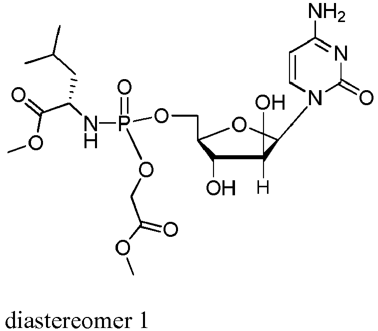 | B | B |
| 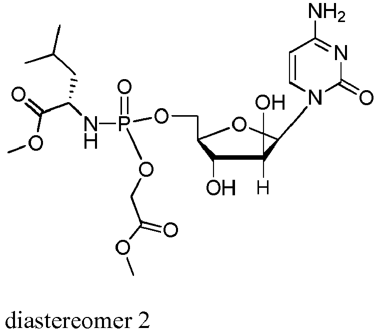 | C | A |
| 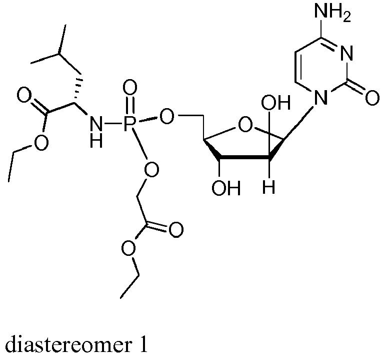 | B | A |
| 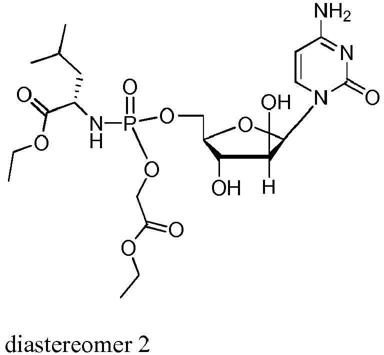 | B | A |
| 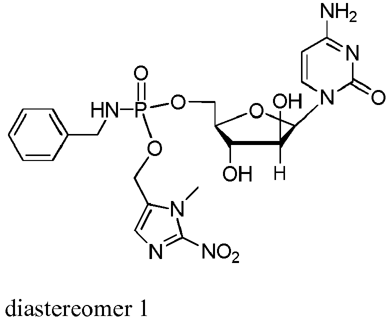 | B | A |
| 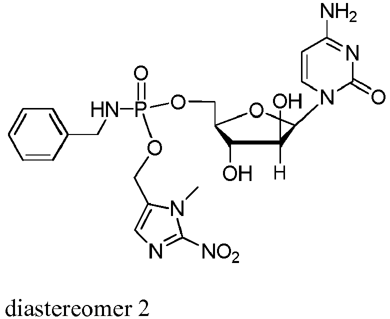 | B | A |
| 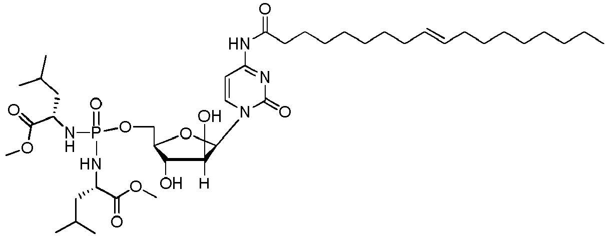 | C | C |
| 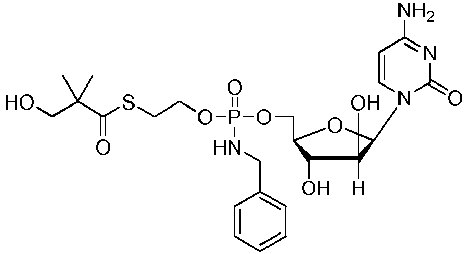 | C | A |
| 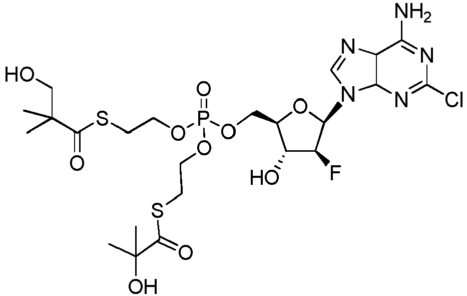 | B | B |

The *ex-vivo* effect of the compounds on AML samples when the plates were incubated for 48 hours is illustrated in Table 3:

| Compound | EC50 abs. (µM) | Emax (%) |
|---|---|---|
| 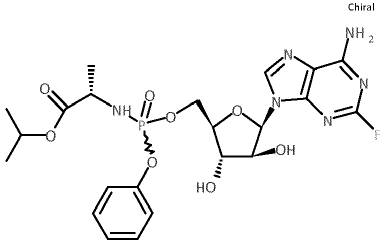 | D | A |
| 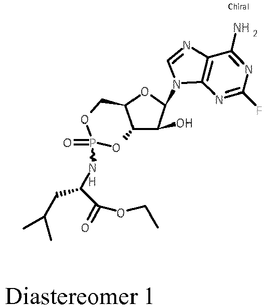 | C | C |
| 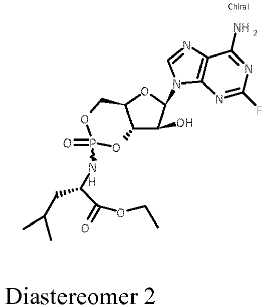 | D | A |
| 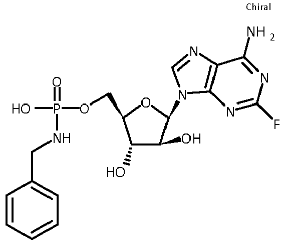 | B | A |
| 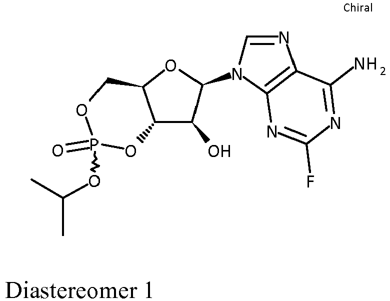 | C | C |
| 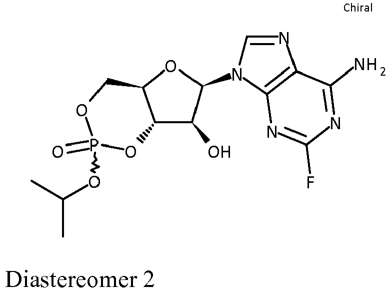 | D | C |
| 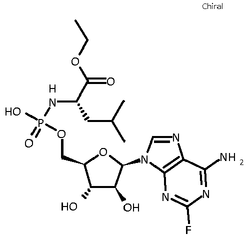 | C | C |
| 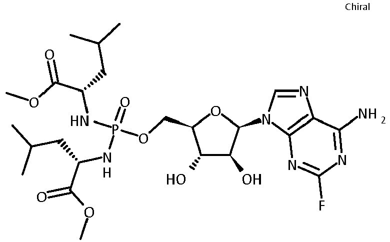 | D | B |
| 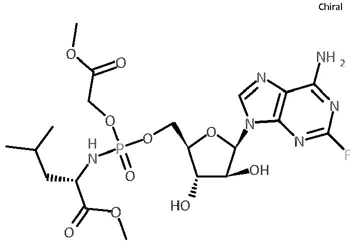 | B | A |
| 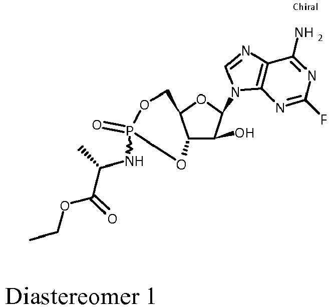 | C | C |
| 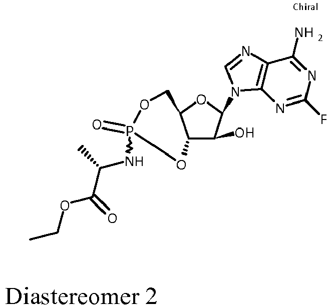 | C | C |

EC₅₀ values are provided as follows:
A ≤ 1 µM, 1 < B ≤ 10 µM, 10 < C < 20 µM, D ≥ 20 µM;

Emax values are provided as follows:
A = 0-10%; B = 10-20%; C > 20%

### In Vitro Inhibition

### Materials

Cells were grown in RPMI-1640 supplemented with L-Glutamine and 10% FBS of the following cancer cell lines:
CCRF-CEM; CCRF-CEM-cytarabine resistant; HL-60;

### Method

18, 96 well plates of each cell line were seeded with the optimized number of cells per well in a total volume of 50 µL per well. The plates were left overnight. Plate wells were seeded with 100 µL media for media control. The following day, cells were exposed to test compounds as described below. At the same time as drug exposure, a CTG assay was conducted on the 18^{th} plate for the 0 hr count.

The compounds were added to cells and medium already on the plate to give desired final concentrations. 50 µL media were added to cell control wells, and 50 µL of mix added to vehicle control wells. 10 µM Doxorubicin was added to appropriate wells as control. Cells exposed to test compound were incubated at 37°C for 72 hr followed by a CTG assay.

### CellTiter-Glo (CTG)

At the end of the 72 hr exposure period, plates were removed for a CellTiter-Glo (CTG) assay from a 37°C, 5% CO₂ incubator and placed on the bench at room temperature for 30 mins. 100 µL of CellTiter-Glo reagent was added and mixed for 2 mins, followed by a further 10 min incubation at room temperature. Luminescence was recorded using Synergy 4.0.

All the exemplified compounds were tested in accordance with this method and were found to have IC₅₀ values of less than 100µM at each cell line. The results for compounds are provided in Table 4.

**Table 4 - In vitro inhibition of CCRF-CEM; CCRF-CEM-cytarabine resistant; and HL-60.**

| Compound | CCRF-CEM | CCRF-CEM-cytarabine resistant | HL-60 |
|---|---|---|---|
| | IC₅₀(µM) | | |
| Compound 10 | A | D | B |
| Compound 11 | A | B | A |
| Compound 24b | A | C | A |
| Compound 24c | A | C | A |
| Compound 24e | A | C | A |
| Compound 24f (diastereoisomer 1) | A | C | C |
| Compound 24f (diastereoisomer 2) | A | C | C |
| Compound 25 (diastereoisomer 1) | A | C | B |
| Compound 25 (diastereoisomer 2) | A | C | B |
| Compound 26a | A | B | A |
| Compound 26b | A | B | A |

The IC₅₀ values in Tables 1 are as follows:
A = < 1 µM
B = ≥ 1 and 10 ≤ µM
C = > 10 and ≤ 25 µM
D = > 25 and ≤ 100 µM

The embodiments described above are intended to be merely exemplary, and those skilled in the art will recognize, or will be able to ascertain using no more than routine experimentation, numerous equivalents of specific compounds, materials, and procedures.

Since modifications will be apparent to those of skill in the art, it is intended that the claimed subject matter be limited only by the scope of the appended claims.

## Claims

1. The compound having the Formula: 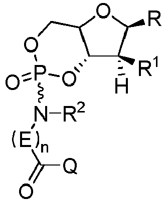 or a pharmaceutically acceptable salt thereof, wherein:
R is 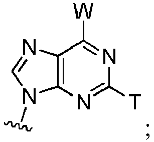
W is NH₂;
T is F or Cl;
R¹ is F;
Q is OR³;
E is CR⁴R⁵;
n is 1;
R² is hydrogen; R³ is alkyl; R⁴ is hydrogen and R⁵ is alkyl, or cycloalkyl; or R⁴ is alkyl or cycloalkyl, and R⁵ is hydrogen,
wherein alkyl is optionally substituted by halogen, oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate,
cycloalkyl is optionally substituted by halogen, oxo, hydroxyl, amino, alkylamino, arylamino, alkylarylamino, alkoxy, aryloxy, thioalkoxy, thioaroxyl, alkyldisulfanyl, acyl, hydroxylcarbonyl, alkoxycarbonyl, aryloxycarbonyl, aminocarbonyl, alkylaminocarbonyl, arylaminocarbonyl, alkylarylaminocarbonyl, acyloxy, acylthio, nitro, cyano, sulfonic acid, sulfate, phosphonic acid, phosphate, or phosphonate;
alkyl is C₁ to C₁₀ alkyl; and
cycloalkyl is C₃ to C₁₀ cycloalkyl.

2. The compound of claim 1 having the following Formula:

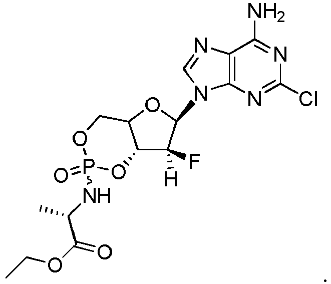

3. A compound of claim 1 selected from:
ethyl (2S)-2-[[(4aR,6R,7S,7aR)-6-(6-amino-2-chloro-purin-9-yl)-7-fluoro-2-oxo-4a,6,7,7a-tetrahydro-4H-furo[3,2-d][1,3,2]dioxaphosphinin-2-yl]amino]propanoate (diastereomer 1 and 2);
and
ethyl (2S)-2-[[(4aR,6R,7S,7aS)-6-(6-amino-2-fluoro-purin-9-yl)-7-hydroxy-2-oxo-4a,6,7,7a-tetrahydro-4H-furo[3,2-d][1,3,2]dioxaphosphinin-2-yl]amino]propanoate (diastereomer 1 and 2);
or a pharmaceutically acceptable salt thereof.

4. A pharmaceutical composition comprising a compound of any one of claims 1-3, or a pharmaceutically acceptable salt thereof and a pharmaceutically acceptable excipient, carrier, or diluents.

5. The pharmaceutical composition of claim 4, wherein the composition is an oral formulation.

6. A compound of any one of claims 1-3, or a pharmaceutically acceptable salt thereof for use in therapy.

7. A compound of any one of claims 1-3, or a pharmaceutically acceptable salt thereof for use in the treatment of a blood cancer.

8. The compound for use of claim 7, wherein the blood cancer is a leukemia cancer.

9. The compound for use of claim 8, wherein the leukemia is acute myelogenous leukemia (AML).

10. A combination comprising a compound of any one of claims 1-3, or a pharmaceutically acceptable salt thereof and a second chemotherapeutic agent.

11. A combination comprising a compound of any one of claims 1-3, or a pharmaceutically acceptable salt thereof and one, two, three or more other therapeutic agents.

## Patentansprüche

1. Die Verbindung mit der Formel: 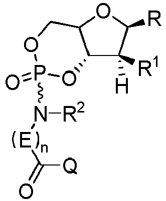 oder ein pharmazeutisch annehmbares Salz davon, wobei:
R 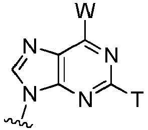 ist,
W NH₂ ist,
T F oder Cl ist,
R¹ F ist,
Q OR³ ist,
E CR⁴R⁵ ist,
n 1 ist,
R² Wasserstoff ist, R³ Alkyl ist, R⁴ Wasserstoff ist und R⁵ Alkyl oder Cycloalkyl ist, oder R⁴ Alkyl oder Cycloalkyl ist und R⁵ Wasserstoff ist,
wobei Alkyl gegebenenfalls substituiert ist durch Halogen, Oxo, Hydroxyl, Amino, Alkylamino, Arylamino, Alkylarylamino, Alkoxy, Aryloxy, Thioalkoxy, Thioaroxyl, Alkyldisulfanyl, Acyl, Hydroxylcarbonyl, Alkoxycarbonyl, Aryloxycarbonyl, Aminocarbonyl, Alkylaminocarbonyl, Arylaminocarbonyl, Alkylarylaminocarbonyl, Acyloxy, Acylthio, Nitro, Cyano, Sulfonsäure, Sulfat, Phosphonsäure, Phosphat oder Phosphonat,
Cycloalkyl gegebenenfalls substituiert ist durch Halogen, Oxo, Hydroxyl, Amino, Alkylamino, Arylamino, Alkylarylamino, Alkoxy, Aryloxy, Thioalkoxy, Thioaroxyl, Alkyldisulfanyl, Acyl, Hydroxylcarbonyl, Alkoxycarbonyl, Aryloxycarbonyl, Aminocarbonyl, Alkylaminocarbonyl, Arylaminocarbonyl, Alkylarylaminocarbonyl, Acyloxy, Acylthio, Nitro, Cyano, Sulfonsäure, Sulfat, Phosphonsäure, Phosphat oder Phosphonat,
Alkyl C₁- bis C₁₀-Alkyl ist und
Cycloalkyl C₃- bis C₁₀-Cycloalkyl ist.

2. Die Verbindung nach Anspruch 1 mit der folgenden Formel:

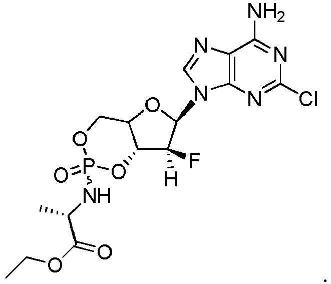

3. Eine Verbindung nach Anspruch 1, ausgewählt aus:
Ethyl-(2S)-2-[[(4aR,6R,7S,7aR)-6-(6-amino-2-chlorpurin-9-yl)-7-fluor-2-oxo-4a,6,7,7a-tetrahydro-4H-furo[3.2-d][1,3,2]dioxaphosphinin-2-yl]amino]propanoate (Diastereomer 1 und 2) und
Ethyl-(2S)-2-[[(4aR,6R,7S,7aS)-6-(6-amino-2-fluorpurin-9-yl)-7-hydroxy-2-oxo-4a,6,7,7a-tetrahydro-4H-furo[3,2-d][1,3,2]dioxaphosphinin-2-yl]amino]propanoate (Diastereomer 1 und 2),
oder ein pharmazeutisch annehmbares Salz davon.

4. Eine pharmazeutische Zusammensetzung, die eine Verbindung nach einem der Ansprüche 1-3 oder ein pharmazeutisch annehmbares Salz davon und einen pharmazeutisch annehmbaren Hilfsstoff, einen pharmazeutisch annehmbaren Träger oder pharmazeutisch annehmbare Verdünnungsmittel umfasst.

5. Die pharmazeutische Zusammensetzung nach Anspruch 4, wobei die Zusammensetzung eine orale Formulierung ist.

6. Eine Verbindung nach einem der Ansprüche 1-3 oder ein pharmazeutisch annehmbares Salz davon zur Verwendung in der Therapie.

7. Eine Verbindung nach einem der Ansprüche 1-3 oder ein pharmazeutisch annehmbares Salz davon zur Verwendung bei der Behandlung eines Blutkrebses.

8. Die Verbindung zur Verwendung nach Anspruch 7, wobei der Blutkrebs ein Leukämie-Krebs ist.

9. Die Verbindung zur Verwendung nach Anspruch 8, wobei die Leukämie akute myeloische Leukämie (AML) ist.

10. Eine Kombination, die eine Verbindung nach einem der Ansprüche 1-3 oder ein pharmazeutisch annehmbares Salz davon und ein zweites chemotherapeutisches Mittel umfasst.

11. Eine Kombination, die eine Verbindung nach einem der Ansprüche 1-3 oder ein pharmazeutisch annehmbares Salz davon und ein, zwei, drei oder mehr andere therapeutische Mittel umfasst.

## Revendications

1. Composé présentant la Formule: 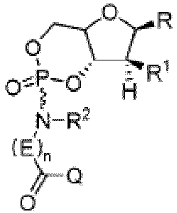 ou un sel pharmaceutiquement acceptable de celui-ci, dans lequel:
R est: 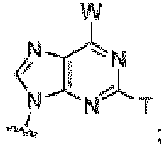
W est NH₂;
T est F ou Cl;
R¹ est F;
Q est QR³;
E est CR⁴R⁵;
n est 1;
R² est un hydrogène; R³ est un alkyle; R⁴ est un hydrogène et R⁵ est un alkyle ou un cycloalkyle; ou R⁴ est un alkyle ou un cycloalkyle et R⁵ est un hydrogène,
dans lequel l'alkyle est optionnellement substitué par: halogène, oxo, hydroxyle, amino, alkylamino, arylamino, alkylarylamino, alcoxy, aryloxy, thioalcoxy, thioaroxyle, alkyldisulfanyle, acyle, hydroxylcarbonyle, alcoxycarbonyle, aryloxycarbonyle, aminocarbonyle, alkylaminocarbonyle, arylaminocarbonyle, alkylarylaminocarbonyle, acyloxy, acylthio, nitro, cyano, acide sulfonique, sulfate, acide phosphonique, phosphate ou phosphonate,
le cycloalkyle est optionnellement substitué par: halogène, oxo, hydroxyle, amino, alkylamino, arylamino, alkylarylamino, alcoxy, aryloxy, thioalcoxy, thioaroxyle, alkyldisulfanyle, acyle, hydroxylcarbonyle, alcoxycarbonyle, aryloxycarbonyle, aminocarbonyle, alkylaminocarbonyle, arylaminocarbonyle, alkylarylaminocarbonyle, acyloxy, acylthio, nitro, cyano, acide sulfonique, sulfate, acide phosphonique, phosphate ou phosphonate,
l'alkyle est un C₁ à C₁₀ alkyle; et
le cycloalkyle est un C₃ à C₁₀ cycloalkyle.

2. Composé selon la revendication 1 présentant la Formule suivante:

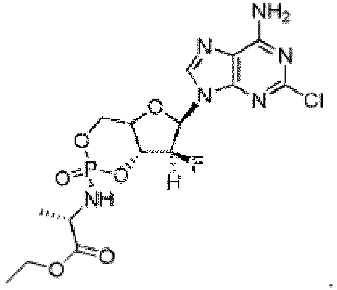

3. Composé selon la revendication 1 choisi parmi:
(2S)-2-[[(4aR,6R,7S,7aR)-6-(6-amino-2-chloro-purin-9-yl)-7-fluoro-2-oxo-4a,6,7,7a-tétrahydro-4H-furo[3,2-d][1,3,2]dioxaphosphinin-2-yl]amino]propanoate d'éthyle (diastéréoisomères 1 et 2);
et
(2S)-2-[[(4aR,6R,7S,7aS)-6-(6-amino-2-chloro-purin-9-yl)-7-hydroxy-2-oxo-4a,6,7,7a-tétrahydro-4H-furo[3,2-d][1,3,2]dioxaphosphinin-2-yl]amino]propanoate d'éthyle (diastéréoisomères 1 et 2);
ou un sel pharmaceutiquement acceptable de ceux-ci.

4. Composition pharmaceutique comprenant un composé selon l'une quelconque des revendications 1-3, ou un sel pharmaceutiquement acceptable de celui-ci, et un excipient, un véhicule ou des diluants pharmaceutiquement acceptables.

5. Composition pharmaceutique selon la revendication 4, où la composition est une formulation orale.

6. Composé selon l'une quelconque des revendications 1-3, ou un sel pharmaceutiquement acceptable de celui-ci, pour une utilisation en thérapie.

7. Composé selon l'une quelconque des revendications 1-3, ou un sel pharmaceutiquement acceptable de celui-ci, pour une utilisation dans le traitement d'un cancer du sang.

8. Composé pour une utilisation selon la revendication 7, où le cancer du sang est un cancer leucémie.

9. Composé pour une utilisation selon la revendication 8, où la leucémie est une leucémie myéloïde aiguë (AML).

10. Combinaison comprenant un composé selon l'une quelconque des revendications 1-3, ou un sel pharmaceutiquement acceptable de celui-ci, et un deuxième agent chimiothérapeutique.

11. Combinaison comprenant un composé selon l'une quelconque des revendications 1-3, ou un sel pharmaceutiquement acceptable de celui-ci, et un, deux, trois ou plus autres agents thérapeutiques.
